# FASCICULE DE BREVET EUROPEEN

(11) **EP 2 262 803 B1**
(45) Date de publication et mention de la délivrance du brevet: **17.10.2012**
(21) Numéro de dépôt: 09754041.3
(22) Date de dépôt: 20.03.2009
(51) Int. Cl.: C07D 471/04, C07D 213/28, C07D 233/56, A61K 31/437, A61P 25/00, A61P 19/00, A61P 35/00

(54) **DÉRIVÉS POLYSUBSTITUES DE 2-ARYL-6-PHENYL-IMIDAZO[1,2- A]PYRIDINES, LEUR PRÉPARATION ET LEUR APPLICATION EN THÉRAPEUTIQUE**
POLYSUBSTITUIERTE 2-ARYL-6-PHENYL-IMIDAZO-[L,2-A-]PYRIDIN-DERIVATE SOWIE DEREN HERSTELLUNG UND THERAPEUTISCHE ANWENDUNG
POLYSUBSTITUTED DERIVATIVES OF 2-ARYL-6-PHENYL-IMIDAZO[L,2-A] PYRIDINES, AND PREPARATION AND THERAPEUTIC USE THEREOF

(30) Priorité: 21.03.2008 FR 0801580
(43) Date de publication de la demande: 22.12.2010
(73) Titulaire: SANOFI, 75008 Paris (FR)
(72) Inventeur: DE PERETTI, Danielle, F-75013 Paris (FR); EVANNO, Yannick, F-75013 Paris (FR)
(74) Mandataire: Le Coupanec, Pascale A.M.P.
(86) Numéro de dépôt international: PCT/FR2009/000297
(87) Numéro de publication internationale: WO 2009/144391

(56) Documents cités:
- WO-A-2008/034974
- FR-A- 2 903 105
- FR-A- 2 903 107
- DIMAURO E F ET AL: "Microwave-Assisted Preparation of Fused Bicyclic Heteroaryl Boronates: Application in One-Pot Suzuki Couplings" JOURNAL OF ORGANIC CHEMISTRY, AMERICAN CHEMICAL SOCIETY, EASTON.; US, vol. 71, no. 10, 1 janvier 2006 (2006-01-01), pages 3959-3962, XP002475376 ISSN: 0022-3263

## Description

La présente invention se rapporte à des dérivés polysubstitués de 2-aryl-6-phényl-imidazo[1,2-a]pyridine, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

La présente invention a pour objet les composés de formule (I) : 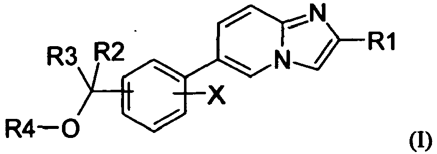 dans laquelle :
R₁ représente :
   un groupe phényle ou un groupe naphtyle, ces deux groupes pouvant éventuellement être substitués par un ou plusieurs atomes ou groupes choisis indépendamment les uns des autres parmi les atomes ou groupes suivants : halogène, (C₁-C₁₀)alkyle, halo(C₁-C₁₀)alkyle, (C₁-C₁₀)alcoxy, halo(C₁-C₁₀)alcoxy, (C₁-C₁₀)thioalkyle, -S(O)(C₁-C₁₀)alkyle, -S(O)₂(C₁-C₁₀-alkyle), hydroxyle, cyano, nitro, hydroxy(C₁-C₁₀)alkylène, NRaRb(C₁-C₁₀)alkylène, (C₁-C₁₀)alcoxy(C₁-C₁₀)alkylène-oxy, NRaRb, CONRaRb, SO₂NRaRb, NRcCORd, OC(O)NRaRb, OCO(C₁-C₁₀)alkyle, NRcC(O)ORe, NRcSO₂Re, aryl(C₁-C₁₀)alkylène, aryle ou hétéroaryle monocyclique, l'aryle ou l'hétéroaryle monocyclique étant éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, oxo, nitro, cyano ou OCO(C₁-C₁₀)alkyle;
X représente de 1 à 4 substituants identiques ou différents l'un de l'autre choisis parmi halogène, (C₁-C₁₀)alkyle, (C₁-C₁₀)alcoxy, NRaRb, cyano, nitro, le (C₁-C₁₀)alkyle pouvant être éventuellement substitué par un ou plusieurs groupes choisis parmi un halogène, (C₁-C₁₀)alcoxy, halo(C₁-C₁₀)alcoxy, NRaRb ou hydroxyle ;
R₂ et R₃ représentent, indépendamment l'un de l'autre,
   un atome d'hydrogène,
   un groupe (C₁₀-C₁₀)alkyle, ce groupe étant éventuellement substitué par un groupe Rf;
   un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro ou cyano ;
R₂ et X peuvent former ensemble avec les atomes de carbone qui les portent un cycle carboné de 5 à 7 atomes de carbone;
R₄ représente :
   un atome d'hydrogène,
   un groupe (C₁-C₁₀)alkyle, ce groupe étant éventuellement substitué par un groupe Rf;
   un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano, (C₁-C₁₀)alkyl(CO)-, CONRaRb, NRcCORd, OC(O)NRaRb, OCO(C₁-C₁₀)alkyle, NRcC(O)ORe ou aryle, l'aryle étant éventuellement substitué par un ou plusieurs substituants choisis parmi un halogène, un groupe (C₁-C₁₀)alkyle, hato(C₁-C₁₀)alkyle, (C₁-C₁₀)alcoxy, halo(C₁-C₁₀)alcoxy, NRaRb, hydroxyle, nitro ou cyano ;
Ra et Rb représentent, indépendamment l'un de l'autre,
   un atome d'hydrogène ou un groupe (C₁-C₁₀)alkyle, aryl(C₁-C₁₀)alkylène ou aryle ;
ou Ra et Rb forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe (C₁-C₁₀)alkyle, aryle ou aryl(C₁-C₁₀)alkylène ;
Rc et Rd représentent, indépendamment l'un de l'autre,
   un atome d'hydrogène ou un groupe (C₁-C₁₀)alkyle, aryl(C₁-C₁₀)alkylène, aryle ;
ou Rc et Rd forment ensemble un groupe (C₂-C₅)alkylène ;
Re représente
   un groupe (C₁-C₁₀)alkyle, aryl(C₁-C₁₀)alkylène, aryle ;
ou Rc et Re forment ensemble un groupe (C₂-C₅)alkylène ;
Rf représente
   un atome d'halogène, un groupe (C₁-C₁₀)alcoxy, halo(C₁-C₁₀)alcoxy, hydroxyle, cyano, NRaRb, C(O)NRaRb, NRcCORd, OC(O)NRaRb, OCO(C₁-C₁₀)alkyle, NRcCOORe, SO₂NRaRb, NRcSO₂Re, aryl(C₁-C₁₀)alkylène, aryle, l'aryle étant éventuellement substitué par un ou plusieurs substituants choisi parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano ou OCO(C₁-C₁₀)alkyle;
à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un un groupe (Cₓ-Cₜ) : un groupe comprenant entre x et t atomes de carbone ;
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyl, cyclopropylméthyl etc ;
- un groupe alkylène : un groupe alkyle divalent
- un groupe alcoxy : un radical -O-alkyle où le groupe alkyle est tel que précédemment défini ;
- un groupe haloalkyle : un groupe alkyle substitué par un ou plusieurs atomes d'halogène identiques ou différents. A titre d'exemples, on peut citer les groupes CF₃, CH₂CF₃, CHF₂, CCl₃.
- un groupe haloalcoxy : un radical -O-alkyle où le groupe alkyle est tel que précédemment défini et substitué par un ou plusieurs atomes d'halogène identiques ou différents. A titre d'exemples, on peut citer les groupes OCF₃, OCHF₂, OCCl₃ ;
- un groupe thioalkyle : un radical S-alkyle où le groupe alkyle est tel que précédemment défini ;
- un groupe aryle : un groupe aromatique mono ou bicyclique comportant de 6 à 10 atomes. A titre d'exemples de groupe aryle, on peut citer les groupes phényle et naphthyle ;
- un groupe hétéroaryle : un groupe aromatique mono ou bicyclique comportant de 5 à 10 atomes dont de 1 à 4 hétéroatomes choisis parmi N, O et S. A titre d'exemples de groupes hétéroaryles monocycliques, on peut citer : pyrrole, furane, thiophène, pyrazole, imidazole, triazole, tétrazole, oxazole, isoxazole, oxadiazole, thiazole, isothiazole, thiadiazole, pyridine, pyrimidine, pyrazine, pyridazine, triazine.
- les atomes de soufre et d'azote peuvent être à l'état oxydé (N-oxide, sulfoxide, sulfone).

Parmi les composés de formule (I) objets de l'invention, un premier groupe de composés est constitué des composés pour lesquels :
R₁ représente un groupe phényle ou un groupe naphtyle, éventuellement substitué par un ou plusieurs atomes ou groupes choisis indépendamment les uns des autres parmi les atomes d'halogène ou groupes (C₁-C₁₀)alkyle, (C₁-C₁₀)alcoxy; nitro, -S(O)₂(C₁-C₁₀-alkyle), halo(C₁-C₁₀)alkyle, cyano, (C₁-C₁₀)thioalkyle, NRaRb; NRcCORd, hydroxy(C₁-C₁₀)alkylène, NRcSO₂Re, CONRaRb, NRcC(O)ORe, SO₂NRaRb, NRaRb(C₁-C₁₀)alkylène ;
Ra et Rb représentent, indépendamment l'un de l'autre, un atome d'hydrogène ou un groupe (C₁-C₁₀)alkyle;
ou Ra et Rb forment ensemble, avec l'atome d'azote qui les porte, un groupe azétidinyle, pyrrolidinyle, morpholinyle ;,
Rc et Rd représentent, indépendamment l'un de l'autre, un atome d'hydrogène ou un groupe (C₁-C₁₀)alkyle;
Re représente un groupe (C₁-C₁₀)alkyle ;
les autres substituants étant tels que définis précédemment.

Parmi les composés de formule (I) objets de l'invention, un deuxième groupe de composés est constitué des composés pour lesquels :
R₁ représente un groupe phényle un groupe naphtyle, éventuellement substitué par un ou plusieurs atomes ou groupes choisis indépendamment les uns des autres parmi les atomes d'halogène, méthyle, méthoxy, nitro, méthylsulfonyle, trifluorométhyle, cyano, méthylthio; pyrrolidinyle, -NHCOCH₃, hydroxyméthyle, -NHSO₂CH₃, -CON(CH₃)₂, -NHC(O)OCH₃, - C(O)NHCH₃, morpholinyle, -NHC(O)-isopropyle, -SO₂N(CH₃)₂, pyrrolidinyl-éthyle ; les autres substituant étant tel que définis précédemment.

Parmi les composés de formule (I) objets de l'invention, un troisième groupe de composés est constitué des composés pour lesquels :
R₁ représente un groupe phényle ou un groupe naphtyle, le groupe phényle étant éventuellement substitué en position 2, 3 ou 4 par un ou plusieurs atomes ou groupes choisis indépendamment les uns des autres parmi les atomes d'halogène, méthyle, méthoxy, nitro, méthylsulfonyle, trifluorométhyle, cyano, méthylthio; pyrrolidinyle,-NHCOCH₃, hydroxyméthyle, -NHSO₂CH₃, -CON(CH₃)₂, -NHC(O)OCH₃, -C(O)NHCH₃, morpholinyle, - NHC(O)-isopropyl, -SO₂N(CH₃)₂, pyrrolidinyl-éthyle ;
les autres substituants étant tels que définis précédemment.

Parmi les composés de formule (I) objets de l'invention, un quatrième groupe de composés est constitué des composés pour lesquels :
X représente 1 ou 2 substituants identiques ou différents l'un de l'autre choisis parmi halogène, (C₁-C₁₀)alkyle, (C₁-C₁₀)alcoxy, le groupe (C₁-C₁₀)alkyle pouvant être éventuellement substitué par un groupe hydroxyle ;
R₂ et X peuvent former ensemble avec les atomes de carbone qui les portent un cycle carboné de 5 ou 6 atomes de carbone ;
les autres substituants étant tels que définis précédemment.

Parmi les composés de formule (I) objets de l'invention, un cinquième groupe de composés est constitué des composés pour lesquels :
X représente 1 ou 2 substituants identiques ou différents l'un de l'autre choisis parmi les atomes d'halogène, les groupes méthyle, méthoxy, hydroxyméthyle;
R₂ et X peuvent former ensemble avec les atomes de carbone qui les portent un cycle carboné de 5 ou 6 carbones ;
les autres substituants étant tels que définis précédemment.

Parmi les composés de formule (I) objets de l'invention, un sixième groupe de composés est constitué des composés pour lesquels :
R₂ et R₃ représentent indépendamment l'un de l'autre un un atome d'hydrogène ou un groupe (C₁-C₁₀)alkyle ;
les autres substituants étant tels que définis précédemment.

Parmi les composés de formule (I) objets de l'invention, un septième groupe de composés est constitué des composés pour lesquels :
R₂ et R₃ représentent indépendamment l'un de l'autre un un atome d'hydrogène ou un groupe méthyle ;
les autres substituants étant tels que définis précédemment.

Parmi les composés de formule (I) objets de l'invention, un huitième groupe de composés est constitué des composés pour lesquels :
R₄ représente un atome d'hydrogène, un groupe (C₁-C₁₀)alkyle, ce groupe étant éventuellement substitué par un groupe Rf ;
Rf représente un groupe (C₁-C₁₀)alcoxy ;
les autres substituants étant tels que définis précédemment.

Parmi les composés de formule (I) objets de l'invention, un neuvième groupe de composés est constitué des composés pour lesquels :
R₄ représente un atome d'hydrogène, méthyle, méthoxyéthyle ;
les autres substituants étant tels que définis précédemment.

Parmi les composés de formule (I) objets de l'invention, un dixième groupe de composés est constitué des composés pour lesquels : la substitution du groupe 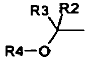 sur le noyau phényle est en position 2, 3 ou 4 ; les substituants des composés de formule (I) étant définis tels que précédemment.

Parmi les composés de formule (I) objets de l'invention, un onzième groupe de composés est constitué des composés pour lesquels :
R₁ représente un groupe phényle ou un groupe naphtyle, éventuellement substitué par un ou plusieurs atomes ou groupes choisis indépendamment les uns des autres parmi les atomes d'halogène ou groupes (C₁-C₁₀)alkyle, (C₁-C₁₀)alcoxy; nitro, -S(O)₂(C₁-C₁₀)-alkyle), halo(C₁-C₁₀)alkyle, cyano, (C₁-C₁₀)thioalkyle, ;NRaRb ; NRcCORd, hydroxy(C₁-C₁₀)alkylène, NRcSO₂Re, CONRaRb, NRcC(O)ORe, SO₂NRaRb, NRaRb(C₁-C₁₀)alkylène ;
Ra et Rb représentent, indépendamment l'un de l'autre, un atome d'hydrogène ou un groupe (C₁-C₁₀)alkyle ;
ou Ra et Rb forment ensemble, avec l'atome d'azote qui les porte, un groupe, pyrrolidine, pipéridinyle, morpholinyle,
Rc et Rd représentent, indépendamment l'un de l'autre, un atome d'hydrogène ou un groupe (C₁-C₁₀)alkyle;
Re représente un groupe (C₁-C₁₀)alkyle ;
X représente 1 ou 2 substituants identiques ou différents l'un de l'autre choisis parmi halogène, (C₁-C₁₀)alkyle, (C₁-C₁₀)alcoxy, le groupe (C₁-C₁₀)alkyle pouvant être éventuellement substitué par un groupe hydroxyle ;
R₂ et X peuvent former ensemble avec les atomes de carbone qui les portent un cycle carboné de 5 ou 6 carbones ;
R₂ et R₃ représentent indépendamment l'un de l'autre un un atome d'hydrogène ou un groupe (C₁-C₁₀)alkyle ;
R₄ représente un atome d'hydrogène, un groupe (C₁-C₁₀)alkyle, ce groupe étant éventuellement substitué par un groupe Rf ;
Rf représente un groupe (C₁-C₁₀)alcoxy ;
la substitution du groupe 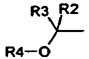 sur le noyau phényle est en position 2, 3 ou 4 ;
à l'état de base ou de sel d'addition à un acide.

Parmi les composés de formule (I) objets de l'invention, un douzième groupe de composés est constitué des composés pour lesquels :
R₁ représente un groupe phényle un groupe naphtyle, éventuellement substitué par un ou plusieurs atomes ou groupes choisis indépendamment les uns des autres parmi les atomes d'halogène, méthyle, méthoxy, nitro, méthylsulfonyle, trifluorométhyle, cyano, méthylthio ;pyrrolidinyle,-NHCOCH₃, hydroxyméthyle, -NHSO₂CH₃, -CON(CH₃)₂, - NHC(O)OCH₃, -C(O)NHCH₃, morpholinyle, -NHC(O)-isopropyle, -SO₂N(CH₃)₂, pyrrolidinyl-éthyle ;
X représente 1 ou 2 substituants identiques ou différents l'un de l'autre choisis parmi les atomes d'hydrogène de fluor, les groupes méthyle, méthoxy, hydroxyméthyle;
R₂ et X peuvent former ensemble avec les atomes de carbone qui les portent un cycle carboné de 5 ou 6 carbones ;
R₂ et R₃ représentent indépendamment l'un de l'autre un un atome d'hydrogène ou un groupe méthyle ;
R₄ représente un atome d'hydrogène, un groupe méthyle, méthoxyéthyle ;
la substitution du groupe 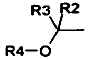 sur le noyau phényle est en position 2, 3 ou 4 ;
à l'état de base ou de sel d'addition à un acide.

Parmi les composés de formule (I) objets de l'invention, un treizième groupe de composés est constitué des composés pour lesquels :
R₁ représente un groupe phényle éventuellement substitué par un halogène, un groupe (C₁-C₁₀)alkyle ou (C₁-C₁₀)alcoxy;
X représente un ou plusieurs halogène, (C₁-C₁₀)alkyle, (C₁-C₁₀)alcoxy, hydroxy(C₁-C₁₀)alkyle;
R₂ et R₃ représentent, indépendamment l'un de l'autre, un atome d'hydrogène ou un groupe (C₁-C₁₀)alkyle;
R₂ et X peuvent former ensemble avec les atomes de carbone qui les portent un cycle carboné de 6 atomes de carbone ;
R₄ représente un atome d'hydrogène,
à l'état de base ou de sel d'addition à un acide.

Parmi les composés de formule (I) objets de l'invention, on peut notamment citer les composés suivants :
- {3-[2-(4-Chlorophényl)imidazo[1,2-a]pyridin-6-yl]-2-fluorophényl}méthanol ;
- 1-{3-[2-(4-Chlorophényl)imidazo[1,2-a]pyridin-6-yl]-2-fluorophényl}éthanol ;
- {3-[2-(4-Chlorophényl)imidazo[1,2-a]pyridin-6-yl]-2,6-difluorophényl}méthanol ;
- 1-{3-[2-(4-Chlorophényl)imidazo[1,2-a]pyridin-6-yl]-2,6-difluorophényl}éthanol ;
- 1-{5-[2-(4-Chlorophényl)imidazo[1,2-a]pyridin-6-yl]-2-fluorophényl}éthanol ;
- {3-[2-(4-Chlorophényl)imidazo[1,2-a]pyridin-6-yl]-5-méthylphényl}méthanol ;
- {3-[2-(4-Chlorophényl)imidazo[1,2-*a*]pyridin-6-yl]-5-méthoxyphényl}méthanol ;
- 7-[2-(4-Chlorophényl)imidazo[1,2-*a*]pyridin-6-yl]-1,2,3,4-tétrahydronaphthalèn-1-ol ;
- 5-[2-(4-Chlorophényl)imidazo[1,2-*a*]pyridin-6-yl]-1,2,3,4-tétrahydronaphthalèn-1-ol ;
- {3-[2-(4-Chlorophényl)imidazo[1,2-*a*]pyridin-6-yl]-4-méthylphényl}méthanol ;
- {5-[2-(4-Chlorophényl)imidazo[1,2-*a*]pyridin-6-yl]-2-fluorophényl}méthanol ;
- {5-[2-(4-Chlorophényl)imidazo[1,2-*a*]pyridin-6-yl]-2-méthylphényl}méthanol ;
- {3-[2-(4-Chlorophényl)imidazo[1,2-*a*]pyridin-6-yl]-2-méthylphényl}méthanol ;
- [2-Fluoro-4-(2-*p*-tolylimidazo[1,2-*a*]pyridin-6-yl)phényl]méthanol ;
- [2-Fluoro-6-(2-*p*-tolylimidazo[1,2-*a*]pyridin-6-yl)phényl]méthanol ;
- {2-Fluoro-6-[2-(3-méthoxyphényl)imidazo[1,2-*a*]pyridin-6-yl]phényl}méthanol ;
- {2-fluoro-4-[2-(3-méthoxyphényl)imidazo[1,2-*a*]pyridin-6-yl]phényl}méthanol et son chlorhydrate ;
- [2-fluoro-4-(2-phénylimidazo[1,2-*a*]pyridin-6-yl)phényl]méthanol et son chlorhydrate ;
- {2-Fluoro-3-[2-(3-méthoxyphényl)imidazo[1,2-*a*]pyridin-6-yl]phényl}méthanol ;
- [2-fluoro-6-(2-phénylimidazo[1,2-*a*]pyridin-6-yl)phényl]méthanol et son chlorhydrate ;
- [2-Fluoro-3-(2+*p*-tolylimidazo[1,2-*a*]pyridin-6-yl)phényl]méthanol ;
- 1-{3-[2-(4-Chlorophényl)imidazo[1,2-*a*]pyridin-6-yl]-4-fluorophényl}éthanol ;
- {3-[2-(4-Chlorophényl)imidazo[1,2-0]pyridin-6-yl]-4-fluorophényl}méthanol ;
- {3-[2-(4-Chlorophényl)imidazo[1,2-*a*]pyridin-6-yl]-4-méthoxyphényl}méthanol ;
- 1-{3-[2-(4-Chlorophényl)imidazo[1,2-*a*]pyridin-6-yl]-4-méthoxyphényl}éthanol ;
- {3-[2-(4-Chlorophényl)imidazo[1,2-*a*]pyridin-6-yl]- 5-(hydroxyméthyl)phényl}méthanol ;
- [2-Fluoro-3-[2-(naphthyl-2-yl)imidazo[1,2-*a*]pyridin-6-yl]phényl]méthanol ;
- [2-Fluoro-3-(2-phénylimidazo[1,2-a]pyridin-6-yl)phényl]méthanol et son chlorhydrate ;
- (+)-1-{3-[2-(4-Chlorophényl)imidazo[1,2-*a*]pyridin-6-yl]-4-fluorophényl}éthanol ;
- (-)-1-{3-[2-(4-Chlorophényl)imidazo[1,2-*a*]pyridin-6-yl]-4-fluorophényl}éthanol ;
- {2-[2-(2,4-Difluorophényl)imidazo[1,2-*a*]pyridin-6-yl]-6-fluorophényl}méthanol ;
- {3-[2-(2,4-Difluorophényl)imidazo[1,2-*a*]pyridin-6-yl]-2,6-difluorophényl}méthanol ;
- 2-{4-Fluoro-3-[2-(3-méthoxyphényl)imidazo[1,2-*a*]pyridin-6-yl]phényl}propan-2-ol ;
- 1-{4-Fluoro-3-[2-(3-méthoxyphényl)imidazo[1,2-*a*]pyridin-6-yl]phényl}éthanol ;
- 2-{3-[2-(4-Chlorophényl)imidazo[1,2-*a*]pyridin-6-yl]-2,6-difluorophényl}propan-2-ol ;
- 1-[4-Fluoro-2-(2-naphthyl-2-ylimidazo[1,2-*a*]pyridin-6-yl)phényl]éthanol ;
- 6-(2,4-Difluoro-3-méthoxyméthylphényl)-2-p-tolylimidazo[1,2-*a*]pyridine ;
- {2-Fluoro-6-[2-(4-nitrophényl)imidazo[1,2-*a*]pyridin-6-yl]phényl}méthanol ;
- {2,6-Difluoro-3-[2-(4-pyrrolidin-1-ylphényl)imidazo[1,2-*a*]pyridin-6-yl]phényl}méthanol ;
- {3-[2-(3,4-Difluorophényl)imidazo[1,2-*a*]pyridin-6-yl]-2,6-difluorophényl}méthanol ;
- {2,6-Difluoro-3-[2-(2-fluorophényl)imidazo[1,2-*a*]pyridin-6-yl]phényl}méthanol ;
- {3-[2-(3-Bromophényl)imidazo[1,2-*a*]pyridin-6-yl]-2,6-difluorophényl}méthanol
- 2,6-Difluoro-3-[2-(4-méthylsulfonylphényl)imidazo[1,2-*a*]pyridin-6-yl]phényl}méthanol ;
- 2-[2-Méthyl-3-(2-phénylimidazo[1,2-*a*]pyridin-6-yl)phényl]propan-2-ol ;
- 7-[2-(2,4-Difluorophényl)imidazo[1,2-*a*]pyridin-6-yl]indan-1-ol ;
- 2-(4-Chlorophényl)-6-[2,4-difluoro-3-[(2-méthoxy-éthyl)oxyméthyl]phényl]imidazo[1,2-*a*]pyridine ;
- 7-[2-(2,4-Difluorophényl)imidazo[1,2-*a*]pyridin-6-yl]-1-méthylindan-1-ol ;
- 2-{3-[2-(4-Chlorophényl)imidazo[1,2-*a*]pyridin-6-yl]-2-méthoxyphényl}propan-2-ol ;
- 1-{3-[2-(4-Chlorophényl)imidazo[1,2-*a*]pyridin-6-yl]-2-méthoxyphényl}éthanol;
- {2-Fluoro-6-[2-(4-trifluorométhylphényl)imidazo[1,2-*a*]pyridin-6-yl]phényl}-méthanol ;
- {2-Fluoro-6-[2-(4-méthoxyphényl)imidazo[1,2-*a*]pyridin-6-yl]phényl}méthanol ;
- {2-Fluoro-6-[2-(4-pyrrolidin-1-ylphényl)imidazo[1,2-*a*]pyridin-6-yl]phényl}méthanol ;
- {2-Fluoro-6-[2-(4-fluorophényl)imidazo[1,2-*a*]pyridin-6-yl]phényl}méthanol ;
- {2-[2-(3,4-Difluorophényl)imidazo[1,2-*a*]pyridin-6-yl]-6-fluorophényl}méthanol ;
- 3-[6-(3-Fluoro-2-hydroxyméthylphényl)imidazo[1,2-*a*]pyridin-2-yl]benzonitrile;
- {2-Fluoro-6-[2-(2-fluorophényl)imidazo[1,2-*a*]pyridin-6-yl]phényl}méthanol ;
- {2-[2-(3-Bromophényl)imidazo[1,2-*a*]pyridin-6-yl]-6-fluorophényl}méthanol ;
- {2-[2-(4-Chloro-3-méthylphényl)imidazo[1,2-*a*]pyridin-6-yl]-6-fluorophényl}méthanol ;
- {2-[2-(3-Chloro-4-méthylphényl)imidazo[1,2-*a*]pyridin-6-yl]-6-fluorophényl}méthanol ;
- {2-Fluoro-6-[2-(4-méthylsulfonylphényl)imidazo[1,2-*a*]pyridin-6-yl]phényl}méthanol ;
- {2,6-Difluoro-3-[2-(4-méthoxyphényl)imidazo[1,2-*a*]pyridin-6-yl]phényl}méthanol ;
- {2,6-Difluoro-3-[2-(4-fluorophényl)imidazo[1,2-*a*]pyridin-6-yl]phényl}méthanol ;
- 3-[6-(2,4-Difluoro-3-hydroxyméthylphényl)imidazo[1,2-*a*]pyridin-2-yl]benzonitrile ;
- {2,6-Difluoro-3-[2-(4-chloro-3-méthylphényl)imidazo[1,2-*a*]pyridin-6-yl]phényl}méthanol ;
- {2,6-Difluoro-3-[2-(3-chloro-4-méthylphényl)imidazo[1,2-*a*]pyridin-6-yl]phényl}méthanol ;
- {2,6-Difluoro-3-[2-(4-méthylthiophényl)imidazo[1,2-*a*]pyridin-6-yl]phényl}méthanol ;
- 2-[2-Chloro-3-[2-(4-chlorophényl)imidazo[1,2-*a*]pyridin-6-yl)phényl]propan-2-ol ;
- 1-[2-Chloro-3-[2-(4-Chlorophényl)imidazo[1,2-*a*]pyridin-6-yl)phényl]éthanol ;
- {2-[2-(4-Chlorophényl)imidazo[1,2-*a*]pyridin-6-yl]-6-fluorophényl}méthanol ;
- 2-(4-Chlorophényl)-6-[3-fluoro-2-[(2-méthoxy-éthyl)oxyméthyl]phényl]imidazo[1,2-*a*]pyridine ;
- *N*-{3-[6-(3-Fluoro-2-hydroxyméthylphényl)imidazo[1,2-*a*]pyridin-2-yl]phényl}acétamide ;;
- {4-[6-(3-Fluoro-2-hydroxyméthylphényl)imidazo[1,2-*a*]pyridin-2-yl]phényl}méthanol
- {3-[6-(3-Fluoro-2-hydroxyméthylphényl)imidazo[1,2-*a*]pyridin-2-yl]phényl}méthanol ;
- *N*-{4-[6-(3-Fluoro-2-hydroxyméthylphényl)imidazo[1,2-*a*]pyridin-2-yl]phényl}méthanesulfonamide ;
- 4-[6-(3-Fluoro-2-hydroxyméthylphényl)imidazo[1,2-*a*]pyridin-2-yl]-*N,N-*diméthylbenzamide ;
- {4-[6-(3-Fluoro-2-hydroxyméthylphényl)imidazo[1,2-*a*]pyridin-2-yl]phényl}carbamate de méthyle ;
- 4-[6-(3-Fluoro-2-hydroxyméthylphényl)imidazo[1,2-*a*]pyridin-2-yl]-*N-*méthylbenzamide ;
- N-{4-[6-(3-Fluoro-2-hydroxyméthylphényl)imidazo[1,2-*a*]pyridin-2-yl]phényl}acétamide ;
- {2-Fluoro-6-[2-(4-morpholin-4-ylphényl)imidazo[1,2-*a*]pyridin-6-yl]phényl}méthanol ;
- 3-[6-(3-Fluoro-2-hydroxyméthylphényl)imidazo[1,2-*a*]pyridin-2-yl]-*N,N-*diméthylbenzènesulfonamide ;
- (2-Fluoro-6-{2-[4-(1-pyrrolidin-1-yléthyl)phényl]imidazo[1,2-*a*]pyridin-6-yl}phényl)méthanol ;
- 2-Fluoro-4-[6-(3-fluoro-2-hydroxyméthylphényl)imidazo[1,2-*a*]pyridin-2-yl]-*N*,*N-*diméthylbenzamide ;
- N-{3-[6-(2,4-Difluoro-3-hydroxyméthylphényl)imidazo[1,2-*a*]pyridin-2-yl]phényl}acétamide ;
- {4-[6-(2,4-Difluoro-3-hydroxyméthylphényl)imidazo[1,2-*a*]pyridin-2-yl]phényl}méthanol;
- {3-[6-(2,4-Difluoro-3-hydroxyméthylphényl)imidazo[1,2-*a*]pyridin-2-yl]phényl}méthanol;
- 4-[6-(2,4-Difluoro-3-hydroxyméthylphényl)imidazo[1,2-*a*]pyridin-2-yl]-*N*,*N-*diméthylbenzamide ;
- {4-[6-(2,4-Difluoro-3-hydroxyméthylphényl)imidazo[1,2-*a*]pyridin-2-yl]phényl}carbamate de méthyle ;
- 4-[6-(2,4-Difluoro-3-hydroxyméthylphényl)imidazo[1,2-*a*]pyridin-2-yl]-*N-*méthylbenzamide ;
- N-{4-[6-(2,4-Difluoro-3-hydroxyméthylphényl)imidazo[1,2-*a*]pyridin-2-yl]phényl}acétamide ;
- {2,6-Difluoro-3-[2-(4-morpholin-4-yl-phényl)imidazo[1,2-*a*]pyridin-6-yl]-phényl}méthanol ;
- *N*-{3-[6-(2,4-Difluoro-3-hydroxyméthylphényl)imidazo[1,2-*a*]pyridin-2-yl]phényl}isobutyramide ;
- 3-[6-(2,4-Difluoro-3-hydroxyméthylphényl)imidazo[1,2-*a*]pyridin-2-yl]-*N*,*N* diméthylbenzènesulfonamide ;
- (2,6-Difluoro-3-{2-[4-(1-pyrrolidin-1-yl-éthyl)phényl]imidazo[1,2-*a*]pyridin-6-yl}phényl)méthanol ;
- 4-[6-(2,4-Difluoro-3-hydroxyméthylphényl)imidazo[1,2-*a*]pyridin-2-yl]-2-fluoro-*N*,*N*-diméthylbenzamide.

Conformément à l'invention, on peut préparer les composés de formule générale (I) selon le procédé décrit dans le schéma 1.

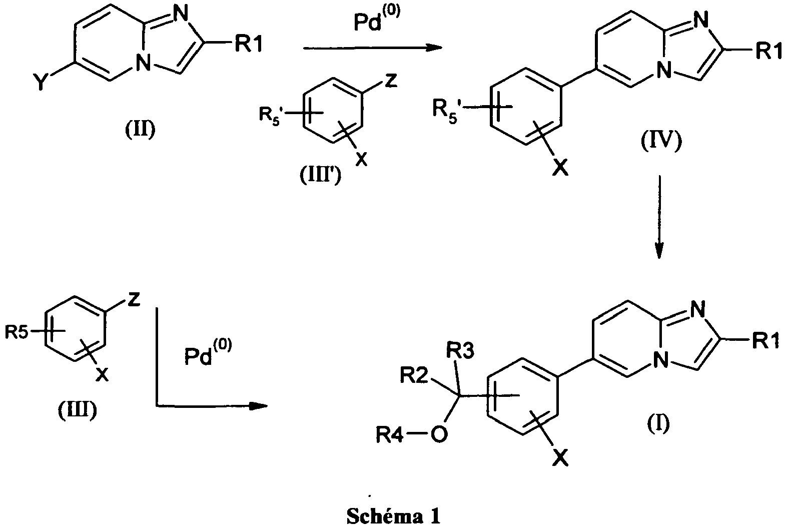

On peut préparer les composés de l'invention suivant le schéma 1 par une réaction de couplage, catalysée par un métal tel que le palladium, entre une imidazopyridine de formule générale (II), dans laquelle R1 est défini comme précédemment et Y représente un atome d'halogène ou un dérivé de bore, et un dérivé de formule générale (III) dans laquelle X est défini comme précédemment, Z représente un dérivé de bore ou d'étain si Y représente un atome d'halogène, ou bien un atome d'halogène si Y représente un dérivé de bore, et R5 représente le groupe 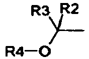 pour obtenir les composés de formules générale (I), par exemple, selon la méthode décrite par A. Gueiffier dans Helv. Chim. Acta 2001, 84, 3610-3615.

On peut aussi préparer les composés de l'invention suivant le schéma 1 par une réaction de couplage, catalysée par un métal tel que le palladium, entre une imidazopyridine de formule générale (II), dans laquelle R1 est défini comme précédemment et Y représente un atome d'halogène ou un dérivé de bore, et un dérivé de formule générale (III') dans laquelle X est défini comme précédemment, Z représente un dérivé de bore ou d'étain si Y représente un atome d'halogène, ou bien un atome d'halogène si Y représente un dérivé de bore, et R5' représente un dérivé carbonylé R₂CO, dans lequel R2 est défini comme précédemment ou bien R5' représente un carboxylate d'alkyle, pour obtenir les composés de formule générale (IV), par exemple, selon la méthode décrite par A. Gueiffier dans Helv. Chim. Acta 2001, 84, 3610-3615.

Ensuite, on peut transformer les composés de formule générale (IV) en composés de formule générale (I), pour lesquels R4 représente un atome d'hydrogène, par action d'un dérivé organométallique tel qu'un organomagnésien, par exemple R₃MgBr dans lequel R3 est défini comme précédemment, ou par réduction du groupement carbonyle au moyen d'un hydrure métallique, par exemple le borohydrure de sodium ou un de ses dérivés, ou toute autre méthode connue de l'Homme du métier.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier. En particulier, les imidazopyridines de formule générale (II), dans laquelle Y représente un dérivé de bore, peuvent être obtenues, par exemple, selon la méthode décrite par E. DiMauro dans J. Org. Chem. 2006, 71, 3959.

Conformément à l'invention, on peut également préparer les composés de formule générale (I), selon le procédé décrit dans le schéma 2.

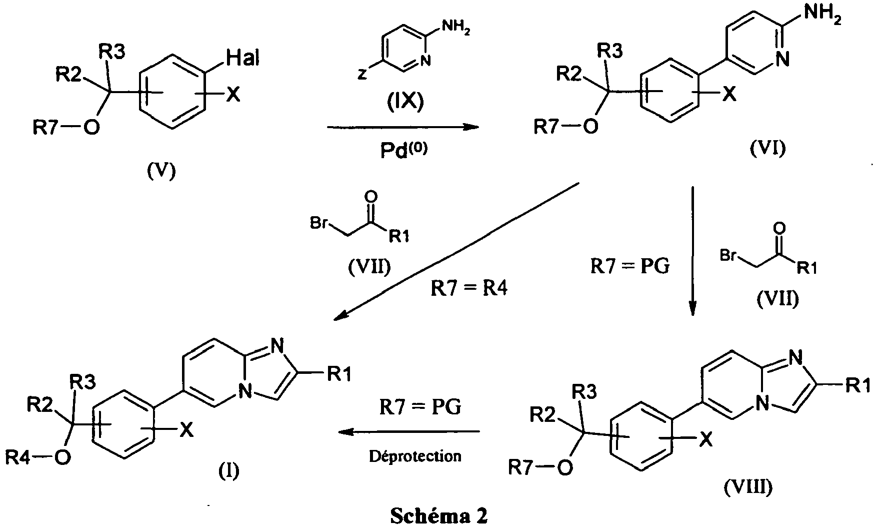

On peut préparer les composés de l'invention suivant le schéma 2 par une réaction de condensation entre une aminopyridine de formule générale (VI), dans laquelle R2, R3 et X sont définis comme précédemment, et R7 représente R4 ou un groupement protecteur (PG) de fonction hydroxyle, et une bromocétone de formule générale (VII), dans laquelle R1 est défini comme précédemment, pour obtenir une imidazopyridine de formule générale (I), dans laquelle R1, R2, R3, R4 et X sont définis comme précédemment, ou une imidazopyridine de formule générale (VIII), dans laquelle R7 représente un groupe protecteur PG, par exemple selon la méthode décrite par M. Fisher dans J. Med. Chem 1972, 15, 982. On peut citer comme groupement protecteur de fonction hydroxyle, ceux décrits par exemple par T. Greene dans « Protective Groups in Organic Synthesis » (Wiley Interscience*)*, par exemple un groupe terbutyldiméthylsilyle.

Enfin, lorsque R7 représente un groupement protecteur (PG) de fonction hydroxyle, les composés de formule générale (VIII) sont soumis à une réaction de déprotection, comme décrit par exemple par T. Greene dans « Protective Groups in Organic Synthesis » (Wiley Interscience*)*, pour obtenir les composés de formule générale (I), dans laquelle R4 représente l'atome d'hydrogène.

Les composés de formule générale (VI) peuvent être obtenus par une réaction de couplage, catalysée par un métal tel que le palladium, entre un dérivé halogéné de formule générale (V) dans laquelle R2, R3, X et Hal sont définis comme précédemment, et R7 représente R4 ou un groupement protecteur (PG) de fonction hydroxyle et une 2-aminopyridine (IX) substituée par un groupement Z qui représente un dérivé de bore ou d'étain telle que, par exemple, la 5-(4,4,5,5-tétraméthyl-1,3,2-dioxaborolan-2-yl)pyridin-2-ylamine.

Conformément à l'invention, on peut également préparer les composés de formule générale (I), selon le procédé décrit dans le schéma 3.

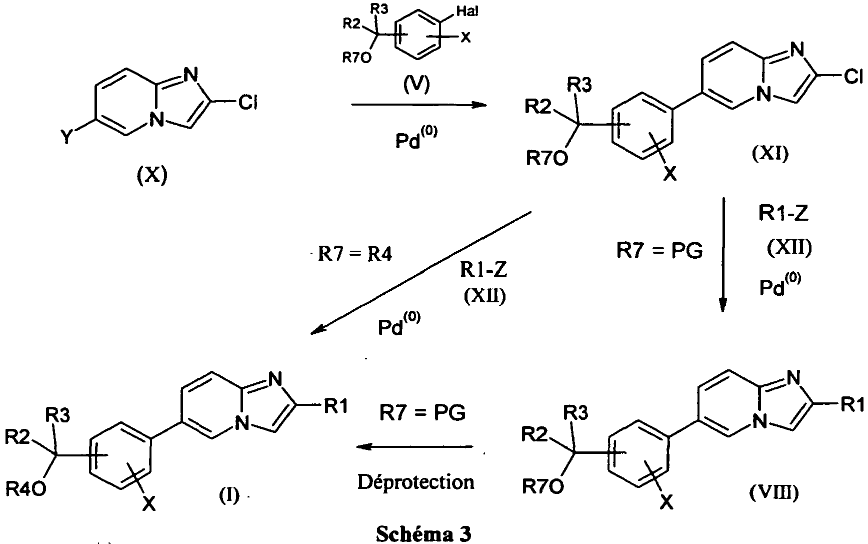

On peut préparer les composés de l'invention suivant le schéma 3 par une réaction de couplage, catalysée par un métal tel que le palladium, entre une imidazopyridine de formule générale (XI), dans laquelle X, R2 et R3 sont définis comme précédemment et R7 représente le groupe R4 et un dérivé de formule générale (XII), dans laquelle R1 est défini comme précédemment et Z représente un dérivé de bore ou d'étain, pour obtenir les composés de formule générale (I), par exemple, selon la méthode décrite par S. Buchwald dans J.A.C.S. 2005, 127, 4685.

On peut aussi préparer les composés de l'invention suivant le schéma 3 par une réaction de couplage, catalysée par un métal tel que le palladium, entre une imidazopyridine de formule générale (XI), dans laquelle X, R2 et R3 sont définis comme précédemment et R7 représente un groupement protecteur (PG) de fonction hydroxyle, tel que décrit par exemple par T. Greene dans « Protective Groups in Organic Synthesis » (Wiley Interscience*),* et un dérivé de formule générale (XII) dans laquelle R1 est défini comme précédemment et Z représente un dérivé de bore ou d'étain, pour obtenir les composés de formule générale (VIII), par exemple, selon la méthode décrite par S. Buchwald dans J.A.C.S. 2005, 127, 4685. Ensuite, on peut transformer les composés de formule générale (VIII) en composés de formule générale (I), dans laquelle R4 représente un atome d'hydrogène, en réalisant une réaction de déprotection telle que décrite par exemple par T. Greene dans « Protective Groups in Organic Synthesis » (Wiley Interscience*),* ou par toute autre méthode connue de l'Homme du métier.

Les imidazopyridines de formule générale (XI) peuvent être obtenues par une réaction de couplage, catalysée par un métal tel que le palladium, entre une imidazopyridine de formule générale (X), dans laquelle Y représente un dérivé de bore, et un dérivé de formule générale (V) dans laquelle R2, R3 et X sont définis comme précédemment, R7 représente le groupe R4 ou un groupement protecteur (PG) de fonction hydroxyle, et Hal représente un atome d'halogène différent du chlore, par exemple, selon la méthode décrite par A. Gueiffier dans Helv. Chim. Acta 2001, 84, 3610-3615.

Dans le schéma 3, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier. En particulier, les imidazopyridines chlorées de formule générale (X) peuvent être obtenues, par exemple, selon la méthode décrite par C. Townsend dans Syn. Commun. 1997, 27, 1763-1765.

Les produits de formule (I), peuvent être soumis, si désiré et si nécessaire, à toutes réactions connues de l'homme de l'art, dans un ordre quelconque, pour être transformés en d'autres produits de formule (I).

A titre d'exemples de réactions, on peut citer : les réactions d'estérification ou d'amidification de fonction acide, les réactions de carbamoylation, les réactions d'hydrolyse de fonction ester, les réactions de transformation de fonction hydroxyle en fonction alcoxy, les réactions de couplage catalysées par un métal de transition, les réactions de protection des fonctions réactives, les réaction d'élimination des groupements protecteurs que peuvent porter les fonctions réactives protégées, les réactions de salification par un acide minéral ou organique ou par une base pour obtenir le sel correspondant, les réactions de dédoublement des formes racémiques en énantiomères, lesdits produits de formule (I) ainsi obtenus étant le cas échéant sous toutes les formes isomères possibles racémiques, énantiomères et diastéréoisomères.

L'invention, selon un autre de ses aspects, a également pour objet les composés de formules (IIa), (IIb), (IIc), (IId), (IIe), (IIf), (IIg),.(VIa), (VIb), (Xa), (XIa) et (XIb). Ces composés sont utiles comme intermédiaires de synthèse des composés de formule (I).

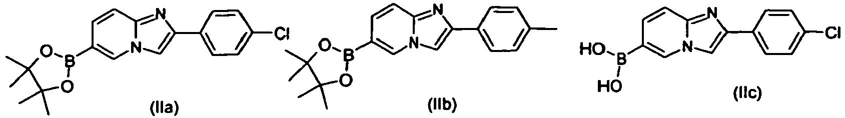

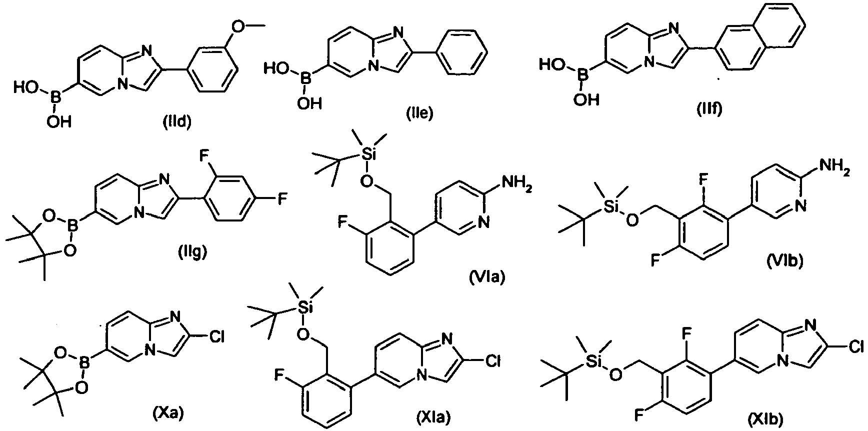

Les composés de formules (IIa), (IIb), (IIc), (IId), (IIe), (IIf) et (IIg) peuvent être préparés en une étape (esters boroniques) ou deux étapes (acides boroniques), par exemple selon le procédé décrit dans les exemples N°4 et N° 6. Dans une première étape, on peut effectuer une condensation entre une aminopyridine substituée par un dérivé de bore telle que, par exemple, une 5-(4,4,5,5-tétraméthyl-1,3,2-dioxaborolan-2-yl)pyridin-2-ylamine et une alphabromocétone telle qu'une 2-bromo-1-(aryl)éthanone, par exemple dans un solvant tel que le n-propanol en présence d'une base telle que, par exemple, l'hydrogénocarbonate de sodium pour obtenir les esters boronates correspondants. Ensuite, dans une deuxième étape, on hydrolyse les esters boroniques en acides boroniques correspondants, par exemple dans un mélange d'acétone, d'eau et d'acide chlorhydrique.

Les composés de formules (VIa) et (VIb) peuvent être préparés en une étape, par exemple selon le procédé décrit dans les exemples N°13 et N°14. On peut effectuer une réaction de couplage catalysée par un métal tel que le palladium entre un dérivé de tertbutyldiméthyloxyméthylbromobenzène et une aminopyridine substituée par un dérivé de bore telle que, par exemple, une 5-(4,4,5,5-tétraméthyl-1,3,2-dioxaborolan-2-yl)pyridin-2-ylamine.

Le composé de formule (Xa) peut être préparé, par exemple, selon le procédé décrit dans l'exemple N° 17 Dans une première étape, on peut effectuer une condensation entre une aminopyridine subsituée par un dérivé de bore telle que, par exemple, la 5-(4,4,5,5-tétraméthyl-1,3,2-dioxaborolan-2-yl)pyridin-2-ylamine et le 2-bromoacétate d'éthyle. Dans une deuxième étape, le composé est soumis à une réaction de cyclisation et de chloration en présence d'un agent chlorant tel que l'oxychlorure de phosphore qui conduit au composé (Xa).

Les composés de formule (XIa), et (XIb) peuvent être préparés par une réaction de couplage, catalysée par un métal, tel que le palladium, entre par exemple, le composé (Xa) et un dérivé de tertbutyldiméthyloxyméthylbromobenzène, tel que décrit dans les exemples N° 17 et 18

Les composés de formules (IIa), (IIb), (IIc), (IId), (IIe), (IIf), (IIg), (VIa), (VIb), (Xa), (XIa) et (XIb) ont été préparés à l'état de poudre ou d'huile, à l'état de base ou de sel d'addition à un acide. Le tableau 1 rassemble quelques données physicochimiques de ces intermédiaires.

Dans ce tableau, la colonne « Set/base », « - » représente un composé sous forme de base libre, alors que « HCl » représente un composé sous forme de chlorhydrate et le rapport entre parenthèses est le rapport (acide : base).

**Tableau 1**

| N° | R.M.N. ¹H (DMSO-d6, δ ppm) ; M+H | Sel / base |
|---|---|---|
| (IIa) | 1,35 (s, 12H) ; 7,35 (d, 1H) ; de 7,5 à 7,6 (m, 3H) ; 7,95 (d, 2H) ; 8,45 (d, 1H) ; 7,85 (s, 1H). M+H = 355. | - |
| (IIb) | 1,45 (s, 12H) ; 2,45 (s, 3H) ; 7,3 (d, 2H) ; de 7,5 à 7,7 (m, 2H) ; de 7,85 à 8 (m, 3H) ; 8,6 (s, 1H) ; M+H = 335 | - |
| (IIc) | De 7,6 à 7,75 (m, 2H) ; 7,95 (m, 1H) ; de 8,05 à 8,15 (m, 2H) ; 8,2 (m, 1H) ; 8,9 (s, 1H) ; 9,1 (s, 1H). M+H = 273. | HCl (1:1) |
| (IId) | 3,75 (s, 3H) ; 6,95 (d, 1H) ; de 7,3 à 7,65 (m, 3H) ; 7,8 (d, 1H) ; 8,05 (d, 1H) ; 8,75 (s, 1H) ; 8,9 (s, 1H). M+H = 325 | HCl (1:1) |
| (IIe) | 7,55 (m, 1H) ; 7,6 (m, 2H) ; 8,0 (m, 1H) ; 8,1 (m, 2H) ; 8,25 (d, 1H) ; 8,9 (s, 1H) ; 9,1 (s, 1H). M+H = 275 | HCl (1:1) |
| (IIf) | 7,65 (m, 2H) ; de 7,95 à 8,05 (m, 3H) ; 8,15 (m,2H) ; 8,25 (d, 1H) ; 8,7 (s, 1H) ; 9,0 (s, 1H) ; 9,15 (m, 1H). M+H = 325. | HCl (1:1) |
| (IIg) | 1,35 (s, 12H) ; 7,25 (t, 1H) ; de 7,35 à 7,45 (m, 2H) ; 7,6 (d, 1H) ; de 8,25 à 8,35 (m, 1H) ; 8,45 (s, 1H) ; 8,9 (s, 1H) M+ = 356. | - |
| (VIa) | 0 (s, 6H) ; 0,85 (s, 9H) ; 4,5 (s, 2H) ; 6,05 (s, 2H) ; 6,45 (d, 1H) ; de 7,05 à 7,15 (m, 2H) ; de 7,3 à 7,4 (m, 1H) ; de 7,45 à 7,5 (m, 1H) ; 8,0 (d, 1H). M+H = 333 | - |
| (VIb) | (CDCl3) : 0 (s, 6H) ; 0,8 (s, 9H) ; 4,4 (s, 2H) ; 6,05 (s, 2H) ; 6,45 (d, 1H) ; 7,05 (t, 1H) ; de 7,35 à 7,45 (m, 2H) ; 8,0 (s, 1H). M+H = 351 | - |
| (Xa) | 1,35 (m, 12H) ; 7,4 (d, 1H) ; 7,5 (d, 1H) ; 8,1 (s, 1H) ; 8,85 (s, 1H) ; M+H = 279 ; PF = 115 - 120°C. | - |
| (XIa) | (CDCl3) : 0 (s, 6H) ; 0,85 (s, 9H) ; 4,5 (s, 2H) ; de 7,05à 7,1 (m, 2H) ; de 7,25 à 7,3 (m, 2H) ; 7,4 (s, 1H) ; 7,45 (s, 1H) ; 8,3 (s, 1H). M+H = 391. | - |
| (XIb) | 0,0 (s, 6H) ; 0,8 (s, 9H) ; 4,7 (s, 2H) ; 7,15 (t, 1H) ; 7,4 (d, 1H) ; de 7,5 à 7,6 (m, 2H) ; 8,0 (s, 1H) ; 8,65 (s, 1H). M+H = 409. | - |

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau 2 ci-après, qui illustre les structures chimiques de quelques composés selon l'invention.

La nomenclature employée est la nomenclature suivant les recommandations IUPAC (International Union of Pure and Applied Chemistry).

### Exemple 1 : {3-[2-(4-Chlorophényl)imidazo[1,2-a]pyridin-6-yl]-2 fluorophényl} méthanol (composé 1 du tableau)

### 1.1 6-Bromo-2-(4-chlorophényl)imidazo[1,2-a]pyridine

On place dans un ballon 2,33 g de 5-bromopyridin-2-ylamine, 3,14 g de 2-bromo-1-(4-chlorophényl)éthanone dans 110 ml de n-propanol. On ajoute 1,58 g d'hydrogénocarbonate de sodium. On chauffe à 80°C durant 16 h, laisse refroidir à température ambiante et ajoute 400 ml d'eau. Le précipité est recueilli par filtration, on le lave à l'eau et le sèche à l'étuve sous pression réduite. On obtient 2,89 g de composé. Spectre RMN 1H (DMSO-d6, δ en ppm) : 7,4 (d, 1H) ; 7,5 (d, 2H) ; 7,6 (d, 1H) ; 8,0 (d, 2H) ; 8,4 (s, 1H) ; 8,9 (s, 1H). M+H = 308.

### 3-[2-(4-Chlorophényl)imidazo[1,2-a]pyridin-6-yl]-2-fluorobenzaldéhyde

Sous un courant d'argon, 500 mg de 6-bromo-2-(4-chlorophényl)imidazo[1,2-a]pyridine, 473 mg d'acide 2-fluoro-3-formylbenzèneboronique et 93 mg de tétrakis(triphénylphosphine)palladium sont placés dans un ballon contenant un mélange de 5 ml d'acétonitrile, 5 ml de toluène et 6 ml d'une solution 2M de carbonate de sodium préalablement dégazé sous courant d'argon. Le mélange réactionnel est chauffé à 70°C pendant 21h. Après refroidissement, le mélange réactionnel est dilué à l'acétate d'éthyle et l'eau puis la phase organique est séparée, séchée et concentrée sous pression réduite. Le résidu est purifié par chromatographie sur gel de silice en éluant par un mélange dichlorométhane/acétate d'éthyle 98/02. On obtient 324 mg de composé.
Spectre RMN 1H (DMSO-d6, δ en ppm) : de 7,45 à 7,55 (m, 4H) ; 7,7 (d, 1H) ; 7,9 (m, 1H) ; de 7,95 à 8,05 (m, 3H) ; 8,5 (s, 1H) ; 8,9 (s, 1H) ; 10,35 (s, 1H). M+H = 351.

### 1.3 {3-[2-(4-Chlorophényl)imidazo[1,2-a]pyridin-6-yl]-2-fluorophényl}méthanol

A 150 mg de 3-[2-(4-chlorophényl)imidazo[1,2-a]pyridin-6-yl]-2-fluorobenzaldéhyde dissous dans 20 ml de méthanol, on ajoute par fractions 162 mg de borohydrure de sodium. Le mélange est ensuite agité à température ambiante pendant 2 heures puis le solvant est évaporé sous pression réduite. Le résidu est repris entre l'eau et le dichlorométhane, la phase organique est séparée, séchée sur sulfate de sodium et concentrée sous pression réduite. Le résidu est purifié par chromatographie sur gel de silice en éluant par un mélange dichlorométhane/méthanol 98/02. On obtient 87 mg de composé.
PF = 200 - 202°C. Spectre RMN 1H (DMSO-d6, δ en ppm) : 4,65 (d, 2H) ; 5,35 (t, 1H) ; 7,35 (t, 1H) ; de 7,4 à 7,6 (m, 5H) ; 7,7 (d, 1H) ; 8,0 (d, 2H) ; 8,5 (s, 1H) ; 8,8 (s, 1H). M+H = 353.

### Exemple 2 : 1-{3-[2-(4-Chlorophényl)imidazo[1,2-a]pyridin-6-yl]-2-fluorophényl}éthanol (composé 2 du tableau)

Sous courant d'argon, 170 mg de 3-[2-(4-chlorophényl)imidazo[1,2-a]pyridin-6-yl]-2-fluorobenzaldéhyde (composé obtenu selon le protocole décrit dans l'exemple 1.2) est placé dans un ballon et dissous dans 30 ml de tétrahydrofurane. On refroidit à 0°C par un bain de glace et ajoute goutte à goutte 2,60 ml d'une solution préalablement titrée à 0,56 M de bromure de méthylmagnésium dans l'éther dibutylique. On laisse agiter dans le bain de glace pendant une heure, puis ajoute 5 ml d'une solution aqueuse saturée de chlorure d'ammonium. La phase organique est séparée, séchée sur sulfate de sodium et le solvant est évaporé sous pression réduite. Le résidu est purifié par chromatographie sur gel de silice en éluant par un mélange dichlorométhane/méthanol. On obtient 64 mg de composé. PF = 193 - 195°C. Spectre RMN 1H (DMSO-d6, δ en ppm) : 1,4 (d, 3H) ; 5,1 (m, 1H) ; 5,35 (d, 1H) ; 7,35 (m, 1H) ; de 7,45 à 7,65 (m, 5H) ; 7,7 (d, 1H) ; 8,05 (d, 2H) ; 8,5 (s, 1H) ; 8,75 (s, 1H). M+H = 367.

### Exemple 3 : {3-[2-(4-Chloropbényl)imidazo[1,2-a]pyridin-6-yl]-2,6-difluorophényl} méthanol (composé 3 du tableau)

### 3-[2-(4-Chlorophényl)imidazo[1,2-a]pyridin-6-yl]-2,6-difluorobenzaldéhyde

Sous un courant d'argon, 500 mg de 6-bromo-2-(4-chlorophényl)imidazo[1,2-a]pyridine (composé obtenu selon le protocole décrit dans l'exemple 1.1), 364 mg d'acide 2,4-difluoro-3-formylbenzèneboronique et 93 mg de tétrakis(triphénylphosphine)palladium sont placés dans un ballon contenant un mélange de 5 ml d'acétonitrile, 5 ml de toluène et 6 ml d'une solution 2M de carbonate de sodium préalablement dégazé sous courant d'argon. Au bout de 24h de chauffage à 75°C, on ajoute 60 mg d'acide 2,4-difluoro-3-formylbenzèneboronique, 18 mg de catalyseur et un mélange de 2 ml d'acétonitrile, 2 ml de toluène et 2 ml d'une solution 2M de carbonate de sodium. On continue de chauffer 2 heures à 75°C. On laisse revenir le mélange réactionnel à température ambiante et on le dilue à l'acétate d'éthyle et l'eau. La phase organique est ensuite séparée, séchée et le solvant est concentré sous pression réduite. Le résidu est purifié par chromatographie sur gel de silice en éluant par un mélange dichlorométhane/acétate d'éthyle. On obtient 340 mg de composé. Spectre RMN 1H (DMSO-d6, δ en ppm) : de 7,4 à 7,6 (m, 4H) ; 7,7 (d, 1H); de 8,0 à 8,1 (m, 3H) ; 8,5 (s, 1H) ; 8,85 (s, 1H) ; 10,35 (s, 1H). M+H = 369.

### 3.2 {3-[2-(4-Chlorophényl)imidazo[1,2-a]pyridin-6-yl]-2,6-difluorophényl}méthanol

A 150 mg de 3-[2-(4-chlorophényl)imidazo[1,2-a]pyridin-6-yl]-2,6-difluorobenzaldéhyde dissous dans 20 ml de méthanol, on ajoute par fractions 154 mg de borohydrure de sodium. Le mélange est ensuite agité à température ambiante pendant 3 heures puis le solvant est évaporé sous pression réduite. Le résidu est repris entre l'eau et le dichlorométhane puis la phase organique est séparée, séchée sur sulfate de sodium et concentrée sous pression réduite. Le résidu est trituré dans le pentane et recueilli par filtration, puis séché à l'étuve sous pression réduite. On obtient 67 mg de composé.
PF = 214 - 216°C. Spectre RMN 1H (DMSO-d6, δ en ppm) : 4,6 (d, 2H) ; 5,3 (m, 1H) ; 7,25 (m, 1H) ; 7,45 (d, 1H) ; 7,55 (d, 2H) ; de 7,6 à 7,7 (m, 2H) ; 8,0 (d, 2H) ; 8,5 (s, 1H) ; 8,75 (s, 1H). M+H = 371.

### Exemple 4 : 7-[2-(4-Chlorophényl)imidazo[1,2-a]pyridin-6-yl]-1,2,3,4-tétrahydronaphthalèn-1-ol (composé 8 du tableau)

### 2-(4-Chloropbényl)-6-(4,4,5,5-tétraméthyl-1,3,2-dioxaborolan-2-yl)imidazo[1,2-a]pyridine

On place dans un ballon 5,0 g de 5-(4,4,5,5-tétraméthyl-1,3,2-dioxaborolan-2-yl)pyridin-2-ylamine, 5,30 g de 2-bromo-1-(4-chlorophényl)éthanone dans 150 ml de n-propanol. On ajoute 2,67 g d'hydrogénocarbonate de sodium. On chauffe à 80°C pendant 16h. Après refroidissement, le mélange réactionnel est concentré sous pression réduite. On obtient 10,93 g de composé utilisé tel quel dans les étapes suivantes.
Spectre RMN 1H (DMSO-d6, δ en ppm) : 1,35 (s, 12H) ; 7,35 (d, 1H) ; de 7,5 à 7,6 (m, 3H) ; 7,95 (d, 2H) ; 8,45 (d, 1 H) ; 7,85 (s, 1H). M+H = 355.

### 4.2 Chlorhydrate (1 :1) d'acide 2-(4-chlorophényl)imidazo[1,2-a]pyridine-6-boronique

7,93 g de 2-(4-chlorophényl)-6-(4,4,5,5-tétraméthyl-1,3,2-dioxaborolan-2-yl)imidazo[1,2-a]pyridine sont dissous dans 200 ml d'acétone et 100 ml d'eau ; on y ajoute, goutte à goutte et sous agitation, 223 ml d'acide chlorhydrique 1N et agite à température ambiante pendant 16h. Le mélange réactionnel est ensuite concentré sous pression réduite. On obtient 4,78 g de composé utilisé tel quel dans les étapes suivantes.
Spectre RMN 1H (DMSO-d6, δ en ppm) : de 7,6 à 7,75 (m, 2H) ; 7,95 (m, 1H) ; de 8,05 à 8,15 (m, 2H) ; 8,2 (m, 1H) ; 8,9 (s, 1H) ; 9,1 (s, 1 H). M+H = 273.

### 4.3 7-[2-(4-Chlorophényl)imidazo[1,2-a]pyridin-6-yl]-3,4-dihydro-2H-naphthalèn-1-one

5 ml d'acétonitrile, 5ml de toluène et 6 ml d'une solution 2M de carbonate de sodium sont introduits dans un ballon et dégazés sous courant d'argon. On ajoute 400 mg d'acide 2-(4-chlorophényl)imidazo[1,2-a]pyridine-6-boronique, 320 mg de 7-bromo-3,4-dihydro-2*H*-naphthalèn-1-one et 75 mg de tétrakis(triphénylphosphine)palladium. On chauffe à 75°C pendant 16h puis laisse revenir à température ambiante et on reprend entre l'acétate d'éthyle et l'eau. La phase organique est séparée, séchée et le solvant est concentré sous pression réduite. Le résidu est purifié par chromatographie sur gel de silice en éluant avec un mélange dichlorométhane/acétone. On obtient 238 mg de composé.
Spectre RMN 1H (DMSO-d6, δ en ppm) : 2,05 (m, 2H); 2,65 (m, 2H); 3,0 (m, 2H) ; 7,55 (m, 3H); de 7,6 à 7,75 (m, 2H); 7,95 (m, 1H) ; 8,05 (d, 2H) ; 8,2 (s, 1H); 8,45 (s, 1H) ; 9,0 (s, 1H). M+H = 373.

### 4.4 7-[2-(4-Chlorophényl)imidazo[1,2-a]pyridin-6-yl]-1,2,3,4-tétrabydronaphthalèn-1-ol

A 238 mg de 7-[2-(4-chlorophényl)imidazo[1,2-*a*]pyridin-6-yl]-3,4-dihydro-2*H-*naphthalèn-1-one dissous dans 25 ml de méthanol, on ajoute par portions 241 mg de borohydrure de sodium. Le mélange est agité à température ambiante pendant 2 heures puis le solvant est évaporé sous pression réduite. Le résidu est repris entre l'eau et l'acétate d'éthyle. La phase organique est décantée, séchée sur sulfate de sodium et concentrée sous pression réduite. Le résidu est purifié par chromatographie sur gel de silice en éluant avec un mélange dichlorométhane/méthanol. On obtient 48 mg de composé.
PF = 232,3 - 233,7°C. Spectre RMN 1H (DMSO-d6, δ en ppm) : 1,75 (m, 2H) ; 1,95 (m, 2H) ; 2,75 (m, 2H); 4,65 (m, 1H); 5,2 (m, 1H); 7,2 (m, 1H) ; 7,5 (m, 3H); 7,6 (m, 1H) ; 7,7 (m, 1H) ; 7,75 (m, 1H); 8,05 (d, 2H) ; 8,45 (s, 1H); 8,85 (s, 1H). M+H = 375.

### Exemple 5 : 5-[2-(4-Chlorophényl)imidazo[1,2-a]pyridin-6-yl]-1,2,3,4-tétrahydro naphthalèn -1-ol (composé 9 du tableau)

### 5-Bromo-3,4-dihydro-2H-naphthatèn-1-one

On place dans un ballon 2,21 g d'AlCl₃ auquel on additionne 0,91 ml de 3,4-dihydro-2*H*-naphthalèn-1-one en 10 mn et 0,41 ml de Br₂ en 5 mn, et on chauffe à 80°C pendant 10 mn. On laisse revenir à température ambiante et on ajoute au mélange une solution contenant 20 g de glace et 2,7 ml d'acide chlorhydrique concentré. Puis, on dilue avec de l'eau et de l'éther diéthylique. La phase organique est ensuite séparée, séchée et concentrée sous pression réduite. Le résidu est purifié par chromatographie préparative en phase inverse. On obtient 172 mg de composé.
Spectre RMN 1H (DMSO-d6, δ en ppm) : 2,1 (m, 2H); 2,65 (m, 2H) ; 2,95 (m, 2H); 7,35 (t, 1H) ; 7,9 (m, 2H). M+H = 226.

### 5-[2-(4-Chlorophényl)imidazo[1,2-a]pyridin-6-yl]-3,4-dihydro-2H-naphthalèn-1-one

En procédant comme dans l'exemple 4.3, en partant de 210 mg d'acide 2-(4-chlorophényl)imidazo[1,2-*a*]pyridine-6-boronique (composé obtenu selon le protocole décrit dans l'exemple 4.2), de 168 mg de 5-bromo-3,4-dihydro-2*H*-naphthalèn-1-one et de 39 mg de tétrakis(triphénylphosphine)palladium, on obtient 300 mg de 5-[2-(4-chlorophényl)imidazo[1,2-*a*]pyridin-6-yl]-3,4-dihydro-2*H*-naphthalèn-1-one. Le composé est purifié par chromatographie sur gel de silice en éluant avec un mélange dichlorométhane/méthanol. On obtient 167 mg de composé.
Spectre RMN 1H (DMSO-d6, δ en ppm) : 2,0 (m, 2H); 2,65 (m, 2H); 2,9 (m, 2H); 7,35 (d, 1H); de 7,45 à 7,55 (m, 3H) ; de 7,6 à 7,7 (m, 2H); de 8,0 à.8,1 (m, 3H) ; 8,45 (s, 1H) ; 8,6 (s, 1H). M+H = 373.

### 5.3 5-[2-(4-Chlorophényl)imidazo[1,2-a]pyridin-6-yl]-1,2,3,4-tétrahydronaphthalèn-1-ol

A 167 mg de 5-[2-(4-chlorophényl)imidazo[1,2-*a*]pyridin-6-yl]-3,4-dihydro-2*H-*naphthalèn-1-one dissous dans 15 ml de méthanol, on ajoute par portions 169 mg de borohydrure de sodium. Le mélange est agité à température ambiante pendant 2 heures puis le solvant est évaporé sous pression réduite. Le résidu est repris entre l'eau et l'acétate d'éthyle. La phase organique est décantée, séchée sur sulfate de sodium et concentrée sous pression réduite. On obtient 106 mg de composé.
PF = 218,1 - 219,6°C. Spectre RMN 1H (DMSO-d6, δ en ppm) : de 1,6 à 2,0 (m, 4H) ; de 2,55 à 2,7 (m, 2H) ; 4,65 (m, 1H); 5,2 (d, 1H); 7,2 (d, 1H); de 7,25 à 7,35 (m, 2H); 7,5 (m, 3H) ; 7,6 (d, 1H); 8,05 (d, 2H) ; 8,4 (s, 1H); 8,5 (s, 1H). M+H = 3 75.

### Exemple 6 : {2-Fluoro-3-[2-(3-méthoxyphényl)imidazo[1,2-a]pyridin-6-yl]phényl}méthanol (composé 19 du tableau)

### 2-(3-Méthogyphényl)-6-(4,4,5,5-tétraméthyl-1,3,2-dioxaborolan-2-yl)imidazo[1,2-a]pyridine

On place dans un ballon 5,00 g de 2-bromo-1-(3-méthoxyphényl)éthanone, 4,80 g de 5-(4,4,5,5-tétraméthyl-1,3,2-dioxaborolan-2-yl)pyridin-2-ylamine dans 220 ml de n-propanol et on ajoute 2,57 g d'hydrogénocarbonate de sodium. On chauffe à 80°C pendant 20h, laisse refroidir et concentre le mélange réactionnel sous pression réduite. Le résidu est repris entre l'eau et l'acétate d'éthyle, la phase organique est décantée, séchée sur sulfate de magnésium puis le solvant est concentré. On obtient 7,47 g de composé.
Spectre RMN 1H (DMSO-d6, δ en ppm) : 1,35 (s, 12H) ; 3,85 (s, 3H) ; 6,9 (d, 1H); de 7,35 à 7,45 (m, 2H) ; de 7,5 à 7,6 (m, 3H) ; 8,45 (s, 1H); 8,8 (s, 1H). M+H = 351.

### 6.2 Chlorhydrate (1 :1) d'acide 2-(3-méthoxyphényl)imidazo[1,2-a]pyridine-6-boronique

7,47 g de 2-(3-méthoxyphényl)-6-(4,4,5,5-tétraméthyl-l,3,2-dioxaborolan-2-yl)imidazo[1,2-*a*]pyridine sont dissous dans 236 ml d'acétone et 118 ml d'eau ; on y ajoute, goutte à goutte et sous agitation, 213 ml d'acide chlorhydrique 1N et agite à température ambiante pendant 24h. Le mélange réactionnel est ensuite concentré sous pression réduite. Le solide obtenu est trituré à l'éther diéthylique et recueilli par filtration, puis séché à l'étuve sous pression réduite à 60°C. On obtient 5,80 g de composé.
Spectre RMN 1H (DMSO-d6, δ en ppm) : 3,75 (s, 3H) ; 6,95 (d, 1H); de 7,3 à 7,65 (m, 3H) ; 7,8 (d, 1H); 8,05 (d, 1H); 8,75 (s, 1H); 8,9 (s, 1H). M+H = 325.

### 6.3 (3-Bromo-2-fluorophényl)méthanol

2,00 g de 3-bromo-2-fluorobenzaldéhyde sont mis en solution dans 98 ml de méthanol ; on y ajoute par portions 560 mg de borohydrure de sodium. Le mélange est agité à température ambiante pendant 2 heures puis le solvant est évaporé sous pression réduite. Le résidu est repris entre l'eau et l'acétate d'éthyle, la phase organique est séparée, séchée et concentrée sous pression réduite. Le résidu est purifié par chromatographie sur gel de silice en éluant avec un mélange dichlorométhane/méthanol. On obtient 1,56 g de composé.
Spectre RMN 1H (DMSO-d6, δ en ppm) : 4,60 (d, 2H) ; 5,4 (t, 1H); 7,15 (m, 1H) ; 7,5 (m, 1H) ; 7.6 (m, 1H). M+H = 206.

### {2-Fluoro-3-[2-(3-méthoxyphényl)imidazo[1,2-a]pyridin-6-yl] phényl}méthanol

On dissout 300 mg de (3-bromo-2-fluorophényl)méthanol dans 15 ml de toluène et 5 ml d'éthanol et dégaze sous courant d'argon pendant 10 mn. On y ajoute ensuite 101 mg de tétrakis(triphénylphosphine)palladium, 580 mg d' acide 2-(3-méthoxyphényl)imidazo[1,2-a]pyridine-6-boronique et 5 ml d'une solution 2M de carbonate de sodium. Le mélange est chauffé 16 heures à 80°C puis concentré sous pression réduite après refroidissement à température ambiante. Le résidu est repris entre l'eau et l'acétate d'éthyle puis la phase organique est séparée, séchée et concentrée sous pression réduite. Le résidu est purifié par chromatographie sur gel de silice en éluant avec un mélange dichlorométhane/acétate d'éthyle. On obtient 254 mg de composé.
PF = 143 - 144°C. Spectre RMN 1H (DMSO-d6, δ en ppm) : 3,85 (s, 3H) ; 4,65 (d, 2H); 5,35 (t, 1H) ; 6,95 (d, 1H) ; de 7,3 à 7,4 (m, 2H) ; 7,45 (d, 1H) ; de 7,5 à 7,65 (m, 4H) ; 7,7 (d, 1H) ; 8,5 (s, 1H); 8,75 (s, 1H). M+H = 349.

### Exemple 7 : 1-{3-[2-(4-Chlorophényl)imidazo[1,2-a]pyndin-6-yl]-4-fluorophényl}éthanol (composé 22 du tableau)

### 3-[2-(4-Chlorophényl)imidazol[1,2-a]pyridin-6-yl]-4-fluorobenzaldéhyde

Sous un courant d'argon, 800 mg de 6-bromo-2-(4-chlorophényl)imidazo[1,2-a]pyridine, 437 mg d'acide 2-fluoro-5-formyl-benzèneboronique et 150 mg de tétrakis(triphénylphosphine)palladium sont placés dans un ballon contenant un mélange de 8 ml d'acétonitrile, 8 ml de toluène et 10 ml d'une solution 0,5M de carbonate de sodium préalablement dégazé sous courant d'argon. Le mélange est chauffé 5 heures à 75°C puis dilué par un mélange d'eau et d'acétate d'éthyle après refroidissement. La phase organique est séparée, séchée et concentrée sous pression réduite. Le résidu est purifié par chromatographie sur gel de silice en éluant avec un mélange dichlorométhane/acétate d'éthyle. On obtient 486 mg de composé.
Spectre RMN 1 H (DMSO-d6, δ en ppm) : de7,65 à 7,75 (m, 4H) ; 8,05 (d, 1H); de 8,15 à 8,25 (m, 4H) ; 8,35 (d, 1H) ; 8,7 (s, 1H). M+H = 351.

### 7.2 1-{3-[2-(4-Chlorophényl)imidazo[1,2-a]pyridin-6-yl]-4-fluorophényl}éthanol

470 mg de 3-[2-(4-chlorophényl)imidazo[1,2-a]pyridin-6-yl]-4-fluorobenzaldéhyde est placé dans un ballon et dissous dans 90 ml d'éther diéthylique et 45 ml de tétrahydrofurane. On refroidit à 0°C par un bain de glace et on y ajoute goutte à goutte 4 ml d'une solution 1M de bromure de méthylmagnésium dans l'éther dibutylique. On agite le mélange dans le bain de glace pendant 1h30 et on ajoute 22 ml d'une solution aqueuse saturée de chlorure d'ammonium. La phase organique est séparée, séchée sur sulfate de magnésium et concentrée sous pression réduite. Le résidu obtenu est purifié par chromatographie sur gel de silice en éluant avec un mélange dichlorométhane/méthanol. On obtient 401 mg de composé
PF = 180 - 182°C. RMN 1H (DMSO-d6, δ en ppm) : 1,55 (d, 3H); 5,0 (q, 1H); de 7,15 à 7,25 (m, 1H); de 7,3 à 7,6 (m, 6H) ; 7,75 (d, 1H); de 7,85 à 8,0 (m, 3H); 8,4 (s, 1H). M+H = 367.

### Exemple 8: (+)-1-{3-[2-(4-Chlorophényl)imidazo[1,2-a]pyridin-6-yl]-+ fluorophényl}éthanol (composé 29 du tableau)

170 mg de 1-{3-[2-(4-Chlorophényl)imidazo[1,2-*a*]pyridin-6-yl]-4-fluorophényl}éthanol racémique (composé obtenu à l'étape 7.2) sont déposés sur une colonne ChiralPAK AD LOT CFB03 20µm 50x350mm. On élue par un mélange de méthanol, d'éthanol et d'heptane 20/30/50. On obtient 60 mg du composé le moins retenu.
alphaD = +18,1 (c = 0,502, méthanol); +13,3 (c = 0,446, DMSO).
PF = 187 - 187.5°C. RMN 1H (DMSO-d6, δ en ppm) : 1,4 (s, 3H) ; 4,8 (m, 1H) ; 5,25 (s, 1H) ; 7,3 (t, 1H) ; de 7,4 à 7,55 (m, 4H) ; 7,6 (d, 1H) ; 7,7 (d, 1H) ; 8,0 (d, 2H) ; 8,5 (s, 1H) ; 8,8 (s, 1 H).

### Exemple 9: (-)-1-{3-[2-(4-Chlorophényl)imidazo[1,2-a]pyridin-6-yl]-4-fluorophényl}éthanol (composé 30 du tableau)

En procédant comme décrit dans l'exemple 8, et à partir de 170 mg de 1-{3-[2-(4-Chlorophényl)imidazo[1,2-*a*]pyridin-6-yl]-4-fluorophényl}éthanol racémique, on obtient 62 mg de composé le plus retenu.
alphaD = -13,2 (c = 0,318, méthanol); -13,5 (c = 0,308, DMSO).
PF = 184 - 185°C. RMN 1H (DMSO-d6, δ en ppm) : 1,4 (s, 3H) ; 4,8 (m, 1H) ; 5,25 (s, 1H) ; 7,3 (t, 1H) ; de 7,4 à 7,55 (m, 4H) ; 7,6 (d, 1H) ; 7,7 (d, 1H) ; 8,0 (d, 2H) ; 8,5 (s, 1H) ; 8,8 (s, 1H).

### Exemple 10 : {2-[2-(2,4-Difluorophényl)imidazo[1,2-a]pyridin-6-yl]-6-fluorophényl}méthanol (composé 31 du tableau)

### 10.1 2-(2,4-Difluorophényl)-6-(4,4,5,5-tétraméthyl-1,3,2-dioxaborolan-2-yl)imidazo[1,2-a]pyridine

En procédant comme à l'étape 4.1, en partant de 4,76 g de 5-(4,4,5,5-tétraméthyl-1,3,2-dioxaborolan-2-yl)pyridin-2-ylamine et de 5,09 g de 2-bromo-1-(2,4-difluorophényl)éthanone, on obtient 4,84g de composé.
RMN 1H (DMSO-d6, δ en ppm) : 1,35 (s, 12H) ; 7,25 (t, 1H) ; de 7,35 à 7,45 (m, 2H); 7,6 (d, 1H) ; de 8,25 à 8,35 (m, 1H) ; 8,45 (s, 1H).; 8,9 (s, 1H) M+ = 356.

### 10.2 {2-[2-(2,4-Difluorophényl)imidazo[1,2-a]pyridin-6-yl]-6-fluorophényl}méthanol

Dans un tube à pression, on fait dégazer sous argon un mélange de 9,5 ml de DME et de 3,5 ml d'une solution 2M de carbonate de sodium, puis on ajoute le composé préparé en 10.1, 0.29 g de 2-bromo-6-fluorophénylméthanol et 81 mg de tétrakis(triphénylphosphine)palladium, referme le tube et laisse agiter dans un bain thermostaté à 100°C pendant 24h. On refroidit le mélange réactionnel, évapore le solvant sous pression réduite et reprend le résidu entre l'eau et le dichlorométhane. On filtre le précipité entre les 2 phases, le sèche et le purifie par chromatographie sur gel de silice en éluant avec un mélange dichlorométhane/méthanol. On obtient 190 mg de composé que l'on recristallise dans un mélange propan-2-ol / éther diisopropylique. On obtient 117 mg de composé.
RMN 1H (DMSO-d6, δ en ppm) : 4,45 (d, 2H); 5,25 (t, 1H) ; de 7,2 à 7,35 (m, 3H) ; de 7,4 à 7,55 (m, 3H); 7,7 (d, 1H) ; 8,35 (m, 2H) ; 8,75 (s, 1H).
PF = 216,5-217,5°C.

### Exemple 11 : 2-{3-[2-(4-Chlorophényl)imidazo[1,2-a]pyridin-6-yl]-2,6-difluorophényl}propan-2-ol (composé 35 du tableau)

### 11.1 2-(3-Bromo-2,6-difluorophényl)propan-2-ol

Sous un courant d'argon, on introduit dans un ballon 6,3 ml d'une solution 2 M de diisopropylamidure de lithium dans le tétrahydrofurane (préalablement titré à 0,86M) dans 5 ml de tétrahydrofurane. On refroidit le mélange à -78°C et on y ajoute goutte à goutte 0,59 ml de 1-bromo-2,4-difluorobenzène. On agite le mélange à -78°C pendant 30 min et on ajoute l'acétone par quatre fractions de 0,5 ml. Le milieu réactionnel est versé dans 20 ml d'acide chlorhydrique 1N après retour à température ambiante. La phase organique extraite à l'éther diéthylique est ensuite lavée deux fois par 20 ml d'eau, séparée, séchée sur sulfate de magnésium et concentrée sous pression réduite. L'huile obtenue est purifiée par chromatographie sur gel de silice en éluant avec un mélange heptane/acétate d'éthyle. On obtient 0,50 g de composé.
RMN 1H (DMSO-d6, δ en ppm): 1,6 (m, 6H) ; 5,4 (s, 1H) ; de 6,95 à 7,15 (m, 1H) ; de 7,6 à 7,75 (m, 1H).

### 11.2 2-{3-[2-(4-Chlorophényl)imidazo[1,2-a]pyridin-6-yl]-2,6-difluorophényl}propan-2-ol

On dissout 500 mg du composé obtenu à l'étape 11.1 dans 21 ml de toluène et 7 ml d'éthanol et dégaze sous courant d'argon pendant 10 mn. On y ajoute ensuite 138 mg de tétrakis(triphénylphosphine)palladium, 799 mg d'acide 2-(4-chlorophényl)imidazo[1,2-*a*]pyridine-6-boronique (composé obtenu selon le protocole décrit dans l'exemple 4.2) et 5 ml d'une solution 2M de carbonate de sodium. Le mélange est chauffé 1h30 à 80°C puis concentré sous pression réduite après refroidissement à température ambiante. Le résidu est repris entre l'eau et l'acétate d'éthyle puis la phase organique est séparée, séchée et concentrée sous pression réduite. Le résidu est purifié par chromatographie sur gel de silice en éluant avec un mélange heptane/acétate d'éthyle. Le solide obtenu est trituré dans l'éther diisopropylique, recueilli par filtration puis séché à l'étuve sous pression réduite. On obtient 383 mg de composé.
PF = 191 - 192°C. Spectre RMN 1H (DMSO-d6, δ en ppm) : 1,65 (s, 6H) ; 5,3 (s, 1H) ; 7,15 (m, 1H) ; 7,4 (m, 1H); 7,55 (m, 3H) ; 7,7 (m, 1H) ; 8,05 (d, 2H) ; 8,5 (s, 1H) ; 8,75 (s, 1H).

### Exemple 12 : 2-[2-Chloro-3-[2-(4-Chlorophényl)imidazo[1,2-a]pyridin-6-yl]phényl]propan-2-ol (composé 66 du tableau)

### 12.1 3-Bromo-2-chloro-N-méthoxy-N-méthyl-benzamide

On place dans un ballon 500 mg d'acide 3-bromo-2-chlorobenzoïque dans 12,5 ml de tétrahydrofurane. Le mélange réactionnel est refroidi à 0°C par un bain de glace puis on y ajoute 228 mg de chlorhydrate de *N,O*-diméthylhydroxylamine, 814 mg de chlorhydrate de 1-(3-diméthylaminopropyl)-3-éthylcarbodiimide et 0,21 ml de pyridine. Le mélange réactionnel est ensuite agité à température ambiante pendant 16h puis le solvant est évaporé sous pression réduite. Le résidu est repris entre l'eau et le dichlorométhane. La phase organique séparée est ensuite lavée une fois par une solution saturée de chlorure d'ammonium et une fois par une solution saturée d'hydrogénocarnonate de sodium puis séchée sur sulfate de magnésium et concentrée sous pression réduite. On obtient 500 mg de composé.
Spectre RMN 1H (DMSO-d6, δ en ppm) : 3,3 (s, 3H) ; 3,45 (s, 3H) ; 7,35 (t, 1H) ; 7,5 (d, 1H) ; 7,85 (d, 1H).

### 12.2 1-(3-Bromo-2-chlorophényl)éthanone

0,5 g de composé obtenu à l'étape 12.1 est placé dans un ballon et dissous dans 18 ml de tétrahydrofurane. On refroidit à 0°C par un bain de glace et on y ajoute goutte à goutte 2,7 ml d'une solution 3M de bromure de méthylmagnésium dans l'éther éthylique (préalablement titrée à 2M). On laisse agiter à 0°C pendant 1h puis à température ambiante pendant 2 heures. Le mélange réactionnel est ensuite hydrolysé à 0°C par de l'eau et une solution saturée de chlorure d'ammonium puis extrait à l'acétate d'éthyle. La phase organique est séparée, lavée par une solution saturée de chlorure de sodium, séchée sur sulfate de magnésium et concentrée sous pression réduite. On obtient 0,380 g de composé.
RMN 1H (DMSO-d6, δ en ppm) : 2,6 (s, 3H); 7,4 (t, 1H) ; 7,65 (d, 1H) ; 7,95 (d, 1H).

### 12.3 1-[2-Chloro-3-[2-(4-Chlorophényl)imidazo[1,2-a]pyridin-6-yl)phényl]éthanone

On dissout 380 mg de composé obtenu à l'étape 12.2 dans 16 ml de toluène et 8 ml d'éthanol et dégaze sous courant d'argon pendant 10 mn. On y ajoute ensuite 113 mg de tétrakis(triphénylphosphine)palladium, 654 mg d'acide 2-(4-chlorophényl)imidazo[1,2-*a*]pyridine-6-boronique (composé obtenu selon le protocole décrit dans l'exemple 4.2) et 4,07 ml d'une solution 2M de carbonate de sodium. Le mélange réactionnel est chauffé 2 heures à 80°C puis concentré sous pression réduite après refroidissement à température ambiante. Le résidu est repris entre l'eau et l'acétate d'éthyle. La phase organique est décantée, lavée 2 fois à l'eau puis 2 fois par une solution saturée de chlorure de sodium, séchée sur sulfate de magnésium et concentrée sous pression réduite. Le résidu est purifié par chromatographie sur gel de silice en éluant par un mélange dichlorométhane/acétate d'éthyle. Le solide obtenu est trituré dans l'éther diisopropylique, recueilli par filtration puis séché à l'étuve sous pression réduite. On obtient 259 mg de composé.
RMN 1H (DMSO-d6, δ en ppm) : 2,65 (s, 3H) ; 7,35 (d, 1H) ; de 7,5 à 7,65 (m, 3H) ; 7,7 (m, 3H) ; 8,05 (d, 2H) ; 8,5 (s, 1H) ; 8,7 (s, 1H).

### 12.4 2-[2-Chloro-3-[2-(4-Chlorophényl)imidazo[1,2-a]pyridin-6-yl]phényl]propan-2-ol

Sous courant d'azote, 150 mg du composé obtenu à l'étape 12.3 est placé dans un ballon et dissous dans un mélange de 26 ml d'éther diéthylique et 13 ml de tétahydrofurane anhydres. On refroidit à 0°C par un bain de glace et ajoute goutte à goutte 0,59 ml d'une solution 3M de bromure de méthylmagnésium dans l'éther éthylique (préalablement titrée à 2M). On laisse agiter à 0°C pendant 1h puis à température ambiante pendant 2 heures. Le mélange réactionnel est ensuite hydrolysé à 0°C par de l'eau et une solution saturée de chlorure d'ammonium. La phase organique est ensuite séparée, séchée sur sulfate de magnésium et concentrée sous pression réduite. Le résidu est purifié par chromatographie sur gel de silice en éluant par un mélange dichlorométhane/acétate d'éthyle. Le solide obtenu est trituré dans l'éther diisopropylique, recueilli par filtration puis séché à l'étuve sous pression réduite. On obtient 25 mg de composé.
PF = 230 - 231°C. RMN 1H (DMSO-d6, δ en ppm) : 1,65 (s, 6H) ; 5,35 (s, 1H); 7,3 (d, 1H) ; de 7,35 à 7,45 (m, 2H) ; 7,55 (d, 2H) ; 7,65 (d, 1H) ; 7,95 (d, 1H) ; 8,05 (d, 2H) ; 8,45 (s, 1H) ; 8,6 (s, 1H).

### Exemple 13 : 1-[2-Chloro-3-[2-(4-Chlorophényl)imidazo[1,2-a]pyridin-6-yl)phényl]éthanol (composé 67 du tableau)

A 100 mg de composé obtenu à l'étape 12.3 dissous dans 13 ml de méthanol et refroidi à 0°C, on ajoute par portions 99 mg de borohydrure de sodium. Le mélange est agité à température ambiante pendant 1 heure puis le solvant est évaporé sous pression réduite. Le résidu est repris entre l'eau et l'acétate d'éthyle. La phase organique est décantée, séchée sur sulfate de magnésium et concentrée sous pression réduite. Le résidu est purifié par chromatographie sur gel de silice en éluant par un mélange dichlorométhane/acétate d'éthyle. Le solide obtenu est trituré dans l'éther diisopropylique, recueilli par filtration puis séché à l'étuve sous pression réduite. On obtient 73 mg de composé.
PF = 170 - 172°C. RMN 1H (DMSO-d6, δ en ppm) : 1,4 (d, 3H) ; 5,15 (m, 1H) ; 5,45 (d, 1H) ; 7,3 (d, 1H) ; 7,4 (d, 1H) ; de 7,45 à 7,55 (m, 3H) ; 7,65 (d, 1H) ; 7,75 (d, 1H) ; 8,05 (d, 2H) ; 8,45 (s, 1H) ; 8,6 (s, 1H).

### Exemple 14 : {2-Fluoro-6-[2-(4-fluorophényl)imidazo[1,2-a]pyridin-6-yl]phényl}méthanol (composé 53 du tableau)

### 14.1 (2-Bromo-6-fluorophényl)méthanol

20,0 g (0098 mole) de 2-bromo-6-fluorobenzaldéhyde sont mis en solution dans 500 ml de méthanol et refroidi au bain de glace; puis on y ajoute par portions 3,72 g (0.098 mole) de borohydrure de sodium. Le mélange est agité à froid pendant 1 heure puis le solvant est évaporé sous pression réduite. Le résidu est repris entre l'eau et le dichlorométhane, la phase organique est séparée, séchée et concentrée sous pression réduite. Le résidu est cristallisé dans le pentane. On obtient 18,1 g de composé.
RMN 1H (CDCl3, δ en ppm) : 2,15 (t, 1H); 4,95 (d, 2H) ; de 7,05 à 7,3 (m, 2H) ; 7,45 (d, 1H).

### 14.2 (2-Bromo-6-fluorobenzyloxy)tert-butyldiméthylsilane

Dans un ballon de 500 ml, on dissout 15,7g (0.076 mole) du composé obtenu précédemment dans 230 ml de THF et ajoute 7,8 g (0.115 mole) d'imidazole puis 13,8g (0.092 mole) de *tert*-butyldiméthylchlorosilane et agite le mélange réactionnel pendant 16 heures. Puis on évapore le solvant sous pression réduite, reprend le résidu entre l'eau et l'éther diéthylique, décante, lave la phase organique à l'eau et la sèche sur sulfate de sodium. Après évaporation du solvant, on recueille 25 g d'huile.
RMN 1H (CDCl3, δ en ppm) : 0,0 (s, 6H) ; 0,8 (s, 9H) ; 4,7 (s, 2H) ; de 6,8 à 7,05 (m, 2H) ; 7,25 (d, 1H).

### 14.3 5-[2-(tert-Butyldiméthylsilanylozyméthyl)-3-fluorophényl]pyridin-2-ylamine

6,4 g du composé obtenu en 14.2, 4,40 g de 5-(4,4,5,5-tétraméthyl-1,3,2-dioxaborolan-2-yl)pyridin-2-ylamine, 30 ml d'une solution 2M de carbonate de sodium et 816 mg de [1,1'-bis(diphénylphosphino)ferrocène]dichloropalladium sont dissous dans 80 ml de *N,N*-diméthylformamide et placés dans un ballon sous courant d'argon. Le mélange est chauffé 2h à 80°C. Après refroidissement à température ambiante, on évapore les solvants sous pression réduite et reprend le résidu entre l'eau et l'acétate d'éthyle et élimine un insoluble par filtration sur célite. La phase organique est décantée, lavée avec une solution aqueuse saturée en chlorure de sodium et séchée sur sulfate de sodium. Le composé est purifié par chromatographie en éluant avec un mélange de dichlorométhane et de méthanol. On obtient 4,58 g d'huile.
RMN 1H (CDCl3, δ en ppm) : 0 (s, 6H) ; 0,8 (s, 9H) ; 4,4 (s, 2H) ; 6,05 (s, 2H) ; 6,45 (d, 1H) ; 7,05 (t, 1H) ; de 7,35 à 7,45 (m, 2H) ; 8,0 (s, 1H). M+H = 351 manque 1H

### 14.4 6-[2-(tert-Butyldiméthylsilanyloxyméthyl)-3-fluorophényl]-2-(4-fluorophényl)imidazo[1,2-a]pyridine

58 mg (0,7 mmole) de bicarbonate de sodium sont pesés dans un tube à microondes. On y ajoute 83 mg (0,25 mmole) du composé obtenu en 14.3 en solution dans 2 ml de propan-1-ol, puis 0,375 mmole de 1-(4-fluorophényl)-2-bromoéthanone en solution dans 1 ml de propan-1-ol Le tube est scellé puis agité à 80°C pendant 16 heures. Le mélange réactionnel est refroidi à température ambiante, on y ajoute 200 mg de propanethiol sur silice (Biotage Si-Thiol) et le mélange est agité 6h à température ambiante, puis filtré et le filtrat est évaporé sous pression réduite. Le composé est utilisé tel quel pour l'étape suivante

### 14.5 {2-Fluoro-6-[2-(4-fluorophényl)imidazo[1,2-a]pyridin-6-yl]phényl}méthanol

Le composé brut obtenu en 14.4 est dissous dans 5 ml de THF contenant 0,5 mmole de fluorure de tétrabutylammonium hydraté. Le mélange est agité 16h à température ambiante, puis le solvant est évaporé sous pression réduite. Le composé est purifié par chromatographie. On obtient 24,9 mg de composé.
RMN 1H (DMSO-d6, δ en ppm) : 4,45 (d, 2H) ; 5,3 (t, 1H); 7,3 (m, 4H) ; de 7,4 à 7,5 (m, 2H) ; 7,65 (d, 1H); 8,05 (m, 2H) ; 8,45 (s, 1H) ; 8,65 (s, 1H).

### Exemple 15 : {3-[2-(3,4-Difluorophényl)imidazo[1,2-a]pyridin-6-yl]-2,6-difluorophényl}méthanol (composé 40 du tableau)

### 15.1 (3-bromo-2,6-difluorophényl)méthanol

20 g de 3-bromo-2,6-difluorobenzaldéhyde sont mis en solution dans 450 ml de méthanol et refroidi au bain de glace; puis on y ajoute par portions 3,42 g de borohydrure de sodium. Le mélange est agité à température ambiante pendant 1 heure puis le solvant est évaporé sous pression réduite. Le résidu est repris entre l'eau et le dichlorométhane, la phase organique est séparée, séchée et concentrée sous pression réduite. Le résidu est cristallisé dans le n-pentane. On obtient 14,6 g de composé.
Spectre RMN 1H (CDCl3, δ en ppm) : 2,0 (s, 1H) ; 4,9 (s, 2H) ; de 6,85 à 7,0 (m, 1H) ; de 7,5 à 7,65 (m, 1H).

### 15.2 (3-Bromo-2,6-difluorobenzyloxy)tert-butyldiméthylsilane

11,15 g du composé obtenu en 15.1 sont dissous dans 150 ml de THF, on ajoute 5,1 g d'imidazole puis 9,04 g de chloro-*tert*-butyldiméthylsilane et agite le mélange à température ambiante pendant 24 heures. Puis on évapore le solvant, reprend le résidu et l'eau et l'éther diéthylique, décante, lave la phase organique à l'eau et la sèche sur sulfate de sodium. On évapore le solvant sous pression réduite. On obtient 17,5 g d'huile.
Spectre RMN 1H (CDCl3, δ en ppm) :0,0 (s, 6H) ; 0,8 (s, 9H) ; 4,65 (s, 2H) ; de 6,65 à 6,7 (m, 1H) ; de 7,3 à 7,4 (m, 1H).

### 15.3 5-[3-(tert-Butyldiméthylsilanyloxyméthyl)-2,4-difluorophényl]pyridin-2-ylamine

6,7 g du composé obtenu en 15.2, 4,40 g de 5-(4,4,5,5-tétraméthyl-1,3,2-dioxaborolan-2-yl)pyridin-2-ylamine, 30 ml d'une solution 2M de carbonate de sodium et 816 mg de [1,1'-bis(diphénylphosphino)ferrocène]dichloropalladium sont dissous dans 80 ml de *N,N*-diméthylformamide et placés dans un ballon sous courant d'argon. Le mélange est chauffé 2h à 80°C. Après refroidissement à température ambiante, on évapore les solvants sous pression réduite et reprend le résidu entre l'eau et l'acétate d'éthyle et élimine un insoluble par filtration sur célite. La phase organique est décantée, lavée avec une solution aqueuse saturée en chlorure de sodium et séchée sur sulfate de sodium. Le composé est purifié par chromatographie en éluant avec un mélange de dichlorométhane et de méthanol. On obtient 4,25 g de solide blanc.
RMN 1H (DMSO-d6, δ en ppm) : 0 (s, 6H) ; 0,8 (s, 9H) ; 4,4 (s, 2H) ; 6,05 (s, 2H) ; 6,45 (d, 1H) ; 7,05 (t, 1H) ; de 7,35 à 7,45 (m, 2H) ; 8,0 (s, 1H). M+H = 351.

### 15.4 5[3-(tert-Butyldiméthylsilanyloxyméthyl)-2,4-difluorophényl]-2-(3,4-difluorophényl)imidazo[1,2-a]pyridine

58,8 mg (0,7 mmole) de bicarbonate de sodium sont pesés dans un tube à microondes. On y ajoute 87,5 mg (0,25 mmole) du composé obtenu en 15.3 en solution dans 2 ml de propan-1-ol, puis 0,375 mmole de 2-bromo-1-(3,4-difluorophényl)éthanone en solution dans 1 ml de propan-1-ol. Le tube est scellé puis agité à 80°C pendant 16 heures. Le mélange réactionnel est refroidi à température ambiante, on y ajoute 200 mg de propanethiol sur silice (Biotage Si-Thiol) et le mélange est agité 6h à température ambiante, puis filtré. Le résidu est lavé par 2 fois 2 ml de propan-1-ol, puis le filtrat est évaporé. Le composé est utilisé tel quel pour l'étape suivante.

### 15.5 {3-[2-(3,4-Difluorophényl)imidazo[1,2-a]pyridin-6-yl]-2,6-difluorophényl}méthanol

Le composé brut obtenu en 15.4 est dissous dans 5 ml de THF contenant 0,5 mmole de fluorure de tétrabutylammonium hydraté. Le mélange est agité 16h à température ambiante, puis le solvant est évaporé sous pression réduite. Le composé est purifié par chromatographie. On obtient 49,6 mg de composé.
RMN 1H (DMSO-d6, δ en ppm) : 4,6 (d, 2H); 5,35 (t, 1H) ; 7,25 (t, 1H); 7,45 (d, 1H); de 7,5 à 7,6 (m, 1H); de 7,6 à 7,7 (m, 2H) ; de 7,8 à 7,85 (m, 1H) ; de 7,95 à 8,05 (m, 1H) ; 8,5 (s, 1H) ; 8,75 (s, 1H).

### Exemple 16 : {2-[2-(4-Chlorophényl)imidazo[1,2-a]pyridin-6-yl]-6-fluorophényl}méthanol (composé 68 du tableau)

### 16.1 6-[2-(tert-Butyldiméthylsilanyloxyméthyl)-3-fluorophényl]-2-(4-chlorophényl)imidazo[1,2-a]pyridine

Dans un tricol de 100 mL, muni d'un agitateur magnétique et maintenu sous atmosphère d'azote sont introduits 1,2 g (3,61 mmoles) de composé obtenu en 14.3, 0,94 g (3,97 mmoles) de 2-bromo-(4-chlorophényl)éthanone et 0,42 g (5,05 mmoles) d'hydrogénocarbonate de sodium et 35 ml d'éthanol absolu. Après 18 heures d'agitation à 80°C, le mélange réactionnel est ramené à température ambiante, concentré sous pression réduite puis est dilué avec 50 mL d'eau et 100 ml de CH₂Cl₂. On décante et on sèche la phase organique avec du sulfate de sodium. On purifie le solide obtenu après évaporation du solvant par chromatographie sur colonne de gel de silice en éluant avec un mélange de dichlorométhane et d'acétone. On obtient ainsi 1,47 g du produit attendu.
R.M.N. ¹H (DMSO D₆), δ (ppm) : 0,0 (s, 6H) ; 0,8 (s, 9H) ; 4,5 (d, 2H) ; 7,05 (m, 2H) ; 7,3 (m, 5H); 7,55 (d, 1H); 7,75 (s, 1H); 7,85 (d, 1H); 8,35 (s, 1H).

### 16.2 {2-[2-(4-Chlorophényl)imidazo[1,2-a]pyridin-6-yl]-6-fluorophényl}méthanol

A une solution de 1,4 g (3 mmoles) du composé obtenu en 16.1 dans 50 mL de THF, maintenue sous atmosphère inerte et agitée à température ambiante, est ajoutée 1,57 g (5,99 mmoles) de fluorure de tétrabutylammonium. Après 1 heure de réaction à température ambiante, le solvant est évaporé sous pression réduite. Le résidu est dilué avec 20 mL d'eau et 50 mL de dichlorométhane. On décante et on extrait la phase aqueuse par 2x20 mL de dichlorométhane. Les phases organiques rassemblées sont lavées trois fois avec 20 mL d'eau, séchées sur sulfate de sodium puis concentrées sous pression réduite. On purifie le solide résultant par chromatographie sur colonne de gel de silice en éluant avec un mélange de dichlorométhane et de méthanol. Le solide obtenu est recristallisé dans l'alcool éthylique absolu. Les cristaux formés sont filtrés puis séchés à 100°C sous pression réduite. On obtient 0,60 g du produit attendu.
R.M.N. ¹H (DMSO D₆), δ (ppm) : 4,45 (d, 2H) ; 5,3 (t, 1H) ; 7,3 (m, 2H) ; 7,55-7,4 (m, 4H); 7,7 (d, 1H) ; 8,05 (d, 2H) ; 8,5 (s, 1H) ; 8,7 (s, 1H). PF° : 229-230°C.

### Exemple 17: 2-(4-Chlorophényl)-6-[3-fluoro-2-[(2-méthoxy-éthyl)oxyméthyl]phényl]imidazol[1,2-a]pyridine (composé 69 du tableau)

Dans un ballon de 50 mL sont introduits 0,4 g (1,13 mmole) de composé préparé à l'étape 16.2, 0,47 g (3,39 mmole) de 1-bromo-2-méthoxyéthane et 3,28 g (22,6 mmoles) de fluorure de potassium à 40% sur alumine. Le mélange est dilué avec 10 mL d'acétonitrile et 10 mL de *N,N*-diméthylformamide puis agité à 80°C pendant 4 heures. Après ce temps, le mélange refroidi est filtré. Le solvant est évaporé sous pression réduite et le résidu obtenu est purifié par chromatographie sur colonne de gel de silice en éluant avec un mélange d'acétone et de dichlorométhane. Le solide obtenu après évaporation du solvant sous pression réduite est lavé à l'ébullition de l'éther isopropylique, filtré à froid puis séché à 100°C sous pression réduite. On isole ainsi 120 mg du produit attendu.
R.M.N. ¹H (DMSO D₆), δ (ppm) : 3.45 (s, 3H) ; 3,65 (t, 2H) ; 3,75(t, 2H) ; 4,5 (s, 2H) ; 7,15 (m, 1H) ; 7,25 (d, 1H) ; 7,35 (d, 1H) ; 7,45 (m, 3H) ; 7,7 (d, 1H) ; 7,95 (m, 3H) ; 8,65 (s, 1H). PF=104-105°C

### Exemple 18 : Préparation des composés 70 à 81

### 18.1 Bromhydrate de [2-imino-5-(4,4,5,5-tetraméthyl-1,3,2-dioxaborolan-2-yl)-2H-pyridin-1-yl]acétate d'éthyle

5,0 g de 5-(4,4,5,5-tétraméthyl-1,3,2-dioxaborolan-2-yl)pyridin-2-ylamine dans 7,6 ml de 2-bromoacétate d'éthyle sont placés dans un ballon et le mélange est agité à température ambiante pendant 20 h. Il se forme un précipité que l'on recueille par filtration, lave par de l'éther diéthylique puis par de l'éthanol et sèche à l'étuve sous pression réduite. On obtient 8,78 g de composé.
Spectre RMN 1H (DMSO-d6, δ en ppm) : 1,3 (m, 15H à vérifier) ; de 4,1 à 4,25 (m, 2H) ; 5,2 (s, 2H) ; 7,1 (d, 1H); 8,0 (d, 1H); 8,3 (s, 1H) ; 9,0 (s, 1H). M+H = 388.

### 18.2 2-Chloro-6-(4,4,5,5-tétraméthyl-1,3,2-dioxaborolan-2-yl)imidazo[1,2-a]pyridine (composé intermédiaire (Xa))

Dans un ballon on place 8,78 g de composé obtenu selon le protocole décrit en 18.1 dans 20 ml de POCl₃. Le mélange réactionnel est chauffé à 105°C pendant 16h, refroidi à température ambiante et concentré sous pression réduite. Le résidu est repris entre le dichlorométhane et l'eau à 0°C et on ajoute une solution aqueuse à 30% de NH₄OH jusqu'à pH basique. La phase organique est séparée, séchée sur sulfate de magnésium et concentrée sous pression réduite. On obtient 4,3 g de composé.
PF = 115 - 120°C. Spectre RMN 1H (DMSO-d6, δ en ppm) : 1,35 (m, 12H) ; 7,4 (d, 1H) ; 7,5 (d, 1H) ; 8,1 (s, 1H) ; 8,85 (s, 1H). M+H = 279.

### 18.3 6-[2-(tert-Butyldiméthylsilanyloxyméthyl)-3-fluorophényl]-2-chloroimidazo[1,2-a]pyridine

On dégaze sous courant d'argon 100 ml d'un mélange 85/15 de THF et d'eau puis ajoute 5,3 g du composé préparé en 9.2, 6,07 g de 2-chloro-6-(4,4,5,5,-tétraméthyl-1,2,3-dioxaborolan-2-yl)imidazo[1,2-*a*]pyridine obtenu tel qu'en 18.2, 18,6 g de carbonate de césium et 466 mg de de [1,1'-bis(diphénylphosphino)ferrocène]dichloropalladium. On agite le mélange 2 heures dans un bain thermostaté à 80°C. Après refroidissement à température ambiante, les solvants sont évaporés sous pression réduite. Le résidu est repris entre l'eau et l'éther diéthylique. On élimine un solide par filtration. La phase organique lavée par 2 fois avec une solution saturée de chlorure de sodium est ensuite séchée sur sulfate de sodium et concentrée sous pression réduite. Le résidu obtenu est purifié par chromatographie sur gel de silice en éluant avec un mélange dichlorométhane/méthanol. Le solide obtenu est trituré au pentane, recueilli par filtration, puis séché à l'étuve sous pression réduite. On obtient 4,74 g de composé. RMN 1H (CDCl3, δ en ppm) : 0 (s, 6H) ; 0,85 (s, 9H) ; 4,5 (s, 2H) ; de 7,05à 7,1 (m, 2H) ; de 7,25 à 7,3 (m, 2H) ; 7,4 (s, 1H) ; 7,45 (s, 1H) ; 8,3 (s, 1H). M+H = 391.

### 18.4 Procédé de préparation des composés 70 à 81

**18.4.1** 0,495 mmole d'acétate de palladium, et 0,99 mmole de S-Phos sont pesés dans un ballon de 100 ml purgé à l'argon. On y ajoute 55 ml de toluène dégazé et on agite le mélange dans un bain à ultrasons jusqu'à dissolution complète.
**18.4.2** 0,3 mmole d'acide arylboronique est pesée dans un tube à réaction, on y ajoute successivement 0,36 mmole de phosphate de potassium finement pulvérisé et séché, 0,5 ml d'éthanol anhydre dégazé, 0,18 mmole du composé obtenu en 18.3 en solution dans 2 ml de toluène puis le tube est purgé à l'argon. On ajoute ensuite 1 ml de la solution préparée en 18.4.1. Le tube est fermé et agité 16h à 75°C. On rajoute 0,5 ml de solution préparée en 18.4.1 et prolonge le chauffage pendant 10h. La solution refroidie est diluée par 5 ml d'acétate d'éthyle, on y ajoute 100 mg de silice-propanethiol (Biotage Si-Thiol) et le mélange est agité 4h à température ambiante. Le solide est séparé par filtration et lavé par 2x2 ml de THF. Le filtrat est évaporé à sec et le résidu est utilisé tel quel pour l'étape suivante.
**18.4.3** Dans un tube à réaction, on mélange le composé obtenu en 18.4.2, 0,36 mmole de fluorure de césium en solution dans 3 ml de méthanol et 21 µl d'acide acétique. La solution est agitée 16 heures à température ambiante, les solvants sont ensuite évaporés. Le résidu est purifié par HPLC en éluant avec un mélange acétonitrile/eau.

### Exemple 19 : Préparation des composés 82 à 93

### 19.1 6-[3-(tert-Butyldiméthylsilanyloxyméthyl)-2,4-difluorophényl]-2-chloroimidazo[1,2-a]pyridine

On dégaze sous courant d'argon 100 ml d'un mélange 85/15 de THF et d'eau puis ajoute 5,3 g du composé préparé en 15.2, 6,07 g de 2-chloro-6-(4,4,5,5, tétraméthyl-1,2,3-dioxaborolan-2-yl)imidazo[1,2-*a*]pyridine obtenu tel qu'en 18.2, 18,6 g de carbonate de césium et 466 mg de de [1,1'-bis(diphénylphosphino)ferrocène]dichloropalladium. On agite le mélange 2 heures dans un bain thermostaté à 80°C. Après refroidissement à température ambiante, les solvants sont évaporés sous pression réduite. Le résidu est repris entre l'eau et l'éther diéthylique. On élimine un solide par filtration. La phase organique lavée par 2 fois avec une solution saturée de chlorure de sodium est ensuite séchée sur sulfate de sodium et concentrée sous pression réduite. Le résidu obtenu est purifié par chromatographie sur gel de silice en éluant avec un mélange dichlorométhane/méthanol. Le solide obtenu est trituré au pentane, recueilli par filtration, puis séché à l'étuve sous pression réduite. On obtient 4,74 g de composé. RMN 1H (DMSO-d6, δ en ppm) : 0,0 (s, 6H) ; 0,8 (s, 9H) ; 4,7 (s, 2H) ; 7,15 (t, 1H) ; 7,4 (d, 1H) ; de 7,5 à 7,6 (m, 2H) ; 8,0 (s, 1H) ; 8,65 (s, 1H). M+H = 409.

### 19.2 Procédé de préparation des composés 82 à 93

**19.2.1** 0,3 mmole d'acide arylboronique est pesée dans un tube à réaction, on y ajoute successivement 0,36 mmole de phosphate de potassium finement pulvérisé et séché, 0,5 ml d'éthanol anhydre dégazé, 0,18 mmole du composé obtenu en 19.1 en solution dans 2 ml de toluène puis le tube est purgé à l'argon. On ajoute ensuite 1 ml de la solution préparée en 18.4.1. Le tube est fermé et agité 16h à 75°C. On rajoute 0,5 ml de solution préparée en 18.4.1 et prolonge le chauffage pendant 10h. La solution refroidie est diluée par 5 ml d'acétate d'éthyle, on y ajoute 100 mg de silice-propanethiol (Biotage Si-Thiol) et le mélange est agité 4h à température ambiante. Le solide est séparé par filtration et lavé par 2x2 ml de THF. Le filtrat est évaporé à sec et le résidu est utilisé tel quel pour l'étape suivante.
**19.2.2** Dans un tube à réaction, on mélange le composé obtenu en 19.2.1, 0,36 mmole de fluorure de césium en solution dans 3 ml de méthanol et 21 µl d'acide acétique. La solution est agitée 16 heures à température ambiante, les solvants sont ensuite évaporés. Le résidu est purifié par HPLC en éluant avec un mélange acétonitrile/eau.

Le tableau 2 illustre les structures chimiques des composés de formule générale (I) objets de l'invention ;

Les tableaux 3 et tableau 4 illlustrent les caractéristiques physicochimiques de quelques exemples de composés selon l'invention.

Dans ces tableaux :
- la colonne « R » renseigne sur la position de substitution du groupe 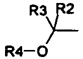 sur le noyau phényle (2, 3 ou 4) ;
- la colonne « PF » renseigne les points de fusion des produits en degrés Celsius (°C) ou, lorsque les produits ont été isolés sous la forme de solide amorphe ou d'huile, ils sont caractérisés par leur masse [M+H] ;
- dans la colonne « Sel/base », « - » représente un composé sous forme de base libre, alors que « HCl » représente un composé sous forme de chlorhydrate et le rapport entre parenthèses est le rapport (acide :base) ;
- Pour les composés 29 et 30, les cellules de la colonne « Sel/base » est renseignent le résultat d'analyse du pouvoir rotatoire de ces composés à la longueur d'onde de 589 nM ; le solvant indiqué entre parenthèses correspond au solvant employé pour réaliser la mesure du pouvoir rotatoire en degrés et la lettre « c » indique la concentration du solvant en g/100 ml ; DMSO signifie diméthylsulfoxide ;
- « Ph » signifie phényle ; « Cl » signifie chlore ; « F » signifie fluor ; « Me » signifie méthyle ; « MeO » signifie méthoxy ; « (F₂CH)O » signifie difluorométhoxy ; IsPr signifie isopropyle ;
- dans la colonne R, le chiffre devant le substituant renseigne la position de sustitution du groupe R sur le noyau phényle ;
- « ND » signifie non déterminé ;

**Tableau 2**

| | | | | | | | |
|---|---|---|---|---|---|---|---|
| 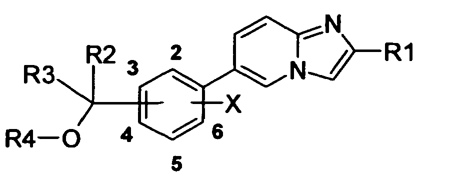 | | | | | | | |

| Ex | R₁ | R | R₄ | R₃ | R₂ | X | Sel/base |
|---|---|---|---|---|---|---|---|
| 1 | 4-Cl-Ph | 3 | H | H | H | 2-F | - |
| 2 | 4-Cl-Ph | 3 | H | Me | H | 2-F | - |
| 3 | 4-Cl-Ph | 3 | H | H | H | 2,4-(F)₂ | - |
| 4 | 4-Cl-Ph | 3 | H | Me | H | 2,4-(F)₂ | - |
| 5 | 4-Cl-Ph | 3 | H | Me | H | 4-F | - |
| 6 | 4-Cl-Ph | 3 | H | H | H | 5-Me | - |
| 7 | 4-Cl-Ph | 3 | H | H | H | 5-MeO | - |
| 8 | 4-Cl-Ph | 3 | H | H | -(C H₂)₃-* | | - |
| 9 | 4-Cl-Ph | 3 | H | H | -(CH ₂)₃-* | | - |
| 10 | 4-Cl-Ph | 3 | H | H | H | 6-Me | - |
| 11 | 4-Cl-Ph | 3 | H | H | H | 4-F | - |
| 12 | 4-Cl-Ph | 3 | H | H | H | 4-Me | - |
| 13 | 4-Cl-Ph | 3 | H | H | H | 2-Me | - |
| 14 | 4-Me-Ph | 4 | H | H | H | 3-F | - |
| 15 | 4-Me-Ph | 2 | H | H | H | 3-F | - |
| 16 | 3-MeO-Ph | 2 | H | H | H | 3-F | - |
| 17 | 3-MeO-Ph | 4 | H | H | H | 3-F | HCl(1:1) |
| 18 | Ph | 4 | H | H | H | 3-F | HCl(1:1) |
| 19 | 3-MeO-Ph | 3 | H | H | H | 2-F | - |
| 20 | Ph | 2 | H | H | H | 3-F | HCl(1:1) |
| 21 | 4-Me-Ph | 3 | H | H | H | 2-F | - |
| 22 | 4-Cl-Ph | 3 | H | Me | H | 6-F | - |
| 23 | 4-Cl-Ph | 3 | H | H | H | 6-F | - |
| 24 | 4-Cl-Ph | 3 | H | H | H | 6-MeO | - |
| 25 | 4-Cl-Ph | 3 | H | Me | H | 6-MeO | - |
| 26 | 4-Cl-Ph | 3 | H | H | H | 5-CH₂OH | - |
| 27 | 2-Naphthyl | 3 | H | H | H | 2-F | - |
| 28 | Ph | 3 | H | H | H | 2-F | HCl(1:1) |
| 29 | 4-Cl-Ph | 3 | H | Me | H | 6-F | +18,1 (c = 0,502, méthanol) ; +13,3 (c = 0,446, DMSO). |
| 30 | 4-Cl-Ph | 3 | H | Me | H | 6-F | -13,2 (c = 0,318, méthanol) ; -13,5 (c = 0,308, DMSO). |
| 31 | 2,4-diF-Ph | 2 | H | H | H | 3-F | - |
| 32 | 2,4-diF-Ph | 3 | H | H | H | 2,4-diF | - |
| 33 | 3-MeO-Ph | 3 | H | Me | Me | 6-F | - |
| 34 | 3-MeO-Ph | 3 | H | Me | H | 6-F | - |
| 35 | 4-Cl-Ph | 3 | H | Me | Me | 2,4-diF | - |
| 36 | 2-Naphthyl | 2 | H | Me | H | 5-F | - |
| 37 | 4-Me-Ph | 3 | Me | H | H | 2,4-diF | - |
| 38 | 4-NO₂-Ph | 2 | H | H | H | 3-F | - |
| 39 | 4-(Pyrrolidin-1-yl)Ph | 3 | H | H | H | 2,4-diF | - |
| 40 | 3,4-diF-Ph | 3 | H | H | H | 2,4-diF | - |
| 41 | 2-F-Ph | 3 | H | H | H | 2,4-diF | - |
| 42 | 3-Br-Ph | .3 | H | H | H | 2,4-diF | - |
| 43 | 4-MeSO₂-Ph | 3 | H | H | H | 2,4-diF | - |
| 44 | Ph | 3 | H | Me | Me | 2-Me | - |
| 45 | 2,4-diF | 2 | H | H | -(CH₂)₂-** | | - |
| 46 | 4-Cl-Ph | 3 | MeO-(CH₂)₂ | H | H | 2,4-diF | - |
| 47 | 2,4-diF | 2 | H | Me | -(CH₂)₂-** | | - |
| 48 | 4-Cl-Ph | 3 | H | Me | Me | 2-OMe | - |
| 49 | 4-Cl-Ph | 3 | H | Me | H | 2-OMe | - |
| 50 | 4-CF₃-Ph | 2 | H | H | H | 3-F | - |
| 51 | 4-MeO-Ph | 2 | H | H | H | 3-F | - |
| 52 | 4-(pyrrolin-1-yl)-Ph | 2 | H | H | H | 3-F | - |
| 53 | 4-F-Ph | 2 | H | H | H | 3-F | - |
| 54 | 3,4-diF-Ph | 2 | H | H | H | 3-F | - |
| 55 | 3-CN-Ph | 2 | H | H | H | 3-F | - |
| 56 | 2-F-Ph | 2 | H | H | H | 3-F | - |
| 57 | 3-Br-Ph | 2 | H | H | H | 3-F | - |
| 58 | 3-Me-4-Cl-Ph | 2 | H | H | H | 3-F | - |
| 59 | 3-Cl-4-Me-Ph | 2 | H | H | H | 3-F | - |
| 60 | 4-MeSO₂-Ph | 2 | H | H | H | 3-F | - |
| 61 | 4-MeO-Ph | 3 | H | H | H | 2,4-diF | - |
| 62 | 4-F-Ph | 3 | H | H | H | 2,4-diF | - |
| 63 | 3-CN-Ph | 3 | H | H | H | 2,4-diF | - |
| 64 | 3-Me-4-Cl-Ph | 3 | H | H | H | 2,4-diF | - |
| 64 bis | 3-Cl-4-Me-Ph | 3 | H | H | H | 2,4-diF | |
| 65 | 4-MeS-Ph | 3 | H | H | H | 2,4-diF | - |
| 66 | 4-Cl-Ph | 3 | H | Me | Me | 2-Cl | - |
| 67 | 4-Cl-Ph | 3 | H | Me | H | 2-Cl | - |
| 68 | 4-Cl-Ph | 2 | H | H | H | 3-F | - |
| 69 | 4-Cl-Ph | 2 | MeO-(CH2)2 | H | H | 3-F | - |
| 70 | 3-MeCONH-Ph | 2 | H | H | H | 3-F | - |
| 71 | 4-(HOCH₂)-Ph | 2 | H | H | H | 3-F | - |
| 72 | 3-(HOCH₂)-Ph | 2 | H | H | H | 3-F | - |
| 73 | 4-MeSO₂NH-Ph | 2 | H | H | H | 3-F | - |
| 74 | 4-Me₂NCO-Ph | 2 | H | H | H | 3-F | - |
| 75 | 4-MeOCONH-Ph | 2 | H | H | H | 3-F | - |
| 76 | 4-MeNHCO-Ph | 2 | H | H | H | 3-F | - |
| 77 | 4-MeCONH-Ph | 2 | H | H | H | 3-F | - |
| 78 | 4-(morpholin-4-yl)Ph | 2 | H | H | H | 3-F | - |
| 79 | 3-(Me₂NSO₂)Ph | 2 | H | H | H | 3-F | - |
| 80 | 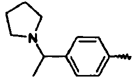 | 2 | H | H | H | 3-F | - |
| 81 | 3-F-4-(Me₂NCO)-Ph | 2 | H | H | H | 3-F | - |
| 82 | 3-MeCONH-Ph | 3 | H | H | H | 2,4-di F | - |
| 83 | 4-(HOCH₂)Ph | 3 | H | H | H | 2,4-di F | - |
| 84 | 3-(HOCH₂)Ph | 3 | H | H | H | 2,4-di F | - |
| 85 | 4-Me₂NCO-Ph | 3 | H | H | H | 2,4-di F | - |
| 86 | 4-MeOCONH-Ph | 3 | H | H | H | 2,4-di F | - |
| 87 | 4-MeNHCO-Ph | 3 | H | H | H | 2,4-di F | - |
| 88 | 4-MeCONH-Ph | 3 | H | H | H | 2,4-di F | - |
| 89 | 4-(morpholin-4-yl)-Ph | 3 | H | H | H | 2,4-di F | - |
| 90 | 3-(IsPrCONH)Ph | 3 | H | H | H | 2,4-di F | - |
| 91 | 3-Me₂NSO₂-Ph | 3 | H | H | H | 2,4-di F | - |
| 92 | 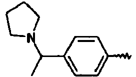 | 3 | H | H | H | 2,4-di F | - |
| 93 | 3-F-4-( Me₂NCO)-Ph | 3 | H | H | H | 2,4-di F | - |

| | | | | | | | |
|---|---|---|---|---|---|---|---|
| • * Pour les composés 8 et 9, X est en position 4 et 2 respectivement • ** Pour les composés 45 et 47, X est en position 3 • Les composés 29 et 30 sont sont les énantiomères du produit racémique 22. | | | | | | | |

**Tableau 3**

| **Ex** | **PF / [M+H]** | **RMN** Spectre RMN ¹H (DMSO-d6, δ en ppm) : |
|---|---|---|
| **1** | 200-202 | 4,65 (d, 2H) ; 5,35 (t, 1H) ; 7,35 (t, 1H) ; de 7,4 à 7,6 (m, 5H) ; 7,7 (d, 1H) ; 8,0 (d, 2H) ; 8,5 (s, 1H) ; 8,8 (s, 1H). |
| **2** | 193-195 | 1,4 (d, 3H) ; 5,1 (m, 1H) ; 5,35 (d, 1H) ; 7,35 (m, 1H) ; de 7,45 à 7,65 (m, 5H) ; 7,7 (d, 1H) ; 8,05 (d, 2H) ; 8,5 (s, 1H) ; 8,75 (s, 1H). |
| **3** | 214-216 | 4,6 (d, 2H) ; 5,3 (m, 1H) ; 7,25 (m, 1H) ; 7,45 (d, 1H) ; 7,55 (d, 2H) ; de 7,6 à 7,7 (m, 2H) ; 8,0 (d, 2H) ; 8,5 (s, 1H) ; 8,75 (s, 1H). |
| **4** | 195-197 | 1,5 (d, 3H) ; 5,2 (m, 1H) ; 5,4 (d, 1H) ; 7,2 (m, 1H); 7,4 (d, 1H) ; de 7,5 à 7,6 (m, 3H); 7,7 (d, 1H) ; 8,05 (d, 2H) ; 8,5 (s, 1H) ; 8,75 (s, 1H). |
| **5** | 208-210 | 1,45 (d, 3H) ; 5,05 (m, 1H) ; 5,4 (d, 1H) ; 7,25 (m, 1H) ; 7,5 (d, 2H) ; de 7,55 à 7,75 (m, 3H); 7,85 (m, 1H); 8,05 (d, 2H) ; 8,45 (s, 1H) ; 8,9 (s, 1H). |
| **6** | 156-156.5 | 2,4 (s, 3H) ; 4,55 (d, 2H) ; 5,2 (m, 1H) ; 7,2 (s, 1H) ; de 7,4 à 7,55 (m, 4H); 7,6 (d, 1H) ; 7,7 (d, 1H) ; 8,05 (d, 2H) ; 8,45 (s, 1H) ; 8,9 (s, 1H). |
| **7** | 156.2-157.4 | 3,85 (s, 3H) ; 4,55 (m, 2H) ; 5,3 (t, 1H) ; 6,95 (s, 1H) ; 7,15 (s, 1H) ; 7,25 (s, 1H) ; 7,55 (d, 2H) ; de 7,6 à 7,7 (m, 2H) ; 8,05 (d, 2H) ; 8,45 (s, 1H) ; 8,9 (s, 1H). |
| **8** | 232.3-233.7 | 1,75 (m, 2H) ; 1,95 (m, 2H) ; 2,75 (m, 2H) ; 4,65 (m, 1H) ; 5,2 (m, 1H) ; 7,2 (m, 1H) ; 7,5 (m, 3H) ; 7,6 (m, 1H) ; 7,7 (m, 1H) ; 7,75 (m, 1H) ; 8,05 (d, 2H) ; 8,45 (s, 1H) ; 8,85 (s, 1H). |
| **9** | 218.1-219.6 | de 1,6 à 2,0 (m, 4H) ; de 2,55 à 2,7 (m, 2H) ; 4,65 (m, 1H) ; 5,2 (d, 1H) ; 7,2 (d, 1H) ; de 7,25 à 7,35 (m, 2H) ; 7,5 (m, 3H) ; 7,6 (d, 1H) ; 8,05 (d, 2H) ; 8,4 (s, 1H); 8,5 (s, 1H). |
| **10** | 91.8-92.5 | 2,3 (s, 3H) ; 4,55 (d, 2H) ; 5,2 (t, 1H) ; de7,25 à 7,35 (m, 4H) ; 7,5 (d, 2H); 7,65 (d, 1H); 8,0 (d, 2H); 8,45 (s, 1H); 8,55 (s, 1H). |
| **11** | 171.9-172.6 | 4,65 (d, 2H) ; 5,4 (t, 1H) ; 7,35 (m, 1H) ; 7,55 (d, 2H) ; 7,65 (d, 1H) ; de 7,7 à 7,8 (m, 2H) ; 7,85 (d, 1H) ; 8,05 (d, 2H) ; 8,5 (s, 1H) ; 8,9 (s, 1H). |
| **12** | 183.4-184.1 | 2,3 (s, 3H); 4,6 (d, 2H); 5,15 (t, 1H) ; 7,25 (d, 1H); 7,55 (m, 3H) ; 7,6 (d, 1H) ; de 7,65 à 7,75 (m, 2H) ; 8,05 (d, 2H) ; 8,45 (s, 1H) ; 8,85 (s, 1H). |
| **13** | 203.4-204 | 2,2 (s, 3H) ; 4,55 (d, 2H) ; 5,2 (t, 1H) ; de 7,2 à 7,35 (m, 3H) ; de 7,45 à 7,55 (m, 3H) ; 7,65 (d, 1H) ; 8,05 (d, 2H) ; 8,4 (s, 1H); 8,45 (s, 1H). |
| **14** | 222.5-223 | 2,35 (s, 3H) ; 4,6 (m, 2H) ; 5,3 (t, 1H) ; 7,3 (d, 2H) ; de 7,55 à 7,7 (m, 5H) ; 7,9 (d, 2H) ; 8,35 (s, 1H) ; 8,95 (s, 1H). |
| **15** | 220-221 | 2,4 (s, 3H) ; 4,45 (m, 2H) ; 5,3 (m, 1H) ; de 7,25 à 7,35 (m, 4H) ; de 7,4 à 7,55 (m, 2H) ; 7,65 (d, 1H) ; 7,9 (d, 2H) ; 8,4 (s, 1H) ; 8,65 (m, 1H). |
| **16** | 190-191 | 3,85 (s, 3H) ; 4,45 (m, 2H) ; 5,3 (m, 1H) ; 6,9 (m, 1H) ; de 7,25 à 7,5 (m, 5H); de 7,55 à 7,65 (m, 2H) ; 7,7 (d, 1H) ; 8,5 (s, 1H) ; 8,7 (s, 1H). |
| **17** | 281-283 | 3,9 (s, 3H) ; 4,65 (s, 2H) ; 7,1 (d, 1H) ; 7,55 (t, 1H) ; de 7,6 à 7,75 (m, 5H); 8,0 (d, 1H); 8,25 (d, 1H); 8,75 (s, 1H); 9,25 (s, 1H). |
| **18** | 305-306 | 4,65 (d, 2H) ; de 7,5 à 7,75 (m, 6H) ; 8,0 (d, 1H) ; 8,05 (d, 2H); 8,2 (d, 1H); 8,7 (s, 1H) ; 9,3 (s, 1H). |
| **19** | 143-144 | 3,85 (s, 3H) ; 4,65 (d, 2H) ; 5,35 (t, 1H) ; 6,95 (d, 1H) ; de 7,3 à 7,4 (m, 2H) ; 7,45 (d, 1H) ; de 7,5 à 7,65 (m, 4H) ; 7,7 (d, 1H) ; 8,5 (s, 1H) ; 8,75 (s, 1H). |
| **20** | 257-258 | 4,45 (d, 2H) ; de 7,3 à 7,45 (m, 2H) ; de 7,5 à 7,7 (m, 4H) ; de 8,0 à 8,15 (m, 4H) ; 8,9 (s, 1H) ; 9,0 (s, 1H). |
| **21** | 208.5-209.5 | 2,35 (s, 3H) ; 4,65 (d, 2H) ; 5,35 (t, 1H) ; 7,3 (d, 2H) ; 7,35 (m, 1H) ; 7,45 (d, 1H) ; 7,55 (m, 2H) ; 7,7 (d, 1H) ; 7,9 (d, 2H) ; 8,45 (s, 1H) ; 8,85 (s, 1H). |
| **22** | 180-182 | 1,55 (d, 3H) ; 5,0 (q, 1H) ; de 7,15 à 7,25 (m, 1H) ; de 7,3 à 7,6 (m, 6H) ; 7,75 (d, 1H); de 7,85 à 8,0 (m, 3H) ; 8,4 (s, 1H). |
| **23** | 228-229 | 4,55 (d, 2H) ; 5,25 (t, 1H); de 7,2 à 7,6 (m, 6H) ; 7,65 (d, 1H); 8,0 (d, 2H) ; 8,45 (s, 1H); 8,75 (s, 1H). |
| **24** | 200-202 | 3,85 (s, 3H) ; 4,7 (s, 2H) ; 7,05 (m, 1H) ; de 7,35 à 7,5 (m, 5H) ; 7,75 (m, 1H) ; de 7,85 à 8,0 (m, 3H) ; 8,35 (s, 1H). |
| **25** | 210-212 | 1,3 (d, 3H) ; 3,75 (s, 3H) ; 4,7 (m, 1H) ; 5,05 (d, 1H) ; 7,05 (d, 1H) ; de 7,3 à 7,6 (m, 6H) ; 7,95 (d, 2H) ; 8,45 (s, 1H) ; 8,6 (s, 1H). |
| **26** | 216-217 | 4,6 (d, 4H) ; 5,2 (t, 2H) ; 7,3 (s, 1H) ; 7,5 (m, 4H) ; de 7,6 à 7,7 (m, 2H) ; 7,95 (d, 2H) ; 8,45 (s, 1H) ; 8,85 (s, 1H). |
| **27** | 195-197 | 4,65 (d, 2H) ; 5,35 (t, 1H) ; 7,35 (t, 1H) ; de 7,45 à 7,55 (m, 5H) ; 7,7 (d, 1H) ; 7,95 (d, 1H) ; 8,05 (m, 2H) ; 8,15 (d, 1H) ; 8,7 (m, 2H) ; 8,85 (s, 1H). |
| **28** | 278-280 | 4,65 (s, 2H) ; 7,4 (t, 1H) ; de 7,55 à 7,7 (m, 5H) ; 8,05 (m, 4H) ; 8,8 (s, 1H); 9,1 (s, 1H). |
| **29** | 187-187,5 | 1,4 (s, 3H) ; 4,8 (m, 1H) ; 5,25 (s, 1H) ; 7,3 (t, 1H) ; de 7,4 à 7,55 (m, 4H) ; 7,6 (d, 1H) ; 7,7 (d, 1H) ; 8,0 (d, 2H) ; 8,5 (s, 1H) ; 8,8 (s, 1H) Alpha D : +18,1 (c = 0,502, méthanol) ; +13,3 (c = 0,446, DMSO).. |
| **30** | 184-185 | 1,4 (s, 3H) ; 4,8 (m, 1H) ; 5,25 (s, 1H) ; 7,3 (t, 1H) ; de 7,4 à 7,55 (m, 4H) ; 7,6 (d, 1H) ; 7,7 (d, 1H) ; 8,0 (d, 2H) ; 8,5 (s, 1H) ; 8,8 (s, 1H). Alpha D : -13,2 (c = 0,318, méthanol) ; -13,5 (c = 0,308, DMSO). |
| **31** | 216,5-217,5 | 4,45 (d, 2H) ; 5,25 (t, 1H) ; de 7,2 à 7,35 (m, 3H) ; de 7,4 à 7,55 (m, 3H) ; 7,7 (d, 1H) ; 8,35 (m, 2H) ; 8,75 (s, 1H). |
| 32 | 199-199,5 | 4,6 (d, 2H) ; 5,35 (t, 1H) ; 7,25 (m, 2H) ; 7,4 (t, 1H) ; 7,45 (d, 1H) ; 7,65 (q, 1H) ; 7,75 (d, 1H) ; 8,35 (q, 1H) ; 8,4 (d, 1H) ; 8,85 (s, 1H). |
| 33 | 144-145 | 1,5 (s, 6H) ; 3,85 (s, 3H) ; 5,15 (s, 1H) ; 6,95 (d, 1H); 7,3 (t, 1H) ; 7,4 (t, 1H) ; 7,5 (d, 1H) ; de 7,55 à 7,65 (m, 3H) ; 7,7 (d, 2H) ; 8,5 (s, 1H) ; 8,8 (s, 1H). |
| 34 | 148-149 | 1,4 (d, 3H) ; 3,85 (s, 3H) ; 4,85 (m, 1H) ; 5,3 (s, 1H) ; 6,95 (d, 1H); de 7,3 à 7,5 (m, 4H) ; 7,6 (m, 3H) ; 7,7 (d, 1H) ; 8,5 (s, 1H) ; 8,8 (s, 1H). |
| 35 | 191-192 | 1,65 (s, 6H) ; 5,3 (s, 1H) ; 7,15 (m, 1H) ; 7,4 (m, 1H) ; 7,55 (m, 3H) ; 7,7 (m, 1H) ; 8,05 (d, 2H) ; 8,5 (s, 1H) ; 8,75 (s, 1H). |
| 36 | 196-197 | 1,3 (d, 3H) ; 4,95 (m, 1H) ; 5,20 (d, 1H) ; 7,15 (d, 1H) ; 7,3 (m, 2H) ; de 7,5 à 7,6 (m, 2H) ; de 7,65 à 7,75 (m, 2H) ; 7,95 (d, 1H) ; de 8,0 à 8,1 (m, 2H) ; 8,15 (d, 1H) ; de 8,55 à 8,65 (m, 3H). |
| 37 | 108-110 | 2,35 (s, 3H) ; 3,3 (m, 3H) ; 4,55 (s, 2H) ; de 7,25 à 7,35 (m, 3H) ; 7,45 (d, 1H) ; de 7,65 à 7,75 (m, 2H) ; 7,9 (d, 2H) ; 8,4 (s, 1H) ; 8,75 (s, 1H). |
| 38 | [364] | 4,45 (d, 2H) ; 5,3 (t, 1H) ; de 7,25 à 7,35 (m, 2H) ; de 7,45 à 7,5 (m, 2H) ; 7,7 (d, 1H) ; de 8,25 à 8,35 (m, 4H) ; 8,7 (s, 2H). |
| 39 | [406] | De 1,95 à 2,0 (m, 4H) ; de 3,3 à 3,35 (m,4H) ; 4,6 (d, 2H) ; 5,3 (s, 1H) ; 6,6 (d, 2H) ; 7,25 (t, 1H) ; 7,35 (d, 1H) ; de 7,6 à 7,65 (m, 2H) ; 7,8 (d, 2H) ; 8,2 (s, 1H) ; 8,7 (s, 1H). |
| 40 | [373] | 4,6 (d, 2H) ; 5,35 (t, 1H) ; 7,25 (t, 1H) ; 7,45 (d, 1H) ; de 7,5 à 7,6 (m, 1H) ; de 7,6 à 7,7 (m, 2H) ; de 7,8 à 7,85 (m, 1H) ; de 7,95 à 8,05 (m, 1H) ; 8,5 (s, 1H) ; 8,75 (s, 1H). |
| 41 | [355] | 4,6 (s, 2H) ; 5,35 (s, 1H) ; 7,25 (t, 1H) ; de 7,3 à 7,5 (m, 4H) ; 7,6 (m, 1H) ; 7,7 (d, 1H) ; 8,3 (t, 1H) ; 8,4 (s, 1H) ; 8,8 (s, 1H). |
| 42 | [415] | 4,6 (d, 2H) ; 5,35 (t, 1H) ; 7,25 (t, 1H) ; de 7,4 à 7,45 (m, 2H) ; 7,5 (d, 1H) ; de 7,6 à 7,7 (m, 2H) ; 8,0 (d, 1H) ; 8,2 (s, 1H) ; 8,55 (s, 1H) ; 8,85 (s, 1H). |
| 43 | [415] | 3,25 (s, 3H) ; 4,6 (s, 2H) ; 5,35 (s, 1H) ; 7,25 (t, 1H) ; 7,45 (d, 1H) ; de 7,6 à 7,65 (m, 1H) ; 7,7 (d, 1H) ; 8,0 (d, 2H) ; 8,25 (d, 2H) ; 8,65 (s, 1H) ; 8,8 (s, 1H). |
| 44 | 215-217 | 1,6 (s, 6H) ; 2,5 (m, 3H) ; 5,0 (s, 1H) ; de 7,15 à 7,25 (m , 3H) ; 7,35 (m, 1H) ; 7,45 (m, 2H) ; 7,55 (d, 1H) ; 7,65 (d, 1H) ; 8,0 (d, 2H) ; 8,4 (s, 1H) ; 8,45 (s, 1H). |
| 45 | 210-212 | 2,0 (m, 1H) ; 2,2 (m, 1H) ; 2,8 (m, 1H) ; 3,25 (m, 1H) ; 5,15 (s, 2H) ; 7,25 (m, 1H) ; de 7,3 à 7,5 (m, 4H) ; 7,7 (s, 2H) ; 8,35 (m, 2H) ; 8,9 (s, 1H). |

| **Ex** | **PF/[M+H]** | **RMN** Spectre RMN ¹H (DMSO-d₆, δ en ppm) : |
|---|---|---|
| 46 | 135-136 | 3,25 (s, 3H) ; 3,5 (t, 2H) ; 3,65 (t, 2H) ; 4,6 (s, 2H) ; de 7,25 à 7,35 (m, 1H) ; 7,45 (d, 1H) ; 7,55 (d, 2H) ; de 7,65 à 7,75 (m, 2H) ; 8,0 (d, 2H) ; 8,5 (s, 1H) ; 8,8 (s, 1H). |
| 47 | 172-174 | 1,2 (s, 3H) ; de 2,0 à 2,15 (m, 2H) ; 2,8 (m, 1H); 3,05 (m, 1H) ; 5,15 (s, 1H) ; 7,1 (m, 1H) ; de 7,2 à 7,35 (m, 3H) ; 7,4 (t, 1H) ; 7,55 (d, 1H) ; 7,65 (s, 1H) ; 8,35 (m, 2H) ; 8,65 (s, 1H). |
| 48 | 174-176 | 1,6 (s, 6H) ; 3,35 (s, 3H) ; 5,05 (s, 1H) ; 7,2 (t, 1H) ; 7,35 (d, 1H) ; 7,45 (d, 1H) ; 7,55 (d, 2H) ; 7,7 (m, 2H) ; 8,0 (d, 2H) ; 8,5 (s, 1H) ; 8,7 (s, 1H). |
| 49 | 220-222 | 1,4 (d, 3H) ; 3,45 (s, 3H) ; 5,15 (m, 2H) ; 7,25 (t, 1H) ; 7,35 (d, 1H) ; de 7,45 à 7,6 (m, 4H) ; 7,65 (d, 1H) ; 8,05 (d, 2H) ; 8,5 (s, 1H) ; 8,75 (s, 1H). |
| 50 | [387] | 4,45 (d, 2H) ; 5,3 (t, 1H) ; 7,3 (m, 2H) ; 7,45 (m, 2H) ; 7,7 (d, 1H) ; 7,8 (m, 2H) ; 8,2 (d, 2H) ; 8,6 (s, 1H) ; 8,7 (s, 1H). |
| 51 | [349] | 3,8 (s, 3H) ; 4,4 (m, 2H) ; 5,3 (t, 1H) ; 7,0 (d, 2H) ; 7,25 (m, 2H) ; 7,4 (d, 1H) ; 7,45 (m, 1H) ; 7,65 (d, 1H) ; 7,9 (d, 2H) ; 8,35 (s, 1H) ; 8,65 (s, 1H). |
| 52 | [388] | 1,95 (m, 4H) ; 3,3 (m, 4H) ; 4,6 (d, 2H) ; 5,3 (t, 1H) ; 6,6 (d, 2H) ; 7,25 (m, 2H) ; 7,35 (d, 1H) ; de 7,4 à 7,5 (m, 1H) ; 7,6 (d, 1H) ; 7,8 (d, 2H) ; 8,2 (s, 1H) ; 8,6 (s, 1H). |
| 53 | [337] | 4,45 (d, 2H) ; 5,3 (t, 1H) ; 7,3 (m, 4H) ; de 7,4 à 7,5 (m, 2H) ; 7,65 (d, 1H) ; 8,05 (m, 2H) ; 8,45 (s, 1H) ; 8,65 (s, 1H). |
| 54 | [355] | 4,45 (d, 2H) ; 5,35 (t, 1H) ; 7,25 (m, 2H) ; de 7,4 à 7,55 (m, 3H) ; 7,65 (d, 1H) ; 7,85 (m, 1H) ; 8,0 (m, 1H) ; 8,5 (s, 1H) ; 8,7 (s, 1H). |
| 55 | [344] | 4,45 (d, 2H) ; 5,3 (t, 1H) ; 7,25 (m, 2H) ; 7,45 (m, 2H) ; 7,65 (m, 2H) ; 7,8 (d, 1H) ; 8,3 (d, 1H) ; 8,4 (s, 1H) ; 8,6 (s, 1H) ; 8,7 (s, 1H). |
| 56 | [337] | 4,45 (d, 2H) ; 5,25 (t, 1H) ; de 7,2 à 7,4 (m, 5H) ; 7,45 (m, 2H) ; 7,7 (d, 1H) ; 8,3 (t, 1H) ; 8,4 (s, 1H) ; 8,75 (s, 1H). |

| **Ex** | **PF / [M+H]** | **RMN** Spectre RMN ¹H (DMSO-d6, δ en ppm) : |
|---|---|---|
| 57 | [399] | 4,45 (d, 2H) ; 5,3 (t, 1H) ; 7,25 (m, 2H) ; de 7,4 à 7,5 (m, 3H) ; 7,55 (m, 1H) ; 7,65 (d, 1H) ; 8,0 (d, 1H) ; 8,2 (s, 1H) ; 8,55 (s, 1H) ; 8,65 (s, 1H). |
| 58 | [367] | 2,4 (s, 3H) ; 4,45 (d, 2H) ; 5,3 (t, 1H) ; 7,25(m, 2H) ; de 7,4 à 7,5 (m, 3H) ; 7,65 (d, 1H) ; 7,85 (m, 1H) ; 8,0 (d, 1H) ; 8,5 (m, 1H) ; 8,65 (m, 1H). |
| 59 | [367] | 2,35 (s, 3H) ; 4,45 (d, 2H) ; 5,35 (t, 1H) ; 7,25(m, 2H) ; de 7,4 à 7,5 (m, 3H) ; 7,65 (d, 1H) ; 7,85 (m, 1H) ; 8,0 (m, 1H) ; 8,5 (m, 1H) ; 8,65 (m, 1H). |
| 60 | [397] | 3,25 (m, 3H) ; 4,4 (d, 2H) ; 5,3 (t, 1H) ; 7,25 (m, 2H) ; 7,45 (m, 2H) ; 7,7 (d, 1H) ; 8,0 (d, 2H) ; 8,25 (d, 2H) ; 8,65 (s, 1H) ; 8,7 (s, 1H). |
| 61 | [367] | 3,8 (s, 3H) ; 4,6 (d, 2H) ; 5,35 (t, 1H) ; 7,0 (d, 2H) ; 7,25 (t, 1H) ; 7,4 (d, 1H) ; de 7,55 à 7,65 (m, 2H) ; 7,9 (d, 2H) ; 8,35 (s, 1H) ; 8,75 (s, 1H). |
| 62 | [355] | 4,6 (d, 2H) ; 5,35 (t, 1H) ; de 7,2 à 7,3 (m, 3H) ; 7,4 (d, 1H) ; de 7,6 à 7,7 (m, 2H) ; 8,05 (m, 2H) ; 8,45 (s, 1H) ; 8,75 (s, 1H). |
| 63 | [362] | 4,6 (d, 2H) ; 5,35 (t, 1H) ; 7,25 (t, 1H) ; 7,45 (d, 1H) ; de 7,6 à 7,75 (m, 3H) ; 7,8 (d, 1H) ; 8,3 (d, 1H) ; 8,4 (s, 1H) ; 8,6 (s, 1H) ; 8,8 (s, 1H). |
| 64 | [385] | 2,4 (s, 3H) ; 4,55 (d, 2H) ; 5,35 (t, 1H) ; 7,25 (t, 1H) ; 7,4 (m, 1H) ; 7,5 (m, 1H) ; de 7,6 à 7,7 (m, 2H) ; 7,85 (m, 1H) ; 8,0 (m, 1H) ; 8,45 (m, 1H) ; 8,75 (m, 1H). |
| 64 bis | [385] | 2,35 (s, 3H) ; 4,55 (d, 2H) ; 5,35 (t, 1H) ; 7,25 (t, 1H) ; 7,4 (m, 1H) ; 7,5 (m, 1H) ; de 7,6 à 7,7 (m, 2H) ; 7,85 (m, 1H) ; 8,0 (m, 1H) ; 8,45 (m, 1H) ; 8,75 (m, 1H). |
| 65 | [383] | 2,5 (m, 3H) ; 4,6 (d, 2H) ; 5,35 (t, 1H) ; 7,25 (t, 1H) ; 7,35 (d, 2H) ; 7,4 (d, 1H) ; de 7,6 à 7,7 (m, 2H) ; 7,95 (d, 2H) ; 8,45 (s, 1H) ; 8,75 (s, 1H). |
| 66 | 230-231 | 1,65 (s, 6H) ; 5,35 (s, 1H) ; 7,3 (d, 1H) ; de 7,35 à 7,45 (m, 2H) ; 7,55 (d, 2H) ; 7,65 (d, 1H) ; 7,95 (d, 1H) ; 8,05 (d, 2H) ; 8,45 (s, 1H) ; 8,6 (s, 1H). |
| 67 | 170-172 | 1,4 (d, 3H) ; 5,15 (m, 1H) ; 5,45 (d, 1H) ; 7,3 (d, 1H) ; 7,4 (d, 1H) ; de 7,45 à 7,55 (m, 3H) ; 7,65 (d, 1H) ; 7,75 (d, 1H) ; 8,05 (d, 2H) ; 8,45 (s, 1H) ; 8,6 (s, 1H). |
| 68 | 229-230 | 4,45 (d, 2H) ; 5,3 (t, 1H) ; 7,3 (m, 2H) ; 7,55-7,4 (m, 4H) ; 7,7 (d, 1H) ; 8,05 (d, 2H) ; 8,5 (s, 1H) ; 8,7 (s, 1H |
| 69 | 104-105 | 3.45 (s, 3H) ; 3,65 (t, 2H) ; 3,75(t, 2H) ; 4,5 (s, 2H) ; 7,15 (m, 1H) ; 7,25 (d, 1H) ; 7,35 (d, 1H) ; 7,45 (m, 3H) ; 7,7 (d, 1H) ; 7,95 (m, 3H) ; 8,65 (s, 1H). |

**Tableau 4**

| Ex | PF / [M+H] | RMN Spectre RMN ¹H (DMSO-d6, δ en ppm) : |
|---|---|---|
| 70 | [376] | 2,07 (s, 3H) ; 4,42 (s, 2H) ; 5,30 (s, 1H) ; 7,25 (m, 2H) ; 7,40 (m, 1H) ; 7,50 (m, 3H) ; 7,60 (d, 1H) ; 7,70 (d, 1H) 8,30 (s, 1H) ; 8,42 (s, 1H) ; 8,70 (s, 1H) ; 10,00 (s, 1H). |
| 71 | [349] | 4,42 (s, 2H) ; 4,55 (s, 2H) ; 5,20 (s, 1H) ; 5,30 (s, 1H) ; 7,30 (m, 2H) ; 7,40 (d, 2H) ; 7,50 (m, 2H) ; 7,70 (d, 1H) ; 7,95 (d, 2H) ; 8,45 (s, 1H) ; 8,70 (s, 1H). |
| 72 | [349] | 4,42 (s, 2H) ; 4,58 (s, 2H) ; 5,30 (m, 2H) ; 7,30 (m, 2H) ; 7,45 (m, 3H) ; 7,60 (s, 1H) ; 7,7 (d, 1H) ; 7,85 (d, 1H) ; 8,0 (s, 1H) ; 8,52 (s, 1H) ; 8,75 (s, 1H). |
| 73 | [412] | 3,05 (s, 3H) ; 4,45 (s, 2H) ; 5,25 (s, 1H) ; 7,25 (m, 4H) ; 7,45 (m, 2H) ; 7,65 (d, 1H) ; 7,95 (m, 2H) ; 8,4 (s, 1H) ; 8,7 (s, 1H) ; 9,9 (s, 1H). |
| 74 | [390] | 3,00 (s, 6H) ; 4,45 (s, 2H) ; 5,35 (s, 1H) ; 7,30 (m, 2H) 7,50 (m, 4H) ; 7,70 (d, 1H) ; 8,05 (d, 2H) ; 8,57 (s, 1H) ; 8,72 (s, 1H). |
| 75 | [392] | 3,70 (s, 3H) ; 4,45 (s, 2H) ; 5,30 (s, 1H) ; 7,30 (m, 2H) ; 7,50 (m, 4H) ; 7,65 (d, 1H) ; 7,90 (d, 2H) ; 8,38 (s, 1H) ; 8,68 (s, 1H).; 9,80 (s, 1H). |
| 76 | [376] | 2,80 (d, 3H) ; 4,45 (s, 2H) ; 5,35 (s, 1H) ; 7,30 (m, 2H) ; 7,48 (m, 2H) ; 7,70 (d, 1H) ; 7,92 (d, 2H) ; 8,10 (d, 2H) ; 8,45 (q, 1H), 8,55 (s, 1H) ; 8,70 (s, 1H). |
| 77 | [376] | 2,10 (s, 3H) ; 4,45 (s, 2H) ; 5,30 (s, 1H) ; 7,30 (m, 2H) ; 7,48 (m, 2H) ; 7,68 (m, 3H) ; 7,92 (d, 2H) ; 8,40 (s, 1H) ; 8,70 (s, 1H) ; 10,00 (s, 1H). |
| 78 | [404] | 3,20 (m, 4H) ; 3,75 (m, 4H ; 4,45 (s, 2H) ; 5,30 (s, 1H) ; 7,05 (m, 2H) ; 7,25 (m, 2H) ; 7,45 (m, 2H) ; 7,65 (d, 1H) ; 7,85 (m, 2H) ; 8,35 (s, 1H) ; 8,70 (s, 1H). |
| 79 | [426] | 2,70 (s, 6H) ; 4,40 (s, 2H) ; 5,30 (s, 1H) ; 7,30 (m, 2H) ; 7,45 (m, 2H) ; 7,70 (m, 3H) ; 8,30 (m, 2H) ; 8,70 (d, 2H) |
| 80 | [416] | 1,65 (d, 3H) ; de 1,8 à 1,95 (m, 4H) ; de 2,5 à 2,55 (m,2H) ;de 2,95 à 3,05 (m, 2H) ; 3,2 (q, 1H) ; 4,45 (d, 2H) ; 5,35 (t, 1H) ; de 7,25 à 7,35 (m, 4H) ; de 7,4 à 7,5 (m, 2H) ; 7,65 (d, 1H) ; 8,1 (d, 2H) ; 8,55 (s, 1H) ; 8,7 (s, 1H). |
| 81 | [408] | 2,7 (m, 6H) ; 4,40 (s, 2H) ; 5,30 (m, 1H) ; 7,30 (m, 2H) ; 7,45 (m, 2H) ;7,75 (m, 2H) ; 7,85 (m,2H) ; 8,60(s, 1H) ; 8,70(s, 1H) |
| 82 | [394] | 2,1 (s, 3H) ; 4,60 (s, 2H) ; 5,3 (m, 1H) ; 7,25 (m, 1H) ; 7,40 (m, 1H) ; 7,60 (m, 4H) ; 7,75 (s, 1H) 8,3 (s, 1H) ; 8,45 (m, 1H) ;8,85 (s, 1H) ; 10 (s, 1H) |
| 83 | [367] | 4,55 (s, 2H) ; 4,60 (s, 2H) ; 5,2 (m, 1H) ; 5,35 (m, 1H) ; 7,25 (t, 1H) ; 7,40 (m, 3H) ; 7,65 (m, 2H) ; 7,95 (m, 2H) ;8,45 (s, 1H) ; 8,75 (s, 1H) : |
| 84 | [367] | 4,55 (m, 4H) ; 5,30 (m, 2H) ; 7,25 (m, 2H) ; 7,45 (m, 2H) ;7,60 (m, 1H) ; 7,70 (m, 1H) ; 7,85 (d, 1H) ; 7,95 (s, 1H) ; 8,45(s, 1H) ; 8,75 (s, 1H) ; |
| 85 | [408] | 2,95 (s, 6H) ; 4,60 (d, 2H) ; 5,35 (t, 1H) ; 7,25 (t, 1H) ; 7,45 (m, 3H) ; 7,65(m, 2H) ;8,05 (m,2H) ;8,50 (s, 1H) ; 8,75 (m, 1H) |
| 86 | [410] | 3,70 (s, 3H); 4,55 (s, 2H) ; 5,35 (m, 1H) ; 7,25 (m, 1H) ; 7,4 (m, 1H) ; 7,55 (d, 2H) ; 7,65 (m, 2H) ; 7,90 (d, 2H) ; 8,3 (s,1H) ; 8,75 (s, 1H) ; 9,80 (s; 1H) |
| 87 | [394] | 2,80 (d, 3H) ; 4,60 (d, 2H) ; 5,85 (t, 1H) ; 7,25 (t, 1H) ; 7,45 (m, 1H) ; 7,65(m, 1H) ; 7,70 (d, 1H) ; 7,90 (d, 2H) ; 8,05 (d,2H) ;8,45 (m, 1H) ; 8,55 (s, 1H) ; 8,75 (s, 1H); |
| 88 | [394] | 2,05 (s, 3H) ; 4,60 (d, 2H) ; 5,35(t, 1H) ; 7,25 (t, 1H) ;7,40 (d, 1H) ;7,65 (m, 4H) ; 7,90 (d, 2H) ;8,35 (s, 1H) ; 8,75 (s, 1H) ; 10,0 (s, 1H) |
| 89 | [422] | 3,2 (m, 4H) ; 3,75 (m, 4H) ; 4,6 (m, 2H) ; 5,35 (m, 1H) ; 7,03 (d, 2H) ; 7,27 (m, 1H); 7,5 (m, 1H) ; 7,68 (m, 2H) ; 7,84 (d, 2H); 8,37 (s, 1H) ; 8,78 (s, 1H). |
| 90 | [422] | 1,12 (d, 6H) ; 2,6 (d, 1H) ; 4,6 (t, 2H) ; 5,35 (m, 1H) ; 7,22 (m, 1H) ; 7,35 (m, 1H) ; 7,42 (m, 1H) ; 7,63 (m, 4H) ; 8,3 (s, 1H) ; 8,4 (s, 1H) ; 8,78 (s, 1H); 9,98 (s, 1H). |
| 91 | [444] | 2,68 (s, 6H) ; 4,6 (m, 2H) ; 5,35 (m, 1H) ; 7,25 (m, 1H) ; 7,47 (d, 1H) ; 7,62 (m, 1H) ; 7,72 (m, 3H) ; 8,3 (d, 1H) ; 8,35 (s, 1H) ; 8,65 (s, 1H) ; 8,79 (s, 1H). |
| 92 | [434] | 1,65 (d, 3H ; de 1,8 à 2,05 (m, 4H) ; de 2,5 à 2,6 (m,2H) ;de 2,9 à 3,0 (m, 2H) ; 3,2 (q, 1H) ; 4,6 (d, 2H) ; 5,35 (t, 1H) ; 7,25 (m, 2H) ; 7,4 (m, 1H) ; de 7,55 à 7,7 (m, 4H) ; 8,1 (d, 1H) ; 8,5 (s, 1H) ; 8,75 (s, 1H). |
| 93 | [426] | 2,9 (s, 6H) ; 4,58 (m, 2H) ; 5,35 (m, 1H) ; 7,28 (m, 1H) ; 7,5 (m, 2H) ; 7,7 (m, 3H) ; 7,9 (m, 1H) ; ;8,6 (s, 1H) ; 8,79 (s, 1H). |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,01 et 10 µM. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.

Par exemple, les composés n° 3, 8, 11, 17, 29, 33, 37, 38, 42 ont montré une EC₅₀ de respectivement 0,5 ; 42 ; 7,5 ; 93 ; 0,1 ; 25 ; 74 ; 211 ; 1 nM. Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcereuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées; l'ostéoporose; les cancers.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composés de formule (I) : 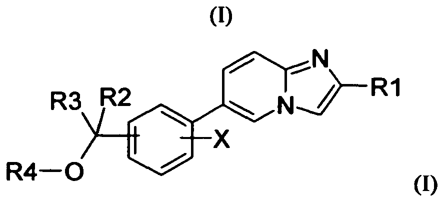 dans laquelle :
R₁ représente :
un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₁₀)alkyle, halo(C₁-C₁₀)alkyle, (C₁-C₁₀)alcoxy, halo(C₁-C₁₀)alcoxy, (C₁-C₁₀)thioalkyle, -S(O)(C₁-C₁₀)alkyle, -S(O)₂(C₁-C₁₀-alkyle), hydroxyle, cyano, nitro, hydroxy(C₁-C₁₀)alkylène, NPaRb(C₁-C₁₀)alkylène, (C₁-C₁₀)alcoxy(C₁-C₁₀)alkylène-oxy, NRaRb, CONRaRb, SO₂NRaRb, NRcCORd, OC(O)NRaRb, OCO(C₁-C₁₀)alkyle, NRcC(O)ORe, NRcSO₂Re, aryl(C₁-C₁₀)alkylène, aryle ou hétéroaryle monocyclique, l'aryle ou l'hétéroaryle monocyclique étant éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, oxo, nitro, cyano ou OCO(C₁-C₁₀)alkyle;
X représente de 1 à 4 substituants identiques ou différents l'un de l'autre choisis parmi halogène, (C₁-C₁₀)alkyle, (C₁-C₁₀)alcoxy, NRaRb, cyano, nitro, le (C₁-C₁₀)alkyle pouvant être éventuellement substitué par un ou plusieurs groupes choisis parmi un halogène, (C₁-C₁₀)alcoxy, halo(C₁-C₁₀)alcoxy, NRaRb ou hydroxyle ;
R₂ et R₃ représentent, indépendamment l'un de l'autre,
un atome d'hydrogène,
un groupe (C₁-C₁₀)alkyle, ce groupe étant éventuellement substitué par un groupe Rf ;
un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro ou cyano ;
R₂ et X peuvent former ensemble avec les atomes de carbone qui les portent un cycle carboné de 5 à 7 atomes de carbone;
R₄ représente :
un atome d'hydrogène,
un groupe (C₁-C₁₀)alkyle, ce groupe étant éventuellement substitué par un groupe Rf ;
un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano, (C₁-C₁₀)alkyl(CO)-, CONRaRb, NRcCORd, OC(O)NRaRb, OCO(C₁-C₁₀)alkyle, NRcC(O)ORe ou aryle, l'aryle étant éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro ou cyano ;
Ra et Rb représentent, indépendamment l'un de l'autre,
un atome d'hydrogène ou un groupe (C₁-C₁₀)alkyle, aryl(C₁-C₁₀)alkylène ou aryle ;
ou Ra et Rb forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe (C₁-C₁₀)alkyle, aryle ou aryl(C₁-C₁₀)alkylène ;
Rc et Rd représentent, indépendamment l'un de l'autre,
un atome d'hydrogène ou un groupe (C₁-C₁₀)alkyle, aryl(C₁-C₁₀)alkylène, aryle ;
ou Rc et Rd forment ensemble un groupe (C₂-C₅)alkylène ;
Re représente
un groupe (C₁-C₁₀)alkyle, aryl(C₁-C₁₀)alkylène, aryle ;
ou Rc et Re forment ensemble un groupe (C₂-C₅)alkylène ;
Rf représente
un atome d'halogène, un groupe (C₁-C₁₀)alcoxy, halo(C₁-C₁₀)alcoxy, hydroxyle, cyano, NRaRb, C(O)NRaRb, NRcCORd, OC(O)NRaRb, OCO(C₁-C₁₀)alkyle, NRcCOORe, SO₂NRaRb, NPcSO₂Re, aryl(C₁-C₁₀)alkylène, aryle, l'aryle étant éventuellement substitué par un ou plusieurs substituants choisi parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano ou OCO(C₁-C₁₀)alkyle, à l'état de base ou de sel d'addition à un acide.

2. Composés de formule (I) selon la revendication 1, **caractérisés en ce que** :
R₁ représente un groupe phényle ou un groupe naphtyle, éventuellement substitué par un ou plusieurs atomes ou groupes choisis indépendamment les uns des autres parmi les atomes d'halogène ou groupes (C₁-C₁₀)alkyle, (C₁-C₁₀)alcoxy; nitro, -S(O)₂(C₁-C₁₀-alkyle), halo(C₁-C₁₀)alkyle, cyano, (C₁-C₁₀)thioalkyle, NRaRb; NRcCORd, hydroxy(C₁-C₁₀)alkylène, NRcSO₂Re, CONRaRb, NRcC(O)ORe, SO₂NRaRb, NRaRb(C₁-C₁₀)alkylène ;
Ra et Rb représentent, indépendamment l'un de l'autre, un atome d'hydrogène ou un groupe (C₁-C₁₀)alkyle;
ou Ra et Rb forment ensemble, avec l'atome d'azote qui les porte, un groupe pyrrolidinyle, morpholinyle ;
Rc et Rd représentent, indépendamment l'un de l'autre, un atome d'hydrogène ou un groupe (C₁-C₁₀)alkyle;
Re représente un groupe (C₁-C₁₀)alkyle.

3. Composés de formule (I) selon la revendication 1 ou 2, **caractérisés en ce que** :
X représente 1 ou 2 substituants identiques ou différents l'un de l'autre choisis parmi halogène, (C₁-C₁₀)alkyle, (C₁-C₁₀)alcoxy, le (C₁-C₁₀)alkyle pouvant être éventuellement substitué par un groupe hydroxyle ;
R₂ et X peuvent former ensemble avec les atomes de carbone qui les portent un cycle carboné de 5 ou 6 carbones.

4. Composés de formule (I) selon l'une quelconque des revendications précédentes, **caractérisés en ce que** :
R₂ et R₃ représentent indépendamment l'un de l'autre un un atome d'hydrogène ou un groupe (C₁-C₁₀)alkyle.

5. Composés de formule (I) selon l'une quelconque des revendications précédentes, **caractérisés en ce que** :
R₄ représente un atome d'hydrogène, un groupe (C₁-C₁₀)alkyle, ce groupe étant éventuellement substitué par un groupe Rf ;
Rf représente un groupe (C₁-C₁₀)alcoxy.

6. Composés de formule (I) selon l'une quelconque des revendications précédentes, **caractérisés en ce que** : la substitution du groupe 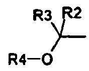 sur le noyau phényle est en position 2, 3 ou 4.

7. Composés de formule (I) selon l'une quelconque des revendications précédentes, **caractérisés en ce que** :
R₁ représente un groupe phényle ou un groupe naphtyle, éventuellement substitué par un ou plusieurs atomes ou groupes choisis indépendamment les uns des autres parmi les atomes d'halogène ou groupes (C₁-C₁₀)alkyle, (C₁-C₁₀)alcoxy; nitro, -S(O)₂(C₁-C₁₀-alkyle), halo(C₁-C₁₀)alkyle, cyano, (C₁-C₁₀)thioalkyle, ;NRaRb; NRcCORd, hydroxy(C₁-C₁₀)alkylène, NRcSO₂Re, CONRaRb, NRcC(O)ORe, SO₂NRaRb, NRaRb(C₁-C₁₀)alkylène ;
Ra et Rb représentent, indépendamment l'un de l'autre, un atome d'hydrogène ou un groupe (C₁C₁₀)alkyle ;
ou Ra et Rb forment ensemble, avec l'atome d'azote qui les porte, un groupe pyrrolidinyle, morpholinyle,
Rc et Rd représentent, indépendamment l'un de l'autre, un atome d'hydrogène ou un groupe (C₁-C₁₀)alkyle;
Re représente un groupe (C₁-C₁₀)alkyle ;
X représente 1 ou 2 substituants identiques ou différents l'un de l'autre choisis parmi halogène, (C₁-C₁₀)alkyle, (C₁-C₁₀)alcoxy, le groupe (C₁-C₁₀)alkyle pouvant être éventuellement substitué par un groupe hydroxyle ;
R₂ et X peuvent former ensemble avec les atomes de carbone qui les portent un cycle carboné de 5 ou 6 carbones ;
R₂ et R₃ représentent indépendamment l'un de l'autre un un atome d'hydrogène ou un groupe (C₁-C₁₀)alkyle ;
R₄ représente un atome d'hydrogène, un groupe (C₁-C₁₀)alkyle, ce groupe étant éventuellement substitué par un groupe Rf ;
Rf représente un groupe (C₁-C₁₀)alcoxy ;
la substitution du groupe 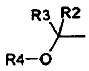 sur le noyau phényle est en position 2, 3 ou 4 ;
à l'état de base ou de sel d'addition à un acide.

8. Composés de formule (I) selon la revendication 1, **caractérisés en ce que**
R₁ représente un groupe phényle éventuellement substitué par un halogène, un groupe (C₁-C₁₀)alkyle ou (C₁-C₁₀)alcoxy;
X représente un ou plusieurs halogène, (C₁-C₁₀)alkyle, (C₁-C₁₀)alcoxy, hydroxy(C₁-C₁₀)alkyle;
R₂ et R₃ représentent, indépendamment l'un de l'autre, un atome d'hydrogène ou un groupe (C₁-C₁₀)alkyle ;
R₂ et X peuvent former ensemble avec les atomes de carbone qui les portent un cycle carboné de 6 atomes de carbone ;
R₄ représente un atome d'hydrogène,
à l'état de base ou de sel d'addition à un acide.

9. Composés :
• {3-[2-(4-Chlorophényl)imidazo[1,2-*a*]pyridin-6-yl]-2-fluorophényl}méthanol ;
• 1-{3-[2-(4-Chlorophényl)imidazo[1,2-*a*]pyridin-6-yl]-2-fluorophényl}éthanol ;
• {3-[2-(4-Chlorophényl)imidazo[1,2-*a*]pyridin-6-yl]-2,6-difluorophényl}méthanol ;
• 1-{3-[2-(4-Chlorophényl)imidazo[1,2-*a*]pyridin-6-yl]-2,6-difluorophényl}éthanol ;
• 1-{5-[2-(4-Chlorophényl)imidazo[1,2-*a*]pyridin-6-yl]-2-fluorophényl}éthanol
• {3-[2-(4-Chlorophényl)imidazo[1,2-*a*]pyridin-6-yl]-5-méthylphényl}méthanol ;
• {3-[2-(4-Chlorophényl)imidazo[1,2-*a*]pyridin-6-yl]-5-méthoxyphényl}méthanol ;
• 7-[2-(4-Chlorophényl)imidazo[1,2-*a*]pyridin-6-yl]-1,2,3,4-tétrahydronaphthalèn-1-ol ;
• 5-[2-(4-Chlorophényl)imidazo[1,2-*a*]pyridin-6-yl]-1,2,3,4-tétrahydronaphthalèn-1-ol ;
• {3-[2-(4-Chlorophényl)imidazo[1,2-*a*]pyridin-6-yl]-4-méthylphényl}méthanol ;
• {5-[2-(4-Chlorophényl)imidazo[1,2-*a*]pyridin-6-yl]-2-fluorophényl}méthanol ;
• {5-[2-(4-Chlorophényl)imidazo[1,2-*a*]pyridin-6-yl]-2-méthylphényl}méthanol ;
• {3-[2-(4-Chlorophényl)imidazo[1,2-*a*]pyridin-6-yl]-2-méthylphényl}méthanol ;
• [2-Fluoro-4-(2-p-tolylimidazo[1,2-*a*]pyridin-6-yl)phényl]méthanol ;
• [2-Fluoro-6-(2-p-tolylimidazo[1,2-*a*]pyridin-6-yl)phényl]méthanol ;
• {2-Fluoro-6-[2-(3-méthoxyphényl)imidazo[1,2-*a*]pyridin-6-yl]phényl}méthanol ;
• {2-fluoro-4-[2-(3-méthoxyphényl)imidazo[1,2-*a*]pyridin-6-yl]phényl}méthanol et son chlorhydrate ;
• [2-fluoro-4-(2-phénylimidazo[1,2-*a*]pyridin-6-yl)phényl]méthanol et son chlorhydrate ;
• {2-Fluoro-3-[2-(3-méthoxyphényl)imidazo[1,2-*a*]pyridin-6-yl]phényl}méthanol ;
• [2-fluoro-6-(2-phénylimidazo[1,2-*a*]pyridin-6-yl)phényl]méthanol et son chlorhydrate ;
• [2-Fluoro-3-(2-p-tolylimidazo[1,2-*a*]pyridin-6-yl)phényl]méthanol ;
• 1-{3-[2-(4-Chlorophényl)imidazo[1,2-*a*]pyridin-6-yl]-4-fluorophényl}éthanol ;
• {3-[2-(4-Chlorophényl)imidazo[1,2-*a*]pyridin-6-yl]-4-fluorophényl}méthanol ;
• {3-[2-(4-Chlorophényl)imidazo[1,2-*a*]pyridin-6-yl]-4-méthoxyphényl}méthanol ;
• 1-{3-[2-(4-Chlorophényl)imidazo[1,2-*a*]pyridin-6-yl]-4-méthoxyphényl}éthanol ;
• {3-[2-(4-Chlorophényl)imidazo[1,2-*a*]pyridin-6-yl]- 5-(hydroxyméthyl)phényl}méthanol ;
• [2-Fluoro-3-[2-(naphthyl-2-yl)imidazo[1,2-*a*]pyridin-6-yl]phényl]méthanol ;
• [2-Fluoro-3-(2-phénylimidazo[1,2-*a*]pyridin-6-yl)phényl]méthanol et son chlorhydrate ;
• (+)-1-{3-[2-(4-Chlorophényl)imidazo[1,2-*a*]pyridin-6-yl]-4-fluorophényl}éthanol ;
• (-)-1-{3-[2-(4-Chlorophényl)imidazo[1,2-*a*]pyridin-6-yl]-4-fluorophényl}éthanol ;
• {2-[2-(2,4-Difluorophényl)imidazo[1,2-*a*]pyridin-6-yl]-6-fluorophényl}méthanol ;
• {3-[2-(2,4-Difluorophényl)imidazo[1,2-*a*]pyridin-6-yl]-2,6-difluorophényl}méthanol ;
• 2-{4-Fluoro-3-[2-(3-méthoxyphényl)imidazo[1,2-*a*]pyridin-6-yl]phényl}propan-2-ol ;
• 1-{4-Fluoro-3-[2-(3-méthoxyphényl)imidazo[1,2-*a*]pyridin-6-yl]phényl}éthanol ;
• 2-{3-[2-(4-Chlorophényl)imidazo[1,2-*a*]pyridin-6-yl]-2,6-difluorophényl}propan-2-ol ;
• 1-[4-Fluoro-2-(2-naphthyl-2-ylimidazo[1,2-*a*]pyridin-6-yl)phényl]éthanol ;
• 6-(2,4-Difluoro-3-méthoxyméthylphényl)-2-p-tolylimidazo[1,2-*a*]pyridine ;
• {2-Fluoro-6-[2-(4-nitrophényl)imidazo[1,2-*a*]pyridin-6-yl]phényl}méthanol;
• {2,6-Difluoro-3-[2-(4-pyrrolidin-1-ylphényl)imidazo[1,2-*a*]pyridin-6-yl]phényl}méthanol ;
• {3-[2-(3,4-Difluorophényl)imidazo[1,2-*a*]pyridin-6-yl]-2,6-difluorophényl}méthanol ;
• {2,6-Difluoro-3-[2-(2-fluorophényl)imidazo[1,2-*a*]pyridin-6-yl]phényl}méthanol ;
• {3-[2-(3-Bromophényl)imidazo[1,2-*a*]pyridin-6-yl]-2,6-difluorophényl}méthanol ;
• 2,6-Difluoro-3-[2-(4-méthylsulfonylphényl)imidazo[1,2-*a*]pyridin-6-yl]phényl}méthanol ;
• 2-[2-Méthyl-3-(2-phénylimidazo[1,2-*a*]pyridin-6-yl)phényl]propan-2-ol ;
• 7-[2-(2,4-Difluorophényl)imidazo[1,2-*a*]pyridin-6-yl]indan-1-ol ;
• 2-(4-Chlorophényl)-6-[2,4-difluoro-3-[(2-méthoxy-éthyl)oxyméthyl]phényl]imidazo[1,2-*a*]pyridine ;
• 7-[2-(2,4-Difluorophényl)imidazo[1,2-*a*]pyridin-6-yl]-1-méthylindan-1-ol ;
• 2-{3-[2-(4-Chlorophényl)imidazo[1,2-*a*]pyridin-6-yl]-2-méthoxyphényl}propan-2-ol ;
• 1-{3-[2-(4-Chlorophényl)imidazo[1,2-*a*]pyridin-6-yl]-2-méthoxyphényl}éthanol ;
• {2-Fluoro-6-[2-(4-trifluorométhylphényl)imidazo[1,2-*a*]pyridin-6-yl]phényl}-méthanol ;
• {2-Fluoro-6-[2-(4-méthoxyphényl)imidazo[1,2-*a*]pyridin-6-yl]phényl}méthanol ;
• {2-Fluoro-6-[2-(4-pyrrolidin-1-ylphényl)imidazo[1,2-*a*]pyridin-6-yl]phényl}méthanol;
• {2-Fluoro-6-[2-(4-fluorophényl)imidazo[1,2-*a*]pyridin-6-yl]phényl}méthanol ;
• {2-[2-(3,4-Difluorophényl)imidazo[1,2-*a*]pyridin-6-yl]-6-fluorophényl}méthanol ;
• 3-[6-(3-Fluoro-2-hydroxyméthylphényl)imidazo[1,2-*a*]pyridin-2-yl]benzonitrile ;
• {2-Fluoro-6-[2-(2-fluorophényl)imidazo[1,2-*a*]pyridin-6-yl]phényl}méthanol ;
• {2-[2-(3-Bromophényl)imidazo[1,2-*a*]pyridin-6-yl]-6-fluorophényl}méthanol ;
• {2-[2-(4-Chloro-3-méthylphényl)imidazo[1,2-*a*]pyridin-6-yl]-6-fluorophényl}méthanol ;
• {2-[2-(3-Chloro-4-méthylphényl)imidazo[1,2-*a*]pyridin-6-yl]-6-fluorophényl}méthanol ;
• {2-Fluoro-6-[2-(4-méthylsulfonylphényl)imidazo[1,2-*a*]pyridin-6-yl]phényl}méthanol ;
• {2,6-Difluoro-3-[2-(4-méthoxyphényl)imidazo[1,2-*a*]pyridin-6-yl]phényl}méthanol ;
• {2,6-Difluoro-3-[2-(4-fluorophényl)imidazo[1,2-*a*]pyridin-6-yl]phényl}méthanol ;
• 3-[6-(2,4-Difluoro-3-hydroxyméthylphényl)imidazo[1,2-*a*]pyridin-2-yl]benzonitrile ;
• {2,6-Difluoro-3-[2-(4-chloro-3-méthylphényl)imidazo[1,2-*a*]pyridin-6-yl]phényl}méthanol ;
• {2,6-Difluoro-3-[2-(3-chloro-4-méthylphényl)imidazo[1,2-*a*]pyridin-6-yl]phényl}méthanol ;
• {2,6-Difluoro-3-[2-(4-méthylthiophényl)imidazo[1,2-*a*]pyridin-6-yl]phényl}méthanol ;
• 2-[2-Chloro-3-[2-(4-chlorophényl)imidazo[1,2-*a*]pyridin-6-yl)phényl]propan-2-ol ;
• 1-[2-Chloro-3-[2-(4-Chlorophényl)imidazo[1,2-*a*]pyridin-6-yl)phényl]éthanol ;
• {2-[2-(4-Chlorophényl)imidazo[1,2-*a*]pyridin-6-yl]-6-fluorophényl}méthanol ;
• 2-(4-Chlorophényl)-6-[3-fluoro-2-[(2-méthoxy-éthyl)oxyméthyl]phényl]imidazo[1,2-*a*]pyridine ;
• N-{3-[6-(3-Fluoro-2-hydroxyméthylphényl)imidazo[1,2-*a*]pyridin-2-yl]phényl}acétamide ;;
• {4-[6-(3-Fluoro-2-hydroxyméthylphényl)imidazo[1,2-*a*]pyridin-2-yl]phényl} méthanol
• {3-[6-(3-Fluoro-2-hydroxyméthylphényl)imidazo[1,2-*a*]pyridin-2-yl]phényl}méthanol ;
• *N*-{4-[6-(3-Fluoro-2-hydroxyméthylphényl)imidazo[1,2-*a*]pyridin-2-yl]phényl}méthanesulfonamide ;
• 4-[6-(3-Fluoro-2-hydroxyméthylphényl)imidazo[1,2-*a*]pyridin-2-yl]-*N,N-*diméthylbenzamide ;
• {4-[6-(3-Fluoro-2-hydroxyméthylphényl)imidazo[1,2-*a*]pyridin-2-yl]phényl}carbamate de méthyle ;
• 4-[6-(3-Fluoro-2-hydroxyméthylphényl)imidazo[1,2-*a*]pyridin-2-yl]-*N* méthylbenzamide ;
• N-{4-[6-(3-Fluoro-2-hydroxyméthylphényl)imidazo[1,2-*a*]pyridin-2-yl]phényl}acétamide ;
• {2-Fluoro-6-[2-(4-morpholin-4-ylphényl)imidazo[1,2-*a*]pyridin-6-yl]phényl}méthanol ;
• 3-[6-(3-Fluoro-2-hydroxyméthylphényl)imidazo[1,2-*a*]pyridin-2-yl]-*N,N* diméthylbenzènesulfonamide ;
• (2-Fluoro-6-{2-[4-(1-pyrrolidin-1-yléthyl)phényl]imidazo[1,2-*a*]pyridin-6-yl}phényl)méthanol ;
• 2-Fluoro-4-[6-(3-fluoro-2-hydroxyméthylphényl)imidazo[1,2-*a*]pyridin-2-yl]-*N,N* diméthylbenzamide ;
• N-{3-[6-(2,4-Difluoro-3-hydroxyméthylphényl)imidazo[1,2-*a*]pyridin-2-yl]phényl}acétamide ;
• {4-[6-(2,4-Difluoro-3-hydroxyméthylphényl)imidazo[1,2-*a*]pyridin-2-yl]phényl}méthanol ;
• {3-[6-(2,4-Difluoro-3-hydroxyméthylphényl)imidazo[1,2-*a*]pyridin-2-yl]phényl}méthanol ;
• 4-[6-(2,4-Difluoro-3-hydroxyméthylphényl)imidazo[1,2-*a*]pyridin-2-yl]-*N,N* diméthylbenzamide ;
• {4-[6-(2,4-Difluoro-3-hydroxyméthylphényl)imidazo[1,2-*a*]pyridin-2-yl]phényl}carbamate de méthyle ;
• 4-[6-(2,4-Difluoro-3-hydroxyméthylphényl)imidazo[1,2-*a*]pyridin-2-yl]-*N* méthylbenzamide ;
• N-{4-[6-(2,4-Difluoro-3-hydroxyméthylphényl)imidazo[1,2-*a*]pyridin-2-yl]phényl}acétamide ;
• {2,6-Difluoro-3-[2-(4-morpholin-4-yl-phényl)imidazo[1,2-*a*]pyridin-6-yl]-phényl}méthanol ;
• *N*-{3-[6-(2,4-Difluoro-3-hydroxyméthylphényl)imidazo[1,2-*a*]pyridin-2-yl]phényl}isobutyramide ;
• 3-[6-(2,4-Difluoro-3-hydroxyméthylphényl)imidazo[1,2-*a*]pyridin-2-yl]-N,N-diméthylbenzènesulfonamide ;
• (2,6-Difluoro-3-{2-[4-(1-pyrrolidin-1-yl-éthyl)phényl]imidazo[1,2-*a*]pyridin-6-yl}phényl)méthanol ;
• 4-[6-(2,4-Difluoro-3-hydroxyméthylphényl)imidazo[1,2-*a*]pyridin-2-yl]-2-fluoro-*N,N*-diméthylbenzamide.

10. Médicament, **caractérisé en ce qu'**il comprend un composé de formule (I) selon l'une quelconque des revendications 1 à 9, ou un sel d'addition de ce composé à un acide pharmaceutiquement acceptable.

11. Composition pharmaceutique, **caractérisée en ce qu'**elle comprend un composé de formule (I) selon l'une quelconque des revendications 1 à 9, ou un sel pharmaceutiquement acceptable, de ce composé, ainsi qu'au moins un excipient pharmaceutiquement acceptable.

12. Utilisation d'un composé de formule (I) selon l'une quelconque des revendications 1 à 9 pour la préparation d'un médicament destiné au traitement et à la prévention des maladies neurodégénératives.

13. Utilisation d'un composé de formule (I) selon l'une quelconque des revendications 1 à 9 pour la préparation d'un médicament destiné au traitement et à la prévention des traumatismes cérébraux et de l'épilepsie.

14. Utilisation d'un composé de formule (I) selon l'une quelconque des revendications 1 à 9 pour la préparation d'un médicament destiné au traitement et à la prévention des maladies psychiatriques.

15. Utilisation d'un composé de formule (I) selon l'une quelconque des revendications 1 à 9 pour la préparation d'un médicament destiné au traitement et à la prévention des maladies inflammatoires.

16. Utilisation d'un composé de formule (I) selon l'une quelconque des revendications 1 à 9 pour la préparation d'un médicament destiné au traitement et à la prévention de l'ostéoporose et les cancers.

17. Utilisation d'un composé de formule (I) selon l'une quelconque des revendications 1 à 9 pour la préparation d'un médicament destiné au traitement et à la prévention de la maladie de Parkinson, d'Alzheimer, des tauopathies, de la sclérose en plaque.

18. Utilisation d'un composé de formule (I) selon l'une quelconque des revendications 1 à 9 pour la préparation d'un médicament destiné au traitement et à la prévention de la schizophrénie, la dépression, la dépendance à une substance, les troubles du déficit de l'attention et de l'hyperactivité.

19. Procédé de synthèse de des composés de formule (I) selon la revendication 1, dans laquelle on fait réagir un composé de formule (II) 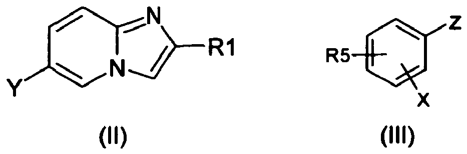 dans laquelle R1 est tel que défini dans la revendication 1 et Y représente un atome d'halogène ou un dérivé de bore avec un dérivé de formule générale (III) dans laquelle X est défini selon la revendication 1 et Z représente un dérivé de bore ou d'étain si Y représente un atome d'halogène, ou bien un atome d'halogène si Y représente un dérivé de bore, et R5 représente le groupe

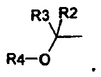

20. Procédé de synthèse de des composés de formule (I) selon la revendication 1, dans laquelle on fait réagir un composé de formule générale (VI), 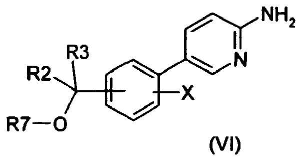 dans laquelle R2, R3 et X sont définis selon la revendication 1, et R7 représente R4 ou un groupement protecteur de fonction hydroxyle PG, et une bromocétone de formule générale (VII), 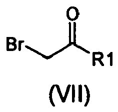 dans laquelle R1 est défini delon la revendication 1.

21. Procédé de synthèse de des composés de formule (I) selon la revendication 1, dans laquelle on fait réagir un composé de formule générale (II) 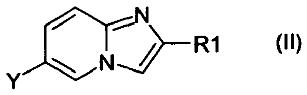 dans laquelle R1 est défini selon la revendication 1 et Y représente un atome d'halogène ou un dérivé de bore avec un dérivé de formule générale (III) 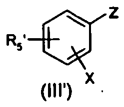 dans laquelle X est défini selon la revendication 1 et Z représente un dérivé de bore ou d'étain lorsque Y représente un atome d'halogène ou Z représente un atome d'halogène lorsque Y représente un dérivé de bore, et R5' représente, soit un dérivé carbonylé R₂CO dans lequel R2 est défini selon la revendication 1, soit un carboxylate d'alkyle, pour obtenir un composé de formule générale (IV), le composé de formule générale (IV) réagit ensuite avec un dérivé organométtalique pour obtenir un composé de formule générale (I).

22. Procédé de synthèse de des composés de formule (I) dans laquelle on fait réagir un composé de formule générale (VI), 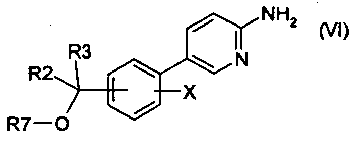 dans laquelle R2, R3 et X sont définis selon la revendication 1, et R7 représente R4 ou un groupement protecteur (PG) de fonction hydroxyle, une bromocétone de formule générale (VII), 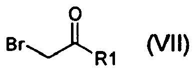 dans laquelle R1 est défini selon la revendication 1.

23. Procédé de synthèse de des composés de formule (I) selon la revendication 1, dans laquelle on fait réagir un composé de formule générale (VI), 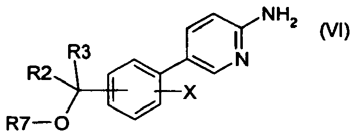 dans laquelle R2, R3 et X sont définis selon la revendication 1, et R7 représente R4 ou un groupement protecteur (PG) de fonction hydroxyle, une bromocétone de formule générale (VII), 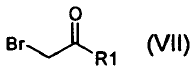 dans laquelle R1 est défini selon la revendication 1, pour obtenir un composé de formule générale (VIII) 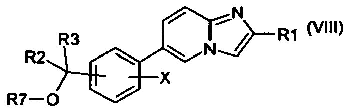 dans laquelle R7 représente un groupe protecteur de fonction hydroxyle, le composé de formula générale (VIII) est ensuite déprotégé pour obtenir un composé de formule gé,érale (I).

24. Procédé de synthèse de des composés de formule (I) selona la revendication 1, dans laquelle on fait réagir un composé de formule générale (XI) 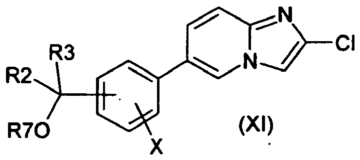 dans laquelle X, R2 et R3 sont définis selon la revendication 1 et R7 représente le groupe R4, avec un dérivé de formule générale (XII)
R1-Z (XII)
dans laquelle R1 est défini selon la revendication 1 et Z représente un dérivé de bore ou d'étain, pour obtenir les composés de formule générale (1).

25. Procédé de synthèse de des composés de formule (I) selon la revendication 1, dans laquelle on fait réagir un composé de formule générale (XI) 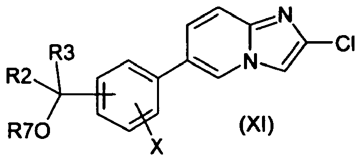 Dans laquelle X, R2 et R3 sont définis selon la revendication 1 et R7 représente le groupement protecteur de fonction hydroxyle, avec un dérivé de formule générale (XII)
R1-Z (XII)
dans laquelle R1 est défini selon la revendication 1 et Z représente un dérivé de bore ou d'étain, pour obtenir les composés de formule générale (VIII), 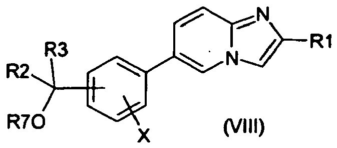 R1, R2, R3 et X sont définis selon la revendication 1 et R4 représente un atome d'hydrogène, le composé de formule générale (VIII) est ensuite déprotégé.

26. Composés intermédiaires

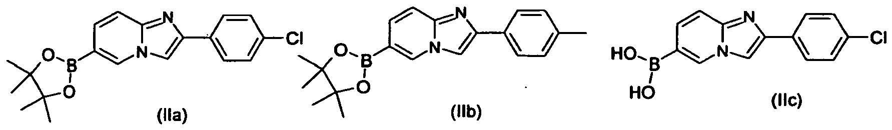

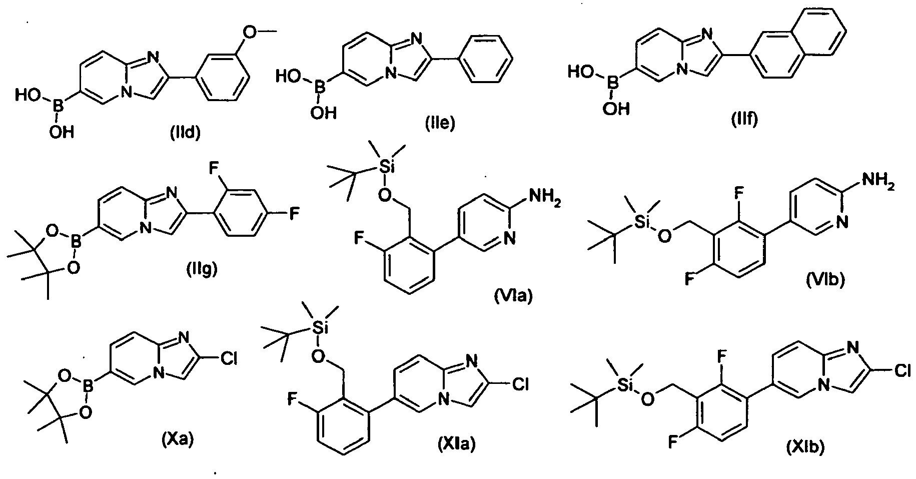

27. Procédé de synthèse selon la revendication 19, 20, 21, 22, 23, 24 ou 25, où les composés de formules générales (II), (VI), (X), (XI) sont les composés de formules générales (IIa), (IIb), (IIc), (IId), (IIe), (IIF), (IIg), (VIa), (VIb), (Xa), (XIa) et(XIb).

## Claims

1. Compounds of formula (I): 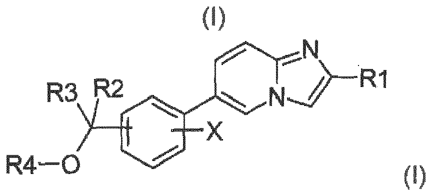 in which:
R₁ represents:
a phenyl group or a naphthyl group, it being possible for these two groups optionally to be substituted by one or more atoms or groups chosen, independently of one another, from the following atoms or groups: halogen, (C₁-C₁₀)alkyl, halo(C₁-C₁₀)alkyl, (C₁-C₁₀)alkoxy, halo(C₁-C₁₀)alkoxy, (C₁-C₁₀)thioalkyl,-S(O)(C₁-C₁₀)alkyl, -S(O)₂(C₁-C₁₀)alkyl, hydroxyl, cyano, nitro, hydroxy(C₁-C₁₀)alkylene, NRaRb(C₁-C₁₀)alkylene, (C₁-C₁₀)alkoxy(C₁-C₁₀)alkyleneoxy, NRaRb, CONRaRb, NRcC(O)ORe, NRCSO₂Re, aryl(C₁-C₁₀)alkylene, monocyclic aryl or monocyclic heteroaryl, the monocyclic aryl or monocyclic heteroaryl optionally being substituted by one or more substituents chosen from a halogen or a (C₁₋C₁₀)alkyl, halo(C₁-C₁₀)alkyl, (C₁-C₁₀)alkoxy, halo(C₁-C₁₀)alkoxy, NRaRb, hydroxyl, oxo, nitro, cyano or OCO(C₁-C₁₀)alkyl group;
X represents from 1 to 4 substituents which are identical to or different from one another and which are chosen from halogen, (C₁-C₁₀)alkyl, (C₁-C₁₀)alkoxy, NRaRb, cyano or nitro, it being possible for the (C₁-C₁₀)alkyl to be optionally substituted by one or more groups chosen from a halogen, (C₁-C₁₀)alkoxy, halo(C₁-C₁₀)alkoxy, NRaRb or hydroxyl;
R₂ and R₃ represent, independently of one another,
a hydrogen atom,
a (C₁-C₁₀)alkyl group, this group being optionally substituted by an Rf group;
an aryl group, optionally substituted by one or more substituents chosen from a halogen or a (C₁-C₁₀)alkyl, halo(C₁-C₁₀)alkyl, (C₁-C₁₀)alkoxy, halo(C₁-C₁₀)alkoxy, NRaRb, hydroxyl, nitro or cyano group;
R₂ and X can together form, with the carbon atoms which carry then, a carbon ring of 5 to 7 carbon atoms;
R₄ represents :
a hydrogen atom,
a (C₁-C₁₀)alkyl group, this group being optionally substituted by an Rf group;
an aryl group, optionally substituted by one or more substituents chosen from a halogen or a (C₁-C₁₀)alkyl, halo(C₁-C₁₀)alkyl, (C₁-C₁₀)alkoxy, halo(C₁-C₁₀)alkoxy, NRaRb, hydroxyl, nitro, cyano, (C₁-C₁₀)alkyl(CO)-, CONRaRb, NRCCORd, OC(O)NRaRb, OCO(C₁-C₁₀)alkyl, NRcC(O)ORe or aryl group, the aryl being optionally substituted by one or more substituents chosen from a halogen or a (C₁-C₁₀)alkyl, halo(C₁-C₁₀)alkyl, (C₁-C₁₀)alkoxy, halo(C₁-C₁₀)alkoxy, NRaRb, hydroxyl, nitro or cyano group;
Ra and Rb represent, independently of one another,
a hydrogen atom or a (C₁-C₁₀)alkyl, aryl(C₁-C₁₀)alkylene or aryl group;
or Ra and Rb together form, with the nitrogen atom which carries them, an azetidine, pyrrolidine, piperidine, azepine, morpholine, thiomorpholine, piperazine or homopiperazine group, this group being optionally substituted by a (C₁-C₁₀)alkyl, aryl or aryl(C₁-C₁₀)alkylene group;
Rc and Rd represent, independently of one another,
a hydrogen atom or a (C₁-C₁₀)alkyl, aryl(C₁-C₁₀)alkylene or aryl group;
or Rc and Rd together form a (C₂-C₅)alkylene group;
Re represents
a (C₁-C₁₀)alkyl, aryl(C₁-C₁₀)alkylene or aryl group;
or Rc and Re together form a (C₂-C₅)alkylene group;
Rf represents
a halogen atom or a halo(C₁-C₁₀)alkoxy, hydroxyl, cyano, NRaRb, C(O)NRaRb, NRcCORd, OC(O)NRaRb, OCO(C₁-C₁₀)alkyl, NRcCOORe, SO₂NRaRb, NRcSO₂Re, aryl(C₁-C₁₀)alkylene or aryl group, the aryl being optionally substituted by one or more substituents chosen from a halogen or a (C₁-C₁₀)alkyl, halo(C₁-C₁₀)alkyl, (C₁-C₁₀)alkoxy, halo(C₁-C₁₀)alkoxy, NRaRb, hydroxyl, nitro, cyano or OCO(C₁-C₁₀)alkyl group;
in the form of the base or of an addition salt with an acid.

2. Compounds of formula (I) according to Claim 1, **characterized in that**:
R₁ represents a phenyl group or a naphthyl group optionally substituted by one or more atoms or groups chosen, independently of one another, from halogen atoms or (C₁-C₁₀)alkyl, (C₁-C₁₀)alkoxy, nitro, -S(O)₂(C₁-C₁₀)alkyl, halo(C₁-C₁₀)alkyl, cyano, (C₁-C₁₀)thioalkyl, NRaRb, NRcCORd, hydroxy(C₁-C₁₀)alkylene, NRcSO₂Re, CONRaRb, NRcC(O)ORe, SO₂NRaRb or NRaRb(C₁-C₁₀)alkylene groups;
Ra and R_{b} represent, independently of one another, a hydrogen atom or a (C₁-C₁₀)alkyl group; a
or Ra and Rb together form, with the nitrogen atom which carries them, a pyrrolidinyl or morpholinyl group;
Rc and Rd represent, independently of one another, a hydrogen atom or a (C₁-C₁₀)alkyl group;
Re represents a (C₁₋C₁₀)alkyl group.

3. Compounds of formula (I) according to Claim 1 or 2, **characterized in that**:
X represents 1 or 2 substituents, identical to or different from one another, chosen from halogen, (C₁-C₁₀)alkyl or (C₁-C₁₀)alkoxy, it being possible for (C₁-C₁₀)alkyl to be optionally substituted by a hydroxyl group;
R₂ and X can together form, with the carbon atoms which carry them, a carbon ring of 5 or 6 carbons.

4. Compounds of formula (I) according to any one of the preceding claims, **characterized in that**:
R₂ and R₃ represent, independently of one another, a hydrogen atom or a (C₁-C₁₀)alkyl group.

5. Compounds of formula (I) according to any one of the preceding claims, **characterized in that**:
R₄ represents a hydrogen atom or a (C₁-C₁₀)alkyl group, this group optionally being substituted by an Rf group;
Rf represents a (C₁-C₁₀)alkoxy group.

6. Compounds of formula (I) according to any one of the preceding claims, **characterized in that**: the substitution of the 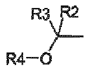 group on the phenyl ring is in the 2, 3 or 4 position.

7. Compounds of formula (I) according to any one of the preceding claims, **characterized in that**:
R₁ represents a phenyl group or a naphthyl group, optionally substituted by one or more atoms or groups chosen, independently of one another, from halogen atoms or (C₁-C₁₀)alkyl, (C₁-C₁₀)alkoxy, nitro, -S(O)₂(C₁-C₁₀)alkyl, halo(C₁-C₁₀alkyl, cyano, (C₁-C₁₀)thioalkyl, NRaRb, NRcCORd, hydroxy(C₁-C₁₀)alkylene, NRcSO₂Re, CONRaRb, NRcC(O)ORe, SO₂NRaRb or NRaRb(C₁-C₁₀)alkylene groups;
Ra and Rb represent, independently of one another, a hydrogen atom or a (C₁-C₁₀)alkyl group;
or Ra and Rb together form, with the nitrogen atom which carries them, a pyrrolidinyl or morpholinyl group;
Rc and Rd represent, independently of one another, a hydrogen atom or a (C₁-C₁₀)alkyl group;
Re represents a (C₁-C₁₀)alkyl group;
X represents 1 or 2 substituents, identical to or different from one another, chosen from halogen, (C₁-C₁₀)alkyl, (C₁-C₁₀)alkoxy, it being possible for the (C₁-C₁₀)alkyl group to be optionally substituted by a hydroxyl group;
R₂ and X can together form, with the carbon atoms which carry them, a carbon ring of 5 or 6 carbons;
R₂ and R₃ represent, independently of one another, a hydrogen atom or a (C₁-C₁₀)alkyl group;
R₄ represents a hydrogen atom or a (C₁-C₁₀)alkyl group, this group being optionally substituted by an Rf group;
Rf represents a (C₁-C₁₀)alkoxy group;
the substitution of the 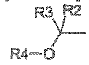 group on the phenyl ring is in the 2, 3 or 4 position;
in the form of the base or of an addition salt with an acid.

8. Compounds of formula (I) according to Claim 1, **characterized in that**
R₁ represents a phenyl group optionally substituted by a halogen or a (C₁-C₁₀)alkyl or (C₁-C₁₀)alkoxy group;
X represents one or more halogen, (C₁-C₁₀)alkyl, (C₁-C₁₀)alkoxy or hydroxy(C₁-C₁₀)alkyl;
R₂ and R₃ represent, independently of one another, a hydrogen atom or a (C₁-C₁₀)alkyl group;
R₂ and X can together form, with the carbon atoms which carry them, a carbon ring of 6 carbon atoms;
R₄ represents a hydrogen atom,
in the form of the base or of an addition salt with an acid.

9. Compounds:
• {3-[2-(4-Chlorophenyl)imidazo[1,2-*a*]pyridin-6-yl]-2-fluorophenyl}methanol;
• 1-{3-[2-(4-Chlorophenyl)imidazo[1,2-*a*]pyridin-6-yl]-2-fluorophenyl}ethanol;
• {3-[2-(4-Chlorophenyl)imidazo[1,2-*a*]pyridin-6-yl]-2,6-difluorophenyl}methanol;
• 1-{3-[2-(4-Chlorophenyl)imidazo[1,2-*a*]pyridin-6-yl]-2,6-difluorophenyl}ethanol;
• 1-{5-[2-(4-Chlorophenyl)imidazo[1,2-*a*]pyridin-6-yl]-2-fluorophenyl}ethanol;
• {3-[2-(4-Chlorophenyl)imidazo[1,2-*a*]pyridin-6-yl]-5-methylphenyl}methanol;
• {3-[2-(4-Chlorophenyl)imidazo[1,2-*a*]pyridin-6-yl]-5-methoxyphenyl}methanol;
• 7-[2-(4-Chlorophenyl)imidazo[1,2-*a*]pyridin-6-yl]-1,2,3,4-tetrahydronaphth-1-ol;
• 5-[2-(4-Chlorophenyl)imidazo[1,2-*a*]pyridin-6-yl]-1,2,3,4-tetrahydronaphth-1-ol;
• {3-[2-(4-Chlorophenyl)imidazo[1,2-*a*]pyridin-6-yl]-4-methylphenyl}methanol;
• {5-[2-(4-Chlorophenyl)imidazo[1,2-*a*]pyridin-6-yl]-2-fluorophenyl}methanol;
• {5-[2-(4-Chlorophenyl)imidazo[1,2-*a*]pyridin-6-yl]-2-methylphenyl}methanol;
• {3-[2-(4-Chlorophenyl)imidazo[1,2-*a*]pyridin-6-yl]-2-methylphenyl}methanol;
• [2-Fluoro-4-(2-(p-tolyl)imidazo[1,2-*a*]pyridin-6-yl)phenyl]methanol;
• [2-Fluoro-6-(2-(p-tolyl)imidazo[1,2-*a*]pyridin-6-yl)phenyl]methanol ;
• {2-Fluoro-6-[2-(3-methoxyphenyl)imidazo[1,2-*a*]pyridin-6-yl]phenyl}methanol;
• {2-Fluoro-4-[2-(3-methoxyphenyl)imidazo[1,2-*a*]pyridin-6-yl]phenyl}methanol and its hydrochloride;
• [2-Fluoro-4-(2-phenylimidazo[1,2-*a*]pyridin-6-yl)phenyl]methanol and its hydrochloride;
• {2-Fluoro-3-[2-(3-methoxyphenyl)imidazo[1,2-*a*]pyridin-6-yl]phenyl}methanol;
• [2-Fluoro-6-(2-phenylimidazo[1,2-*a*]pyridin-6-yl)phenyl]methanol and its hydrochloride;
• [2-Fluoro-3-(2-(p-tolyl)imidazo[1,2-*a*]pyridin-6-yl)phenyl]methanol;
• 1-{3-[2-(4-Chlorophenyl)imidazo[1,2-*a*]pyridin-6-yl]-4-fluorophenyl}ethanol;
• {3-[2-(4-Chlorophenyl)imidazo[1,2-*a*]pyridin-6-yl]-4-fluorophenyl}methanol;
• {3-[2-(4-Chlorophenyl)imidazo[1,2-*a*]pyridin-6-yl]-4-methoxyphenyl}methanol;
• 1-{3-[2-(4-Chlorophenyl)imidazo[1,2-*a*]pyridin-6-yl]-4-methoxyphenyl}ethanol;
• {3-[2-(4-Chlorophenyl)imidazo[1,2-*a*]pyridin-6-yl]-5-(hydroxymethyl)phenyl}methanol;
• [2-Fluoro-3-[2-(naphth-2-yl)imidazo[1,2-*a*]pyridin-6-yl]phenyl]methanol;
• [2-Fluoro-3-(2-phenylimidazo[1,2-*a*]pyridin-6-yl)phenyl]methanol and its hydrochloride;
• (+)-1-{3-[2-(4-Chlorophenyl)imidazo[1,2-*a*]pyridin-6-yl]-4-fluorophenyl}ethanol;
• (-)-1-{3-[2-(4-Chlorophenyl)imidazo[1,2-*a*]pyridin-6-yl]-4-fluorophenyl}ethanol;
• {2-[2-(2,4-Difluorophenyl)imidazo[1,2-*a*]pyridin-6-yl]-6-fluorophenyl}methanol;
• {3-[2-(2,4-Difluorophenyl)imidazo[1,2-*a*]pyridin-6-yl]-2,6-difluorophenyl}methanol;
• 2-{4-Fluoro-3-[2-(3-methoxyphenyl)imidazo[1,2-*a*]pyridin-6-yl]phenyl}propan-2-ol;
• 1-{4-Fluoro-3-[2-(3-methoxyphenyl)imidazo[1,2-*a*]pyridin-6-yl]phenyl}ethanol;
• 2-{3-[2-(4-Chlorophenyl)imidazo[1,2-*a*]pyridin-6-yl]-2,6-difluorophenyl}propan-2-ol;
• 1-[4-Fluoro-2-(2-(naphth-2-yl)imidazo[1,2-*a*]pyridin-6-yl)phenyl]ethanol;
• 6-(2,4-Difluoro-3-methoxymethylphenyl)-2-(p-tolyl)imidazo[1,2-*a*]pyridine;
• {2-Fluoro-6-[2-(4-nitrophenyl)imidazo[1,2-*a*]pyridin-6-yl]phenyl}methanol;
• {2,6-Difluoro-3-[2-(4-(pyrrolidin-1-yl)phenyl)imidazo[1,2-*a*]pyridin-6-yl]phenyl}methanol;
• {3-[2-(3,4-Difluorophenyl)imidazo[1,2-*a*]pyridin-6-yl]-2,6-difluorophenyl}methanol;
• {2,6-Difluoro-3-[2-(2-fluorophenyl)imidazo[1,2-*a*]pyridin-6-yl]phenyl}methanol;
• {3-[2-(3-Bromophenyl)imidazo[1,2-*a*]pyridin-6-yl]-2,6-difluorophenyl}methanol;
• {2,6-Difluoro-3-[2-(4-methylsulphonylphenyl)imidazo[1,2-*a*]pyridin-6-yl]phenyl}methanol;
• 2-[2-Methyl-3-(2-phenylimidazo[1,2-*a*]pyridin-6-yl)phenyl]propan-2-ol;
• 7-[2-(2,4-Difluorophenyl)imidazo[1,2-*a*]pyridin-6-yl]indan-1-ol;
• 2-(4-Chlorophenyl)-6-[2,4-difluoro-3-[(2-methoxyethyl)oxymethyl]phenyl]imidazo[1,2-*a*]pyridine;
• 7-[2-(2,4-Difluorophenyl)imidazo[1,2-*a*]pyridin-6-yl]-1-methylindan-1-ol;
• 2-{3-[2-(4-Chlorophenyl)imidazo[1,2-*a*]pyridin-6-yl]-2-methoxyphenyl}propan-2-ol;
• 1-{3-[2-(4-Chlorophenyl)imidazo[1,2-*a*]pyridin-6-yl]-2-methoxyphenyl}ethanol ;
• {2-Fluoro-6-[2-(4-trifluoromethylphenyl)jmidazo[1,2-*a*]pyridin-6-yl]phenyl}methanol;
• {2-Fluoro-6-[2-(4-methoxyphenyl)imidazo[1,2-*a*]pyridin-6-yl]phenyl}methanol;
• {2-Fluoro-6-[2-(4-(pyrrolidin-1-yl)phenyl)imidazo[1,2-*a*]pyridin-6-yl]phenyl}methanol;
• {2-Fluoro-6-[2-(4-fluorophenyl)imidazo[1,2-*a*]pyridin-6-yl]phenyl}methanol;
• {2-[2-(3,4-Difluorophenyl)imidazo[1,2-*a*]pyridin-6-yl]-6-fluorophenyl}methanol;
• 3-[6-(3-Fluoro-2-hydroxymethylphenyl)imidazo[1,2-*a*]pyridin-2-yl]benzonitrile;
• {2-Fluoro-6-[2-(2-fluorophenyl)imidazo[1,2-*a*]pyridin-6-yl]phenyl}methanol;
• {2-[2-(3-Bromophenyl)imidazo[1,2-*a*]pyridin-6-y]-6-fluorophenyl}methanol;
• {2-[2-(4-Chloro-3-methylphenyl)imidazo[1,2-*a*]pyridin-6-yl]-6-fluorophenyl}methanol;
• {2-[2-(3-Chloro-4-methylphenyl)imidazo[1,2-*a*]pyridin-6-yl]-6-fluorophenyl}methanol;
• {2-Fluoro-6-[2-(4-methylsulphonylphenyl)imidazo[1,2-*a*]pyridin-6-yl]phenyl}methanol;
• {2,6-Difluoro-3-[2-(4-methoxyphenyl)imidazo[1,2-*a*]pyridin-6-yl]phenyl}methanol;
• {2,6-Difluoro-3-[2-(4-fluorophenyl)imidazo[1,2-*a*]pyridin-6-yl]phenyl}methanol;
• 3-[6-(2,4-Difluoro-3-hydroxymethylphenyl)imidazo[1,2-*a*]pyridin-2-yl]benzonitrile;
• {2,6-Difluoro-3-[2-(4-chloro-3-methylphenyl)imidazo[1,2-*a*]pyridin-6-yl]phenyl}methanol;
• {2,6-Difluoro-3-[2-(3-chloro-4-methylphenyl)imidazo[1,2-*a*]pyridin-6-yl]phenyl}methanol;
• {2,6-Difluoro-3-[2-(4-methylthiophenyl)imidazo[1,2-*a*]pyridin-6-yl]phenyl}methanol;
• 2-[2-Chloro-3-[2-(4--chlorophenyl)imidazo[1,2-*a*]pyridin-6-yl]phenyl]propan-2-ol;
• 1-[2-Chloro-3-[2-(4-chlorophenyl)imidazo[1,2-*a*]pyridin-6-yl]phenyl]ethanol;
• {2-[2-(4-Chlorophenyl)imidazo[1,2-*a*]pyridin-6-yl]-6-fluorophenyl}methanol;
• 2-(4-Chlorophenyl)-6-[3-fluoro-2-[(2-methoxyethyl)oxymethyl]phenyl]imidazo[1,2-*a*]pyridine
• *N*-{3-[6-(3-Fluoro-2-hydroxymethylphenyl)imidazo[1,2-*a*]pyridin-2-yl]phenyl}acetamide;
• {4-[6-(3-Fluoro-2-hydroxymethylphenyl)imidazo[1,2-*a*]pyridin-2-yl]phenyl}methanol
• {3-[6-(3-Fluoro-2-hydroxymethylphenyl)imidazo[1,2-*a*]pyridin-2-yl]phenyl}methanol;
• *N*-{4-[6-(3-Fluoro-2-hydroxymethylphenyl)imidazo[1,2-*a*]pyridin-2-yl]phenyl}methanesulphonamide;
• 4-[6-(3-Fluoro-2-hydroxymethylphenyl)imidazo[1,2-*a*]pyridin-2-yl]-*N,N-*dimethylbenzamide;
• Methyl {4-[6-(3-fluoro-2-hydroxymethylphenyl)imidazo[1,2-*a*]pyridin-2-yl]phenyl}carbamate;
• 4-[6-(3-Fluoro-2-hydroxymethylphenyl)imidazo[1,2-*a*]pyridin-2-yl]-*N-*methylbenzamide;
• N-{4-[6-(3-Fluoro-2-hydroxymethylphenyl)imidazo[1,2-*a*]pyridin-2-yl]phenyl}acetamide;
• {2-Fluoro-6-[2-(4-(morpholin-4-yl)phenyl)imidazo[1,2-*a*]pyridin-6-yl]phenyl}methanol;
• 3-[6-(3-Fluoro-2-hydroxymethylphenyl)imidazo[1,2-*a*]pyridin-2-yl]-*N,N-*dimethylbenzenesulphonamide;
• (2-Fluoro-6-{2-[4-(1-(pyrrolidin-1-yl)ethyl)phenyl]imidazo[1,2-*a*]pyridin-6-yl}phenyl)methanol;
• 2-Fluoro-4-[6-(3-fluoro-2-hydroxymethylphenyl)imidazo[1,2-*a*]pyridin-2-yl]-*N,N*-dimethylbenzamide;
• N-{3-[6-(2,4-Difluoro-3-hydroxymethylphenyl)imidazo[1,2-*a*]pyridin-2-yl]phenyl}acetamide;
• {4-[6-(2,4-Difluoro-3-hydroxymethylphenyl)imidazo[1,2-*a*]pyridin-2-yl]phenyl}methanol;
• {3-[6-(2,4-Difluoro-3-hydroxymethylphenyl)imidazo[1,2-*a*]pyridin-2-yl]phenyl}methanol;
• 4-[6-(2,4-Difluoro-3-hydroxymethylphenyl)imidazo[1,2-*a*]pyridin-2-yl]-*N,N*-dimethylbenzamide;
• Methyl {4-[6-(2,4-difluoro-3-hydroxymethylphenyl)imidazo[1,2-*a*]pyridin-2-yl]phenyl}carbamate;
• 4-[6-(2,4-Difluoro-3-hydroxymethylphenyl)imidazo[1,2-*a*]pyridin-2-yl]-*N*-methylbenzamide;
• N-{4-[6-(2,4-Difluoro-3-hydroxymethylphenyl)imidazo[1,2-*a*]pyridin-2-yl]phenyl}acetamide;
• {2,6-Difluoro-3-[2-(4-(morpholin-4-yl)phenyl)imidazo[1,2-*a*]pyridin-6-yl]-phenyl}methanol;
• *N*-{3-[6-(2,4-Difluoro-3-hydroxymethylphenyl)imidazo[1,2-*a*]pyridin-2-yl]phenyl}isobutyramide;
• 3-[6-(2,4-Difluoro-3-hydroxymethylphenyl)imidazo[1,2-*a*]pyridin-2-yl]-*N,N*-dimethylbenzenesulphonamide;
• (2,6-Difluoro-3-{2-[4-(1-(pyrrolidin-1-yl)ethyl)phenyl]imidazo[1,2-*a*]pyridin-6-yl}phenyl)methanol;
• 4-[6-(2,4-Difluoro-3-hydroxymethylphenyl)imidazo[1,2-*a*]pyridin-2-yl]-2-fluoro-*N,N*-dimethylbenzamide.

10. Medicament, **characterized in that** it comprises a compound of formula (I) according to any one of Claims 1 to 9 or an addition salt of this compound with a pharmaceutically acceptable acid.

11. Pharmaceutical composition, **characterized in that** it comprises a compound of formula (I) according to any one of Claims 1 to 9 or a pharmaceutically acceptable salt of this compound and also at least one pharmaceutically acceptable excipient.

12. Use of a compound of formula (I) according to any one of Claims 1 to 9 in the preparation of a medicament intended for the treatment and prevention of neurodegenerative diseases.

13. Use of a compound of formula (I) according to any one of Claims 1 to 9 in the preparation of a medicament intended for the treatment and prevention of cerebral traumas and epilepsy.

14. Use of a compound of formula (I) according to any one of Claims 1 to 9 in the preparation of a medicament intended for the treatment and prevention of psychiatric diseases.

15. Use of a compound of formula (I) according to any one of Claims 1 to 9 in the preparation of a medicament intended for the treatment and prevention of inflammatory diseases.

16. Use of a compound of formula (I) according to any one of Claims 1 to 9 in the preparation of a medicament intended for the treatment and prevention of osteoporosis and cancers.

17. Use of a compound of formula (I) according to any one of Claims 1 to 9 in the preparation of a medicament intended for the treatment and prevention of Parkinson's disease, Alzheimer's disease, tauopathies or multiple sclerosis.

18. Use of a compound of formula (I) according to any one of Claims 1 to 9 in the preparation of a medicament intended for the treatment and prevention of schizophrenia, depression, substance dependence or attention deficit hyperactivity disorders.

19. Process for the synthesis of the compounds of formula (I) according to Claim 1, in which a compound of formula (II) 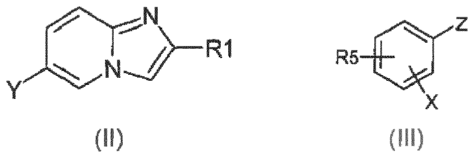 in which R1 is as defined in Claim 1 and Y represents a halogen atom or a boron derivative, is reacted with a derivative of general formula (III), in which X is defined according to Claim 1 and Z represents a boron or tin derivative, if Y represents a halogen atom, or else a halogen atom, if Y represents a boron derivative, and R5 represents the 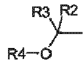 group.

20. Process for the synthesis of the compounds of formula (I) according to Claim 1, in which a compound of general formula (VI): 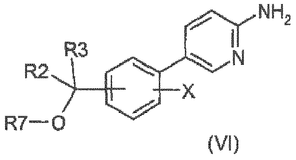 in which R2, R3 and X are defined according to Claim 1 and R7 represents R4 or a protective group for the hydroxyl functional group PG, and a bromoketone of general formula (VII): 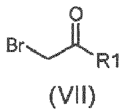 in which R1 is defined according to Claim 1,
are reacted.

21. Process for the synthesis of the compounds of general formula (I) according to Claim 1, in which a compound of general formula (II): 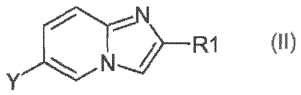 in which R1 is defined according to Claim 1 and Y represents a halogen atom or a boron derivative, is reacted with a derivative of general formula (III') 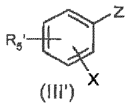 in which X is defined according to Claim 1 and Z represents a boron or tin derivative, when Y represents a halogen atom, or Z represents a halogen atom, when Y represents a boron derivative, and R5' represents either a carbonyl derivative R₂CO, in which R2 is defined according to Claim 1, or an alkyl carboxylate, in order to obtain a compound of general formula (IV), which compound of general formula (IV) subsequently reacts with an organometallic derivative in order to obtain a compound of general formula (I).

22. Process for the synthesis of the compounds of formula (I) according to claim 1, in which a compound of general formula (VI), 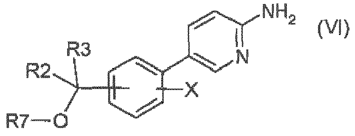 in which R2, R3 and X are defined according to Claim 1 and 7 represents 4 or a protective group (PG) for the hydroxyl functional group, is reacted with a bromoketone of general formula (VII), 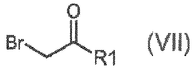 in which R1 is defined according to Claim 1.

23. Process for the synthesis of the compounds of formula (I) according to Claim 1, in which a compound of general formula (VI), 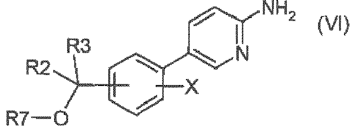 in which R2, R3 and X are defined according to Claim 1 and R7 represents R4 or a protective group (PG) for the hydroxyl functional group, is reacted with a bromoketone of general formula (VII), 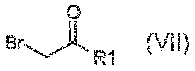 in which R1 is defined according to claim 1, in order to obtain a compound of general formula (VIII) 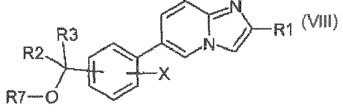 in which R7 represents a protective group for the hydroxyl functional group, which compound of general formula (VIII) is subsequently deprotected in order to obtain a compound of general formula (I).

24. Process for the synthesis of the compounds of formula (I) according to Claim 1, in which a compound of general formula (XI) 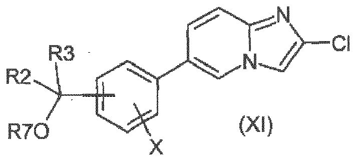 in which X, R2 and R3 are defined according to Claim 1 and R7 represents the R4 group, is reacted with a derivative of general formula (XII)
R1-Z (XII)
in which R1 is defined according to Claim 1 and Z represents a boron or tin derivative, in order to obtain the compounds of general formula (I).

25. Process for the synthesis of the compounds of formula (I) according to Claim 1, in which a compound of general formula (XI) 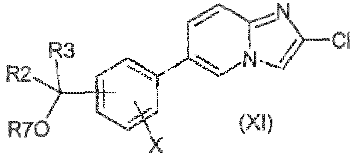 in which X, R2 and R3 are defined according to Claim 1 and R7 represents a protective group for the hydroxyl functional group, is reacted with a derivative of general formula (XII)
R1-Z (XII)
in which R1 is defined according to Claim 1 and Z represents a boron or tin derivative, in order to obtain the compounds of general formula (VIII), 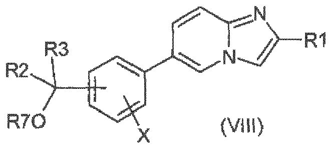 in which R1, R2, R3 and X are defined according to Claim 1 and R7 represents a protective group for the hydroxyl functional group, which compound of general formula (VIII) is subsequently deprotected.

26. Intermediate compounds

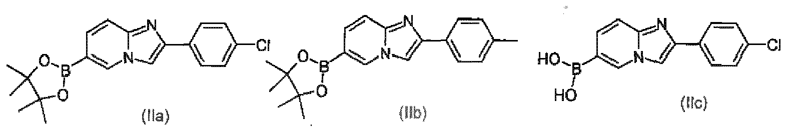

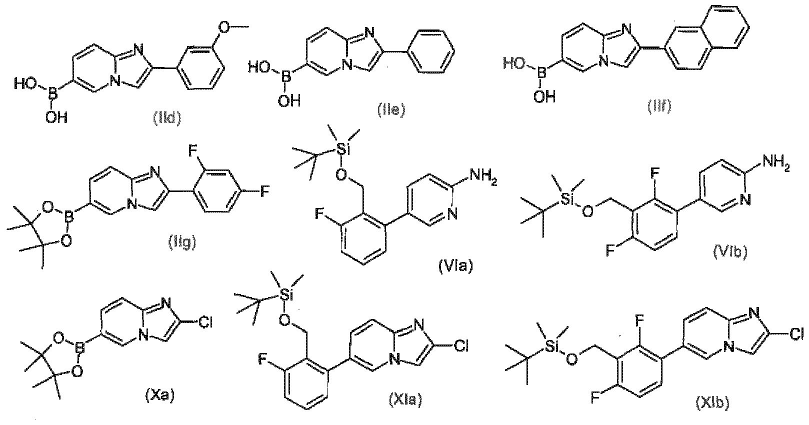

27. Process of synthesis according to Claim 19, 20, 21, 22, 23, 24 or 25, where the compounds of general formulae (II), (VI), (X) and (XI) are the compounds of general formulae (IIa), (IIb), (IIc), (IId), (IIe), (IIf), (IIg), (VIa), (VIb), (Xa), (XIa) and (XIb).

## Patentansprüche

1. Verbindungen der Formel (I) : 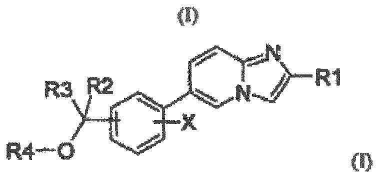 mit den folgenden Bedeutungen:
R₁ bedeutet:
eine Phenylgruppe oder eine Naphthylgruppe, wobei diese beiden Gruppen gegebenenfalls durch ein oder mehrere Atome oder Gruppen, die unabhängig voneinander aus den folgenden Atomen oder Gruppen ausgewählt sind: Halogen, (C₁-C₁₀)-Alkyl, Halogen-(C₁-C₁₀)-alkyl, (C₁-C₁₀)-Alkoxy, Halogen-(C₁-C₁₀)-Alkoxy, (C₁-C₁₀)-Thioalkyl, -S(O) (C₁-C₁₀) Alkyl, -S(O)₂(C₁-C₁₀-Alkyl), Hydroxyl, Cyano, Nitro, Hydroxy-(C₁-C₁₀)-alkylen, NRaRb(C₁-C₁₀) -Alkylen, (C₁-C₁₀)-Alkoxy (C₁-C₁₀)-alkylenoxy, NRaRb, CONRaRb, SO₂NRaRb, NRcCORd, OC(O)NRaRb, OCO(C₁-C₁₀)-Alkyl, NRcC(O)ORe, NRcSO₂Re, Aryl(C₁-C₁₀)-alkylen, monocyclisches Aryl oder Heteroaryl, monocyclisches Aryl oder Heteroaryl, das gegebenenfalls durch einen oder mehrere Substituenten ausgewählt aus der Reihe Halogen, (C₁-C₁₀)-Alkylgruppe, Halogen-(C₁-C₁₀) -alkylgruppe, (C₁-C₁₀) -Alkoxygruppe, Halogen-(C₁-C₁₀) -alkoxygruppe, NRaRb, Hydroxylgruppe, Oxogruppe, Nitrogruppe, Cyanogruppe oder OCO(C₁-C₁₀)-Alkylgruppe substituiert sind, substituiert sein können;
X bedeutet 1 bis 4 gleiche oder voneinander verschiedene Substituenten, die aus der Reihe Halogen,
(C₁-C₁₀) -Alkyl, (C₁-C₁₀) -Alkoxy, NRaRb, Cyano, Nitro ausgewählt sind, wobei das (C₁-C₁₀)-Alkyl gegebenenfalls durch eine oder mehrere Gruppen, die aus der Reihe Halogen, (C₁-C₁₀)-Alkoxy, Halogen-(C₁-C₁₀)-alkoxy, NRaRb oder Hydroxyl ausgewählt sind, substituiert sein kann;
R₂ und R₃ unabhängig voneinander Folgendes bedeuten:
ein Wasserstoffatom,
eine (C₁-C₁₀)-Alkylgruppe, wobei diese Gruppe gegebenenfalls durch eine Gruppe Rf substituiert sein kann;
eine Arylgruppe, die gegebenenfalls durch einen oder mehrere Substituenten, die aus der Reihe Halogen, (C₁-C₁₀)-Alkyl-, Halogen-(C₁-C₁₀)-alkyl-, (C₁-C₁₀) -Alkoxy-, Halogen- (C₁-C₁₀)-alkoxy-, NRaRb-, Hydroxyl-, Nitro- oder Cyanogruppe ausgewählt sind, substituiert sein kann;
R₂ und X gemeinsam mit den Kohlenstoffatomen, von denen sie getragen werden, einen Kohlenstoffring mit 5 bis 7 Kohlenstoffatomen bilden können;
R₄ Folgendes bedeutet:
ein Wasserstoffatom,
eine (C₁-C₁₀)-Alkylgruppe, wobei die Gruppe gegebenenfalls durch eine Gruppe Rf substituiert ist;
eine Arylgruppe, die gegebenenfalls durch einen oder mehrere Substituenten, die aus der Reihe Halogen, (C₁-C₁₀)-Alkyl-, Halogen- (C₁-C₁₀) -alkyl-, (C₁-C₁₀)-Alkoxy-, Halogen-(C₁-C₁₀)-alkoxy-, NRaRb,-, Hydroxyl-, Nitro-, Cyano-, (C₁-C₁₀)-Alkyl(CO)-, CONRaRb-, CCORd-, OC (O) NRaRb-, OCO(C₁-C₁₀)-Alkyl-, NRcC(O)ORe- oder Arylgruppe, wobei das Aryl gegebenenfalls durch einen oder mehrere Substituenten, die aus der Reihe Halogen, (C₁-C₁₀)-Alkyl-, Halogen- (C₁-C₁₀) -alkyl-, (C₁-C₁₀) -Alkoxy-, Halogen-(C₁-C₁₀)-alkoxy, NRaRb-, Hydroxyl-, Nitro - oder Cyanogruppe ausgewählt sind, substituiert ist;
Ra und Rb unabhängig voneinander Folgendes bedeuten:
ein Wasserstoffatom oder eine (C₁-C₁₀)-Alkyl-, Aryl-(C₁-C₁₀)-alkylen- oder Arylgruppe;
oder Ra und Rb gemeinsam mit dem Stickstoffatom, von dem sie getragen werden, eine Azetidin-, Pyrrolidin-, Piperidin-, Azepin-, Morpholin-, Thiomorpholin-, Piperazin-, Homopiperazingruppe bilden, wobei diese Gruppe gegebenenfalls durch eine (C₁-C₁₀)-Alkyl-, Aryl- oder Aryl- (C₁-C₁₀)-alkylengruppe substituiert ist;
Rc und Rd unabhängig voneinander Folgendes bedeuten:
ein Wasserstoffatom oder eine (C₁-C₁₀)-Alkyl-, Aryl- (C₁-C₁₀)-Alkylen-, Arylgruppe;
oder Rc und Rd gemeinsam eine (C₂-C₅)-Alkylengruppe bilden;
Re Folgendes bedeutet:
eine (C₁-C₁₀)-Alkyl-, Aryl-(C₁-C₁₀)-alkylen-, Arylgruppe;
oder Rc und Re gemeinsam eine (C₂-C₅)-Alkylengruppe bilden;
Rf Folgendes bedeutet:
ein Halogenatom, eine (C₁-C₁₀)-Alkoxy-, Halogen-(C₁-C₁₀)-alkoxy-, Hydroxyl-, Cyano-, NRaRb-, C(O)NRaRb-, NRcCORd-, OC(O)NRaRb-, OCO(C₁₀-C₁₀)-Alkyl-, NRcCOORe- , SO₂NRaRb-, NRcSO₂Re-, Aryl-(C₁-C₁₀)-alkylen-, Arylgruppe, wobei das Aryl gegebenenfalls durch einen oder mehrere Substituenten, die aus der Reihe Halogen, (C₁-C₁₀)-Alkyl-, Halogen- (C₁-C₁₀)-alkyl-, (C₁-C₁₀)-Alkoxy-, Halogen-(C₁-C₁₀)-alkoxy, NRaRb-, Hydroxyl-, Nitro-, Cyano- oder OCO(C₁-C₁₀)-Alkylgruppe ausgewählt sind, substituiert ist,
als Base oder als Säureadditionssalz.

2. Verbindungen der Formel (I) nach Anspruch 1, **dadurch gekennzeichnet, dass**
R₁ eine Phenylgruppe oder eine Naphthylgruppe bedeutet, die gegebenenfalls durch ein oder mehrere Atome oder Gruppen, die unabhängig voneinander aus der Reihe Halogenatome oder (C₁-C₁₀) -Alkyl-, (C₁-C₁₀) -Alkoxy-, Nitro-, -S(O)₂(C₁-C₁₀)-Alkyl-, Halogen-(C₁-C₁₀) -alkyl-, Cyano-, (C₁-C₁₀)-Thioalkyl-, NRaRb-, NRcCORd-, Hydroxy(C₁-C₁₀)-alkylen-, NRcSO₂Re-, CONRaRb-, NRcC(O)ORe-, SO₂NRaRb-, NRaRb(C₁-C₁₀)-Alkylengruppe ausgewählt sind, substituiert ist;
Ra und Rb unabhängig voneinander ein Wasserstoffatom oder eine (C₁-C₁₀)-Alkylgruppe bedeuten;
oder Ra und Rb gemeinsam mit dem. Stickstoffatom, von dem sie getragen werden, eine Pyrrolidinyl-, Morpholinylgruppe bilden;
Rc und Rd unabhängig voneinander ein Wasserstoffatom oder eine (C₁-C₁₀)-Alkylgruppe bedeuten;
Re eine (C₁-C₁₀)-Alkylgruppe bedeutet.

3. Verbindungen der Formel (I) nach Anspruch 1 oder 2, **dadurch gekennzeichnet, dass**
X 1 oder 2 identische oder voneinander verschiedene Substituenten, die aus der Reihe, Halogen, (C₁-C₁₀)-Alkyl, (C₁-C₁₀)-Alkoxy ausgewählt sind, bedeutet, wobei das (C₁-C₁₀)-Alkyl gegebenenfalls durch eine Hydroxylgruppe substituiert sein kann;
R₂ und X gemeinsam mit den Kohlenstoffatomen, von denen sie getragen werden, einen Kohlenstoffring mit 5 oder 6 Kohlenstoffen bilden können.

4. Verbindungen der Formel (I) nach einem der vorhergehenden Ansprüche, **dadurch gekennzeichnet, dass** R₂ und R₃ unabhängig voneinander ein Wasserstoffatom oder eine (C₁-C₁₀)-Alkylgruppe bedeuten.

5. Verbindungen der Formel (I) nach einem der vorhergehenden Ansprüche, **dadurch gekennzeichnet, dass** R₄ ein Wasserstoffatom, eine (C₁-C₁₀)-Alkylgruppe, wobei diese Gruppe gegebenenfalls durch eine Gruppe Rf substituiert ist, bedeutet;
Rf eine (C₁-C₁₀)-Alkoxygruppe bedeutet.

6. Verbindungen der Formel (I) nach einem der vorhergehenden Ansprüche, **dadurch gekennzeichnet, dass** die Substitution der Gruppe 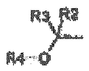 an Phenylring in 2-, 3- oder 4-Stellung steht.

7. Verbindungen der Formel (I) nach einem der vorhergehenden Ansprüche, **dadurch gekennzeichnet, dass** R₁ eine Phenylgruppe oder eine Naphthylgruppe bedeutet, die gegebenenfalls durch ein oder mehrere Atome oder Gruppen, die unabhängig voneinander aus der Reihe Halogenatome oder (C₁-C₁₀) -Alkyl-, (C₁-C₁₀)-Alkoxy-, Nitro-, -S(O)₂(C₁-C₁₀)-Alkyl-, Halogen-(C₁-C₁₀) -alkyl-,
Cyano-, (C₁-C₁₀)-Thioalkyl-, NRaRb-, NRcCORd-, Hydroxy(C₁-C₁₀)-alkylen-, NRcSO₂Re-, CONRaRb-, NRcC(O)ORe-, SO₂NRaRb-, NRaRb(C₁-C₁₀) -Alkylengruppen ausgewählt sind, substituiert ist;
Ra und Rb unabhängig voneinander ein Wasserstoffatom oder eine (C₁-C₁₀)-Alkylgruppe bedeuten;
oder Ra und Rb gemeinsam mit dem Stickstoffatom, von dem sie getragen werden, eine Pyrrolidinyl-, Morpholinylgruppe bilden,
Rc und Rd unabhängig voneinander ein Wasserstoffatom oder eine (C₁-C₁₀)-Alkylgruppe bedeuten;
Re eine (C₁-C₁₀)-Alkylgruppe bedeutet;
X 1 oder 2 identische oder voneinander verschiedene Substituenten, die aus der Reihe Halogen, (C₁-C₁₀) - Alkyl, (C₁-C₁₀)-Alkoxy ausgewählt sind, bedeutet, wobei die (C₁-C₁₀)-Alkylgruppe gegebenenfalls durch eine Hydroxylgruppe substituiert sein kann;
R₂ und X gemeinsam mit den Kohlenstoffatomen, von denen sie getragen werden, einen Kohlenstoffring mit 5 oder 6 Kohlenstoffen bilden können;
R₂ und R₃ unabhängig voneinander ein Wasserstoffatom oder eine (C₁-C₁₀)-Alkylgruppe bedeuten;
R₄ ein Wasserstoffatom, eine (C₁-C₁₀)-Alkylgruppe, wobei diese Gruppe gegebenenfalls durch eine Gruppe Rf substituiert ist, bedeutet;
Rf eine (C₁-C₁₀)-Alkoxygruppe bedeutet;
die Substitution der Gruppe 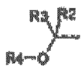 am Phenylring in 2-3- oder 4-Stellung steht;
als Base oder als Säureadditionssalz.

8. Verbindungen der Formel (I) nach Anspruch 1, **dadurch gekennzeichnet, dass**
R₁ eine Phenylgruppe, die gegebenenfalls durch ein Halogen, eine (C₁-C₁₀) -Alkylgruppe oder eine (C₁-C₁₀) - Alkoxygruppe substituiert ist, bedeutet;
X ein oder mehrere aus der Reihe Halogen, (C₁-C₁₀) Alkyl, (C₁-C₁₀)-Alkoxy, Hydroxy-(C₁-C₁₀)-alkyl bedeutet; R₂ und R₃ unabhängig voneinander ein Wasserstoffatom oder eine (C₁-C₁₀)-Alkylgruppe bedeuten;
R₂ und X gemeinsam mit den Kohlenstoffatomen, von denen sie getragen werden, einen Kohlenstoffring mit 6 Kohlenstoffatomen bilden können;
R₄ ein Wasserstoffatom bedeutet,
als Base oder als Säureadditionssalz.

9. Verbindungen:
• {3-[2-(4-Chlorphenyl)imidazo[1,2-a]pyridin-6-yl]-2-fluorphenyl}methanol;
• 1-{3-[2-(4-Chlorphenyl)imidazo[1,2-a]pyridin-6-y1]-2-fluorphenyl}ethanol;
• {3-[2-(4-Chlorphenyl)imidazo[1,2-a]pyridin-6-yl]-2,6-difluorphenyl}methanol;
• 1-{3-[2-(4-Chlorphenyl)imidazo[1,2-a]pyridin-6-yl]-2,6-difluorphenyl}ethanol;
• 1-{5-[2-(4-Chlorphenyl)imidazo[1,2-a]pyridin-6-yl]-2-fluorphenyl}ethanol;
• {3-[2-(4-chlorphenyl)imidazo[1,2-a]pyridin-6-yl]-5-methylphenyl}methanol;
• {3-[2-(4-Chlorphenyl)imidazo[1,2-a]pyridin-6-yl]-5-methoxyphenyl}methanol;
• 7-[2-(4-Chlorphenyl)imidazo[1,2-a]pyridin-6-yl]-1,2,3,4-tetrahydronaphthalin-1-ol;
• 5-[2-(4-Chlorphenyl)imidazo[1,2-a]pyridin-6-yl]-1,2,3,4-tetrahydronaphthalin-1-ol;
• {3-[2-(4-Chlorphenyl)imidazo[1,2-a]pyridin-6-yl]-4-methylphenyl}methanol;
• {5-[2-(4-Chlorphenyl)imidazo[1,2-a]pyridin-6-yl]-2-fluorphenyl}methanol;
• {5-[2-(4-Chlorphenyl)imidazo[1,2-a]pyridin-6-yl]-2-methylphenyl}methanol;
• {3-[2-(4-Chlorphenyl)imidazo[1,2-a]pyridin-6-yl]-2-methylphenyl}methanol;
• [2-Fluor-4-(2-(*p*-tolylimidazo[1,2-*a*]pyridin-6-yl)phenyl]methanol;
• [2-Fluor-6-(2-(*p*-tolylimidazo[1,2-*a*]pyridin-6-yl)phenyl]methanol;
• {2-Fluor-6-[2-(3-methoxyphenyl)imidazo[1,2-*a*]pyridin-6-yl]phenyl}methanol;
• {2-Fluor-4-[2-(3-methoxyphenyl)imidazo[1,2-*a*]pyridin-6-yl]phenyl}methanol und sein Hydrochlorid;
• [2-Fluor-4-(2-phenylimidazo[1,2-*a*]pyridin-6-yl)phenyl]methanol und sein Hydrochlorid;
• {2-Fluor-3-[2-(3-methoxyphenyl)imidazo[1,2-*a*]pyridin-6-yl]phenyl}methanol;
• [2-Fluor-6-(2-phenylimidazo[1,2-*a*]pyridin-6-yl)phenyl]methanol und sein Hydrochlorid;
• [2-Fluor-3-(2-(*p*-tolylimidazo[1,2-*a*]pyridin-6-yl)phenyl]methanol;
• 1-{3-[2-(4-Chlorphenyl)imidazo[1,2-*a*]pyridin-6-yl]-4-fluorphenyl}ethanol;
• {3-[2-(4-Chlorphenyl)imidazo[1,2-*a*]pyridin-6-yl}-4-fluorphenyl)methanol;
• {3-[2-(4-Chlorphenyl)imidazo[1,2-*a*]pyridin-6-yl]-4-methoxyphenyl}methanol;
• 1-{3-[2-(4-Chlorphenyl)imidazo[1,2-*a*]pyridin-6-yl]-4-methoxyphenyl}ethanol;
• {3-[2-(4-Chlorphenyl)imidazo[1,2-*a*]pyridin-6-yl]-5-(hydroxymethyl)phenyl}methanol;
• [2-Fluor-3-[2-(naphthyl-2-yl)imidazo[1,2-*a*]pyridin-6-yl]phenyl]methanol;
• [2-Fluor-3-(2-phenylimidazo[1,2-*a*]pyridin-6-yl)phenyl]methanol und sein Hydrochlorid;
• (+)-1-{3-[2-(4-Chlorphenyl)imidazo[1,2-*a*]pyridin-6-yl]-4-fluorphenyl}ethanol;
• (-)-1-{3-[2-(4-Chlorphenyl)imidazo[1,2-*a*]pyridin-6-yl]-4-fluorphenyl}ethanol;
• {2-[2-(2,4-Difluorphenyl)imidazo[1,2-*a*]pyridin-6-yl]-6-fluorphenyl}methanol;
• {3-[2-(2,4-Difluorphenyl)imidazo[1,2-*a*]pyridin-6-yl]-2,6-difluorphenyl}methanol;
• 2-{4-Fluor-3-[2-(3-methoxyphenyl)imidazo[1,2-*a*]pyridin-6-yl]phenyl}propan-2-ol;
• 1-{4-Fluor-3-[2-(3-methoxyphenyl)imidazo[1,2-a]pyridin-6-yl]phenyl}ethanol;
• 2-{3-[2-(4-Chlorphenyl)imidazo[1,2-*a*]pyridin-6-yl]-2,6-difluorphenyl 2;
• 1-[4-Fluor-2-(2-naphthyl-2-ylimidazo[1,2-*a*]pyridin-6-yl)phenyl]ethanol;
• 6-(2,4-Difluor-3-methoxymethylphenyl)-2-(p-tolyl)imidazo[1,2-*a*]pyridin;
• {2-Fluor-6-[2-(4-nitrophenyl)imidazo[,2-*a*]pyridin-6-yl]phenyl}methanol;
• {2,6-Difluor-3-[2-(4-pyrrolidin-1-ylphenyl)imidazo[1,2-*a*]pyridin-6-yl]phenyl}methanol;
• {3-[2-(3,4-Difluorphenyl)imidazo[1,2-*a*]pyridin-6-yl]-2,6-difluorphenyl}methanol;
• {2,6-Difluor-3-[2-(2-fluorophenyl)imidazo[1,2-a]pyridin-6-yl]phenyl}methanol;
• {3-[2-(3-Bromphenyl)imidazo[1,2-*a*]pyridin-6-yl]-2,6-difluorphenyl}methanol;
• {2,6-Difluor-3-[2-(4-methylsulfonylphenyl)imidazo[1,2-*a*]pyridin-6-yl]phenyl}methanol;
• 2-[2-Methyl-3-(2-phenylimidazo[1,2-*a*]pyridin-6-yl)phenyl]propan-2-ol;
• 7-[2-(2,4-Difluorphenyl)imidazo[1,2-*a*]pyridin-6-yl]indan-1-ol;
• 2-(4-Chlorphenyl)-6-[2,4-difluor-3-[(2-methoxyethyl)oxymethyl]phenyl]imidazo[1,2-*a*]pyridin;
• 7-[2-(2,4-Difluorphenyl)imidazo[1,2-*a*]pyridin-6-yl]-1-methylindan-1-ol;
• 2-{3-[2-(4-Chlorphenyl)imidazo[1,2-*a*]pyridin-6-yl]-2-methoxyphenyl}propan-2-ol;
• 1-{3-[2-(4-Chlorphenyl)imidazo[1,2-*a*]pyridin-6-yl]-2-methoxyphenyl}ethanol;
• {2-Fluor-6-[2-(4-trifluormethylphenyl)imidazo[1,2-a]pyridin-6-yl]phenyl}methanol;
• {2-Fluor-6-[2-(4-methoxyphenyl)imidazo[1,2-*a*]pyridin-6-yl]phenyl}methanol;
• {2-Fluor-6-[2-(4-(pyrrolidin-1-yl)phenyl)imidazo[1,2-*a*]pyridin-6-yl]phenyl}methanol;
• {2-Fluor-6-[2-(4-fluorphenyl)imidazo[1,2-*a*]pyridin-6-yl]phenyl}methanol;
• {2-[2-(3,4-Difluorphenyl)imidazo[1,2-*a*]pyridin-6-yl]-6-fluorphenyl}methanol;
• 3-[6-(3-Fluor-2-hydroxethylphenyl)imidazo[1,2-*a*]pyridin-2-yl]benzonitril;
• {2-Fluor-6-[2-(2-fluorphenyl)imidazo[1,2-*a*]pyridin-6-yl]phenyl}methanol;
• {2-[2-(3-Bromphenyl)imidazo[1,2-*a*]pyridin-6-yl]-6-fluorphenyl}methanol;
• {2-[2-(Chlor-3-methylphenyl[1,2-*a*]pyridin-6-yl]-6-fluorphenyl}methanol;
• {2-[2-(3-Chlor-4-methylphenyl)imidazo[1,2-*a*]pyridin-6-yl]-6-fluorphenyl}methanol;
• {2-Fluor-6-[2-(4-methylsulfonylphenyl)imidazo[1,2-*a*]pyridin-6-yl]phenyl}methanol;
• {2,6-Difluor-3-[2-(4-methoxyphenyl)imidazo[1,2-*a*]pyridin-6-yl]phenyl}methanol;
• {2,6-Difluor-3-[2-(4-fluorphenyl)imidazo[1,2-*a*]pyridin-6-yl]phenyl}methanol;
• 3-[6-(2,4-Difluor-3-hydroxmethylphenyl)imidazo[1,2-*a*]pyridin-2-yl]benzonitril;
• {2,6-Difluor-3-[2-(4-chlor-3-methylphenyl)imidazo[1,2-*a*]pyridin-6-yl]phenyl}methanol;
• (2,6-Difluor-3-[2-(3-chlor-4-methylphenyl)imidazo[1,2-*a*]pyridin-6-yl]phenyl}methanol;
• (2,6-Difluor-3-[2-(4-methylthiophenyl)imidazo[1,2 - *a*]pyridin-6-yl]phenyl}methanol;
• 2-[2-Chlor-3-[2-(4-chlorphenyl)imidazo[1,2-*a*]pyridin-6-yl]phenyl]propan-2-ol;
• 1-[2-Chlor-3-[2-(4-chlorphenyl) imidazo[1,2-*a*]pyridin-6-yl]phenyl]ethanol;
• {2-[2-(4-Chlorphenyl)imidazo[1,2-*a*]pyridin-6-yl]-5-fluorphenyl}methanol;
• 2-(4-Chlorphenyl)-6-[3-fluor-2-[(2-methoxyethyl)oxymethyl]phenyl]imidazo[1,2-*a*]pyridin;
• *N*-{3-[6-(3-Fluor-2-hydroxymethylphenyl)imidazo[1,2-*a*]pyrzdin-2-yl]phenyl}acetid;
• {4-[6-(3-Fluor-2-hydroxymethylphenyl)imidazo[1,2-*a*]pyridin-2-yl]phenyl}methanol;
• {3-[6-(3-Fluor-2-hydroxymethylphenyl)imidazo[1,2-a]pyridin-2-yl]phenyl}methanol;
• *N*-{4-[6-(3-Fluor-2-hydroxmethylphenyl)imidazo[1,2-*a*]pyridin-2-yl]phenyl}methansulfonamid;
• 4-[6-(3-Fluor-2-hydroxmethylphenyl)imidazo[1,2-*a*]pyridin-2-yl]-*N*,*N-*dimethylbenzid;
• Methyl-{4-[6-(3-fluor-2-hydroxymethylphenyl)imidazo[1,2-*a*]pyridin-2-yl]phenyl}carbamat;
• 4-[6-(3-Fluor-2-hydroxymethylphenyl)imidazo[1,2-*a*]pyridin-2-yl]-*N*-methylbenzamid;
• N-{4-[6-(3-Fluor-2-hydroxymethylphenyl)imidazo[1,2-a]pyridin-2-yl]phenyl}acetamid;
• {2-Fluor-6-[2-(4-(morpholin-4-yl)phenyl)imidazo[1,2-a]pyridin-6-yl]phenyl}methanol;
• 3-[6-(3-Fluor-2-hydroxymethylphenyl)imidazo[1,2-*a*]pyridin-2-yl]-*N*,*N*-dimethylbenzolsulfonamid;
• (2-Fluor-6-{2-[4-(1-pyrrolidin-1-ylethyl)phenyl]imidazo[1,2-*a*]pyridin-6-yl}phenyl)methanol;
• 2-Fluor-4-[6-(3-fluor-2-hydroxymethylphenyl)imidazo[1,2*-a*]pyridin-2-yl]-*N,N-*dimethylbenzamid;
• N-{3-[6-(2,4-Difluor-3-hydroxymethylphenyl)imidazo[1,2-*a*]pyridin-2-yl)phenyl}acetamid;
• {4-[6-(2,4-Difl-uor-3-hydroxymethylphenyl)imidazo[1,2-a]pyridin-2-yl]phenyl}methanol;
• {3-[6-(2,4-Difluor-3-hydroxymethylphenyl)imidazo[1,2-*a*]pyridin-2-yl]phenyl}methanol;
• 4-[6-(2,4-Difluor-3-hydroxethylphenyl)imidazo[1,2-*a*]pyridin-2-yl]-*N,N-*dimethylbenzamid;
• Methyl-{4-[6-(2,4-difluor-3-hydroxymethylphenyl)imidazo[1,2-a]pyridin-2-yl]phenyl}carbamat;
• 4-[6-(2,4-Difluor-3-hydroxymethylphenyl)imidazo[1,2-*a*]pyridin-2-yl]-*N*-methylbenzamid;
• N-{4-[6-(2,4-Difluor-3-hydroxymethylphenyl)imidazo[1,2-*a*]pyridin-2-yl]phenyl}acetamid;
• {2,6-Difluor-3-[2-(4-morpholin-4-ylphenyl)imidazo[1,2-a]pyridin-6-yl]phenyl}methanol;
• *N*-{3-[6-(2,4-Difluor-3-hydroxymethylphenyl)imidazo[1,2-*a*]pyridin-2-yl]phenyl}isobutyramid;
• 3-[6-(2,4-Difluor-3-hydroxymethylphenyl)imidazo[1,2-*a*]pyridin-2-yl]-*N,N*-dimethylbenzolsulfonamid;
• (2,6-Difluor-3-{2-[4-(1-pyrrolidin-1-ylethyl)phenyl]imidazo[2,2-a]pyridin-6-yl}phenyl)methanol;
• 4-[6-(2,4-Difluor-3-hydroxymethylphenyl)imidazo[1,2-a]pyridin-2-yl]-2-fluor-*N,N*-dimethylbenzamid.

10. Arzneimittel, **dadurch gekennzeichnet, dass** es eine Verbindung der Formel (I) nach einem der Ansprüche 1 bis 9 oder ein Additionssalz dieser Verbindung mit einer pharmazeutisch unbedenklichen Säure umfasst.

11. Pharmazeutische Zusammensetzung, **dadurch gekennzeichnet, dass** sie eine Verbindung der Formel (I) nach einem der Ansprüche 1 bis 9 oder ein pharmazeutisch unbedenkliches Salz dieser Verbindung sowie mindestens einen pharmazeutisch unbedenklichen Grundstoff umfasst.

12. Verwendung einer Verbindung der Formel (I) nach einem der Ansprüche 1 bis 9 für die Herstellung eines Arzneimittels zur Behandlung und Vorbeugung von neurodegenerativen Erkrankungen.

13. Verwendung einer Verbindung der Formel (I) nach einem der Ansprüche 1 bis 9 für die Herstellung eines Arzneimittels zur Behandlung und Vorbeugung von Hirntraumen und Epilepsie.

14. Verwendung einer Verbindung der Formel (I) nach einem der Ansprüche 1 bis 9 für die Herstellung eines Arzneimittels zur Behandlung und Vorbeugung von psychiatrischen Erkrankungen.

15. Verwendung einer Verbindung der Formel (I) nach einem der Ansprüche 1 bis 9 für die Herstellung eines Arzneimittels zur Behandlung und Vorbeugung von entzündlichen Erkrankungen.

16. Verwendung einer Verbindung der Formel (I) nach einem der Ansprüche 1 bis 9 für die Herstellung eines Arzneimittels zur Behandlung und Vorbeugung von Osteoporose und Krebs.

17. Verwendung einer Verbindung der Formel (I) nach einem der Ansprüche 1 bis 9 für die Herstellung eines Arzneimittels zur Behandlung und Vorbeugung von Morbus Parkinson, Morbus Alzheimer, Tauopathien und Multipler Sklerose.

18. Verwendung einer Verbindung der Formel (I) nach einem der Ansprüche 1 bis 9 für die Herstellung eines Arzneimittels zur Behandlung und Vorbeugung von Schizophrenie, Depression, Substanzabhängigkeit, Aufmerksamkeitsdefizit-Hyperaktivitätssyndrom.

19. Verfahren zur Synthese von Verbindungen der Formel (I) nach Anspruch 1, bei dem man eine Verbindung der Formel (II) 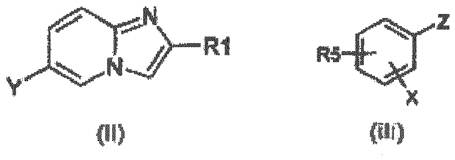 worin R1 wie in Anspruch 1 definiert ist und Y ein Halogenatom oder ein Borderivat bedeutet, mit einem Derivat der allgemeinen Formel (III), worin X wie in Anspruch 1 definiert ist und Z ein Bor- oder zinnderivat bedeutet, wenn Y ein Halogenatom bedeutet, oder Z ein Halogenatom bedeutet, wenn Y ein Borderivat
bedeutet, und R5 die Gruppe 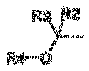 bedeutet, umsetzt.

20. Verfahren zur Synthese von Verbindungen der Formel (I) nach Anspruch 1, bei dem man eine Verbindung der allgemeinen Formel (VI) 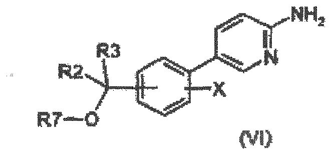 in der R2, R3 und X wie in Anspruch 1 definiert sind und R7 R4 und eine Hydroxylfunktionschutzgruppe PG bedeutet, und ein Bromketon der allgemeinen Formel (VII), 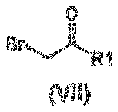 in der R1 wie in Anspruch 1 definiert ist, umsetzt.

21. Verfahren zur Synthese von Verbindungen der Formel (I) nach Anspruch 1, bei dem man eine Verbindung der allgemeinen Formel (II), 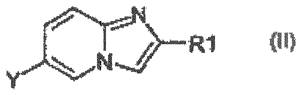 in der R1 wie in Anspruch 1 definiert ist und Y ein Halogenatom oder ein Borderivat bedeutet, mit einem Derivat der allgemeinen Formel (III'), 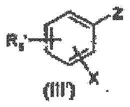 in der X wie in Anspruch 1 definiert ist und Z ein Bor - oder zinnderivat bedeutet, wenn Y ein Halogenatom bedeutet, oder Z ein Halogenatom bedeutet, wenn Y ein Borderivat bedeutet, und R5' entweder ein Carbonylderivat R₂CO, worin R2 wie in Anspruch 1 definiert ist, oder ein Alkylcarboxylat bedeutet, umsetzt, um zu einer Verbindung der allgemeinen Formel (IV) zu gelangen, und die Verbindung der allgemeinen Formel (IV) anschließend mit einem metallorganischen Derivat umsetzt, wodurch man zu einer Verbindung der allgemeinen Formel (I) gelangt.

22. Verfahren zur Synthese von Verbindungen der Formel (I) nach Anspruch 1, bei dem man eine Verbindung der allgemeinen Formel (VI), 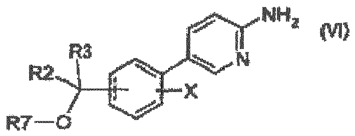 worin R2, R3 und X wie in Anspruch 1 definiert sind und R7 R4 oder eine Hydroxylfunktionschutzgruppe (PG) bedeutet, mit einem Bromketon der allgemeinen Formel (VII), 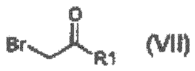 worin R1 wie in Anspruch 1 definiert ist, umsetzt.

23. Verfahren zur Synthese von Verbindungen der Formel (I) nach Anspruch 1, bei dem man eine Verbindung der allgemeinen Formel (VI), 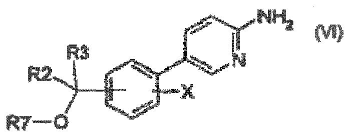 worin R2, R3 und X wie in Anspruch 1 definiert sind und R7 R4 oder eine Hydroxylfunktionschutzgruppe (PG) bedeutet, mit einem Bromketon der allgemeinen Formel (VII), 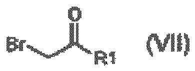 worin R1 wie in Anspruch 1 definiert ist, umsetzt, wodurch man zu einer Verbindung der allgemeinen Formel (VIII) gelangt, 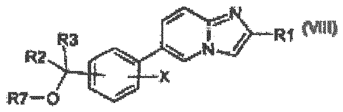 worin R7 eine Hydroxylfunktionsschutzgruppe bedeutet, und die Verbindung der allgemeinen Formel (VIII) anschließend entschützt wird, wodurch man zu einer Verbindung der allgemeinen Formel (I) gelangt.

24. Verfahren zur Synthese von Verbindungen der Formel (I) nach Anspruch 1, bei dem man eine Verbindung der allgemeinen Formel (XI), 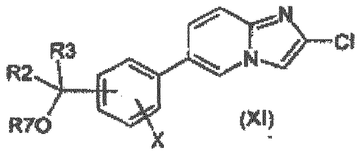 worin X, R2 und R3 wie in Anspruch 1 definiert sind und R7 die Gruppe R4 bedeutet, mit einem Derivat der allgemeinen Formel (XII),
R1-Z (XII)
worin R1 wie in Anspruch 1 definiert ist und Z ein Bor - oder Zinnderivat bedeutet, umsetzt, wodurch man zu den Verbindungen der allgemeinen Formel (I) gelangt.

25. Verfahren zur Synthese von Verbindungen der Formel (I) nach Anspruch 1, bei dem man eine Verbindung der allgemeinen Formel (XI), 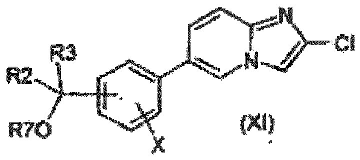 worin X, R2 und R3 wie in Anspruch 1 definiert sind und R7 die Hydroxylfunktionsschutzgruppe bedeutet, mit einem Derivat der allgemeinen Formel (XII),
R1-Z (XII)
worin R1 wie in Anspruch 1 definiert ist und Z ein Bor - oder Zinnderivat bedeutet, umsetzt, wodurch man zu den Verbindungen der allgemeinen Formel (VIII) gelangt, 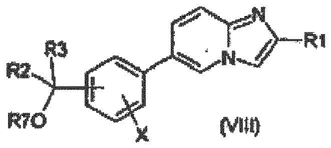 Worin R1, R2, R3 und X wie in Anspruch 1 definiert sind und R7 eine Hydroxylfunktionsschutzgruppe bedeutet, und die Verbindung der allgemeinen Formel (VIII) anschließend entschützt wird.

26. Zwischenprodukte

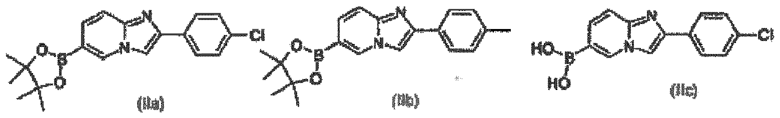

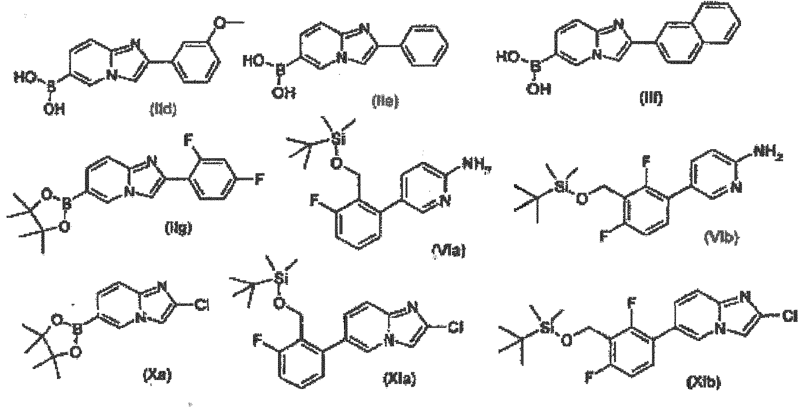

27. Syntheseverfahren nach Anspruch 19, 20, 21, 22, 23, 24 oder 25, wobei es sich bei den Verbindungen der allgemeinen Formeln (II), (VI), (X), (XI) um die Verbindungen der allgemeinen Formeln (IIa), (iib), (IIc), (IId), (IIe), (IIf), (IIg), (VIa), (VIb), (Xa), (XIa) und (XIb) handelt.
